# EUROPEAN PATENT APPLICATION

(11) **EP 4 155 308 A1**
(43) Date of publication of application: **29.03.2023**
(21) Application number: 21842081.8
(22) Date of filing: 08.07.2021
(51) Int. Cl.: C07D 471/06, C07D 487/02, C07D 401/14, C07D 293/10, A61K 31/395, A61K 31/506, A61P 9/00, A61P 35/00, A61P 3/08

(54) **ROCK INHIBITOR, AND PREPARATION METHOD THEREFOR AND USE THEREOF**

(30) Priority: 14.07.2020 CN 202010676711; 11.01.2021 CN 202110040185
(71) Applicant: Wuhan LL Science and Technology Development Co., Ltd., Wuhan, Hubei 430075 (CN)
(72) Inventor: LI, Jinping, Wuhan, Hubei 430075 (CN); ZENG, Jing, Wuhan, Hubei 430075 (CN); GUO, Xiaodan, Wuhan, Hubei 430075 (CN); PENG, Wei, Wuhan, Hubei 430075 (CN); LOU, Jun, Wuhan, Hubei 430075 (CN); LIU, Li, Wuhan, Hubei 430075 (CN); CHEN, Xiaoya, Wuhan, Hubei 430075 (CN); ZHANG, Yihan, Wuhan, Hubei 430075 (CN); CHEN, Yongkai, Wuhan, Hubei 430075 (CN); WANG, Chaodong, Wuhan, Hubei 430075 (CN); WU, Wei, Wuhan, Hubei 430075 (CN); YUAN, Yi, Wuhan, Hubei 430075 (CN)
(74) Representative: Gille Hrabal Partnerschaftsgesellschaft mbB Patentanwälte
(86) International application number: PCT/CN2021/105263
(87) International publication number: WO 2022/012409

(57) **Abstract**

A ROCK inhibitor represented by formula (I), and a preparation method therefor and a use thereof. The ROCK inhibitor has excellent ROCK inhibition activity, particularly shows good selective inhibition on ROCK2 kinase, has good safety and metabolic stability, and is high in bioavailability. The preparation method for the ROCK inhibitor is simple, and the ROCK inhibitor is easy to purify, and therefore has a good application prospect.

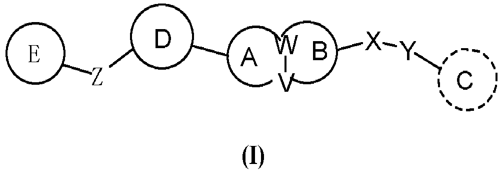

## Description

The present application claims priority to Patent Application No. 202010676711.6 and Patent Application No. 202110040185.9 filed with China National Intellectual Property Administration on Jul. 14, 2020 and Jan. 11, 2021, respectively, and entitled **"ROCK INHIBITOR, AND PREPARATION METHOD THEREFOR AND USE THEREOF",** which are incorporated herein by reference in their entirety.

### TECHNICAL FIELD

The present disclosure relates to the technical field of medicines, and particularly relates to a **ROCK** inhibitor, and a preparation method therefore and use thereof.

### BACKGROUND

Idiopathic interstitial pulmonary fibrosis (IPF) is a chronic and diffuse pulmonary interstitial disease with unknown cause and its pathological change is common interstitial pneumonia, and it mainly shows manifestation of common interstitial pneumonia according to histopathology and imageology examinations. The disease condition progresses irreversibly due to the complex pathogenesis of the disease, and early diagnosis is difficult; the survival rate of patients diagnosed with the disease is remarkably decreased over time, and the 3-year survival rate is 50%, and the 5-year survival rate is only 20%, which is lower than that of most cancers (such as leukemia, breast cancer, colon cancer, uterine tumor and renal cancer), and therefore the disease is called "non-cancerous cancer". At present, there is no definitely significantly effective therapeutic drug for IPF. According to the results of randomized control clinical trials in recent years and the actual clinical conditions in China, drugs such as pirfenidone and nintedanib can be used as appropriate. Only IPF patients with mild to moderate pulmonary dysfunction are recommended to be treated by nintedanib, while whether IPF patients with severe pulmonary dysfunction can benefit from treatment by nintedanib and the course of treatment still need to be further discussed.

Rho GTPase was discovered in 1985, and it belongs to the Ras superfamily and has 25% homology to Ras. At present, Rho GTPase members found in mammalian tissue cells are mainly Rho (A, B, C), Rac (1, 2, 3), Cdc42 (Cdc42Hs/G25K, TC10, Tcl), Rh₀ D, Rh₀ G, Chp (1, 2), Rnd (Rh₀ E/Rnd3, Rnd1/Rho6, Rnd2/Rho7), Rho H/TTF, Rif, Wrch1 and Rho BTB (1, 2), where Rho (A, B, C) is one of the most important members of Rho GTPase. Rho-associated protein kinase (ROCK), also called Rho-associated kinase, belongs to serine/threonine protein kinase, and it has a molecular mass of about 160 kD and is a Rho downstream effector molecule which is studied most detailedly in terms of functional study at present. ROCK includes ROCK1 (ROKβ, p160-ROCK) and ROCK2 (ROKα) subtypes. The amino acid sequences of the two subtypes are 65% identical, with a high degree of similarity (92% identity) in the kinase domain. ROCK is distributed throughout the body, and in comparison, ROCK1 is more highly expressed in non-neural tissues (blood, small intestine, thymus, etc.), while ROCK2 is more highly expressed in brain, heart and colon.

ROCK is involved in occurrence of various cardiovascular and cerebrovascular diseases, including hypertension, atherosclerosis, ischemic stroke, heart disease, diabetic nephropathy, ocular diseases, tumors, nerve injury diseases, radiation damage, autoimmune diseases and the like. For example, the **Rh₀/ROCK** signaling pathway is involved in **NAD(P)H** oxidase activation and induces oxidative stress, inducing cardiac microvascular damage and C-reactive protein-induced atherothrombosis; high glucose can activate **Rh₀/ROCK** pathway, induce the expression of visceral adipokine and type I procollagen in cardioblast, and cause the hyperproliferation of cardioblast and thus induce diabetic cardiomyopathy; activation of Rho/ROCK signaling pathway can regulate NF-κB signaling pathway, up-regulate inflammatory genes and induce the occurrence of diabetic nephropathy; **Rh₀/ROCK** signaling pathway changes biofilm permeability and affects metastasis of cancer cells; in the case of spinal cord injury, Rho is activated, inducing atrophy of growth cones and thereby causing axonal regeneration disorder and simultaneously inducing inhibition against neuron growth by chondroitin sulfate proteoglycan.

In addition, the Rho/ROCK signaling pathway is involved in the occurrence and progression of fibrosis diseases. Activation of Rh₀/ROCK signaling pathway can increase the level of ischemic myocardial fibrosis, and heart tissues of an acute myocardial fibrosis rat show significantly increased Rh₀ and ROCK expression. Activation of Rh₀/ROCK signaling pathway can induce phosphorylation of actin, initiating cellular fibrosis. Both *in vivo* and *in vitro* experimental results demonstrate that cardiopulmonary physiological and pathological damage caused by exposure to radiation over a period of time is associated with fibrosis induced with the involvement of Rh₀/ROCK pathway. Formation of endothelial adhesion fibronectin and focal adhesion, decreased endothelial cell migration and endothelial dysfunction due to ionizing radiation are associated with actin scaffold reorganization and stress fiber formation induced by activation of Rho/ROCK signaling pathway.

With respect to lung injury by IPF, primarily alveolar epithelial cells (ACEs) are the targets, and the death of ACEs triggers wound healing responses including innate immune activation, vascular leakage and extravascular coagulation, fibroblast recruitment, proliferation and activation, synthesis and cross-linking of extracellular matrix, alveolar collapse and epithelial regeneration. ROCK signals can fundamentally regulate the activities of these cells involved in the healing response, particularly those of epithelial cells, endothelial cells, and fibroblasts. The key role of ROCK in these responses further suggests the potential of ROCK inhibitors for the treatment of pulmonary fibrosis.

Currently, no drugs that treat numerous disorders including fibrosis through inhibition of ROCK are available on the market. The development of new medicaments requires careful optimization of the chemical and biological properties of the lead compounds. Further, the compounds must have desired pharmacokinetic and pharmacodynamic characteristics. This laborious development process typically requires extensive experimentation. In many cases, the process of determining the optimal compound often requires the preparation of thousands of structurally similar compounds. Therefore, the development of a novel backbone compound having inhibitory effect on ROCK1 and/or ROCK2 kinases by improving a ROCK kinase inhibitor is of positive significance for the treatment of the above-mentioned diseases.

### SUMMARY

In order to solve the problems in the prior art, the present disclosure provides a compound represented by formula (I) below or a racemate, a stereoisomer, a tautomer, an isotopically labeled compound, a nitrogen oxide, a solvate, a polymorph, a metabolite, an ester, a pharmaceutically acceptable salt or a prodrug thereof: 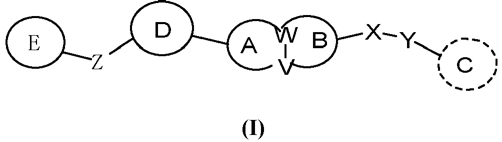 wherein,
ring A is the following group unsubstituted or optionally substituted with one, two or more Rc: 3-20 membered heterocyclyl or 5-20 membered heteroaryl;
ring B is the following group unsubstituted or optionally substituted with one, two or more Rd: C₃₋₂₀ cycloalkyl, 3-20 membered heterocyclyl, C₆₋₂₀ aryl or 5-20 membered heteroaryl;
the ring A and the ring B constitute a fused ring with W and V as connection sites;
Rc and Rd are identical or different and are each independently selected from halogen, amino, hydroxyl, formyl, and the following groups unsubstituted or optionally substituted with one, two or more Rs: C₁₋₂₀ alkyl and C₁₋₂₀ alkoxy;
W and V are each independently C or N, and W and V are not both N at the same time;
X is -C(=O)-;
Y is OH, a chemical bond, C₁₋₂₀ alkoxy, C₁₋₂₀ alkyl, -NH-, or -N(C₁₋₂₀ alkyl)-, -NH(C₁₋₂₀ alkyl) or -NH(C₁₋₂₀ alkoxy), wherein the C₁₋₂₀ alkoxy, the C₁₋₂₀ alkyl and the C₁₋₂₀ alkoxy and C₁₋₂₀ alkyl in the "-N(C₁₋₂₀ alkyl)-, -NH(C₁₋₂₀ alkyl) or -NH(C₁₋₂₀ alkoxy)" can be unsubstituted or optionally substituted with one, two or more Rs; 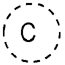
can be present or absent;
provided that Y is OH, C₁₋₂₀ alkoxy, C₁₋₂₀ alkyl, -NH(C₁₋₂₀ alkyl) or -NH(C₁₋₂₀ alkoxy), 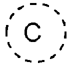 is absent;
when Y is a chemical bond, 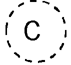 is selected from the following groups unsubstituted or optionally substituted with one, two or more Ra: 3-20 membered heterocyclyl, 5-20 membered heteroaryl, C₃₋₂₀ cycloalkyl and C₆₋₂₀ aryl;
when Y is-NH- or -N(C₁₋₂₀ alkyl)-, 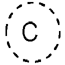 is selected from the following groups unsubstituted or optionally substituted with one, two or more Rb: C₁₋₂₀ alkyl, 3-20 membered heterocyclyl, 5-20 membered heteroaryl, C₃₋₂₀ cycloalkyl and C₆₋₂₀ aryl;
Ra and Rb are identical or different and are each independently selected from halogen, CN, C₁₋₂₀ alkyl unsubstituted or optionally substituted with one, two or more Rs, OH, 5-20 membered heteroaryl and 5-20 membered haloheteroaryl;
ring D is the following group unsubstituted or optionally substituted with one, two or more Re: C₃₋₂₀ cycloalkyl, 3-20 membered heterocyclyl, C₆₋₂₀ aryl or 5-20 membered heteroaryl, and the ring A is connected to the ring D through an N atom;
Re is selected from halogen, CN, OH and the following groups unsubstituted or optionally substituted with one, two or more Rs: C₁₋₂₀ alkyl, C₃₋₂₀ cycloalkyl, C₁₋₂₀ alkoxy, C₆₋₂₀ aryl, 3-20 membered heterocyclyl and 5-20 membered heteroaryl;
or when at least two Re substitutions are present on the ring D, adjacent two Re, together with an atom on the ring D to which they are connected via a bond, form C₃₋₂₀ cycloalkyl, 3-20 membered heterocyclyl or 3-20 membered heteroaryl;
Rs is selected from halogen, CN, OH, C₁₋₂₀ alkyl, C₁₋₂₀ alkoxy, C₆₋₂₀ aryl and 5-20 membered heteroaryl;
Z is -NH-;
ring E is the following group unsubstituted or optionally substituted with one, two or more Rf: C₆₋₂₀ aryl or 5-20 membered heteroaryl;
Rf is selected from halogen, CN, OH, and the following groups unsubstituted or optionally substituted with one, two or more Rg: C₁-C₂₀ alkyl, C₆₋₂₀ aryl and 5-20 membered heteroaryl;
Rg is selected from halogen, CN, OH, C₁-C₂₀ alkyl optionally substituted with one, two or more halogens, and -O-5-20 membered heteroaryl, wherein the -O-5-20 membered heteroaryl is optionally substituted with - C(O)-NH₂.

According to an embodiment of the present disclosure, W is selected from N and C;
V is selected from C;
X is -C(=O)-;
Y is OH, a chemical bond, C₁₋₆ alkoxy, C₁₋₆ alkyl, -NH-, or -N(C₁₋₆ alkyl)-, -NH(C₁₋₆ alkyl) or -NH(C₁₋₆ alkoxy), wherein the C₁₋₆ alkoxy, the C₁₋₆ alkyl and the C₁₋₆ alkoxy and C₁₋₆ alkyl in the "-N(C₁₋₆ alkyl)-, -NH(C₁₋₆ alkyl) or -NH(C₁₋₆ alkoxy)" can be unsubstituted or optionally substituted with one, two or more Rs;
Rs is selected from halogen, CN, OH, C₁₋₆ alkyl, C₁₋₆ alkoxy, C₆₋₁₂ aryl and 5-12 membered heteroaryl.

According to an embodiment of the present disclosure,
when Y is a chemical bond, 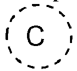 is selected from the following groups unsubstituted or optionally substituted with one, two or more Ra: 3-6 membered heterocyclyl and 5-12 membered heteroaryl;
Ra is selected from F, Cl, CN, OH, and C₁₋₆ alkyl optionally substituted with one, two or more halogens;
when Y is -NH- or -N(C₁₋₆ alkyl)-, 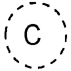 is selected from the following groups unsubstituted or optionally substituted with one, two or more Rb: C₃₋₆ cycloalkyl, C₁₋₆ alkyl, 3-6 membered heterocyclyl and 5-12 membered heteroaryl;
Rb is selected from F, Cl, CN, OH, and 5-12 membered heteroaryl substituted with F or Cl;
In one embodiment, when Y is a chemical bond, X is connected to a heteroatom of the 3-6 membered heterocyclyl represented by 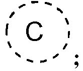
ring A is the following group unsubstituted or optionally substituted with one, two or more Rc: 3-6 membered heterocyclyl or 5-12 membered heteroaryl;
ring B is the following group unsubstituted or optionally substituted with one, two or more Rd: 3-6 membered heterocyclyl, C₆₋₁₂ aryl or 5-12 membered heteroaryl;
the ring A and the ring B constitute a fused ring with W and V as connection sites;
Rc and Rd are identical or different and are each independently selected from F, Cl, amino, hydroxyl, formyl and C₁₋₆ alkyl, wherein the C₁₋₆ alkyl is unsubstituted or optionally substituted with one, two or more halogens;
the ring D is the following group unsubstituted or optionally substituted with one, two or more Re: C₆₋₁₂ aryl or 5-12 membered heteroaryl; and the ring A is connected to the ring D through an N atom;
Re is selected from halogen, CN, OH, and the following groups unsubstituted or optionally substituted with one, two or more Rs: C₁₋₆ alkyl, C₃₋₆ cycloalkyl, C₁₋₆ alkoxy, C₆₋₁₂ aryl, 3-6 membered heterocyclyl and 5-12 membered heteroaryl;
or when at least two Re substitutions are present on the ring D, adjacent two Re, together with an atom on the ring D to which they are connected via a bond, form C₃₋₆ cycloalkyl, 3-6 membered heterocyclyl or 5-12 membered heteroaryl;
Rs is selected from halogen, CN, OH, C₁₋₆ alkyl, C₁₋₆ alkoxy, C₆₋₁₂ aryl and 5-12 membered heteroaryl;
Z is -NH-;
the ring E is C₆₋₁₂ aryl or 5-12 membered heteroaryl unsubstituted or optionally substituted with one, two or more Rf;
Rf is selected from F, Cl, and the following groups unsubstituted or optionally substituted with one, two or more Rg: C₆₋₁₂ aryl and 5-12 membered heteroaryl;
Rg is selected from F, Cl and -0-5-12 membered heteroaryl, wherein the -0-5-12 membered heteroaryl is optionally substituted with -C(O)-NH₂.

According to a preferred embodiment of the present disclosure, W is selected from N and C;
V is selected from C;
X is -C(=O)-;
Y is OH, a chemical bond, -NH- or -N(methyl)-, provided that when Y is OH, 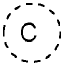 is absent;
when Y is a chemical bond, 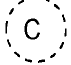 is selected from the following groups: azetidinyl unsubstituted or substituted with one, two or more F; azetidinyl unsubstituted or substituted with one, two or more CN; azetidinyl unsubstituted or substituted with one, two or more CF₂; azetidinyl unsubstituted or substituted with one, two or more CF₃; and azolidinyl unsubstituted or substituted with F and/or OH;
when Y is -NH- or -N(methyl)-, 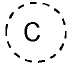 is selected from the following groups: cyclobutyl unsubstituted or substituted with one, two or more F; isopropyl; pyridazinyl; azetidinyl unsubstituted or substituted with one, two or more CN; azolidinyl substituted with F and/or OH; ethyl unsubstituted or substituted with one, two or more F; -N-chloropyridin-piperidinyl; and pyridinyl unsubstituted or substituted with one, two or more F;
In one embodiment, when Y is a chemical bond, X is connected to a heteroatom of the heterocyclyl represented by 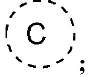 In one embodiment, when 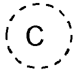 is absent, Y is methyl, ethyl, propyl, butyl, -CH₂CF₃, -NH-CH₂CF₃, - NHCH₂(CH₂)₂, -OCH₂CF₃ or -OCH₂CH₃;

According to an embodiment of the present disclosure, the ring A and the ring B constitute a fused ring as follows:

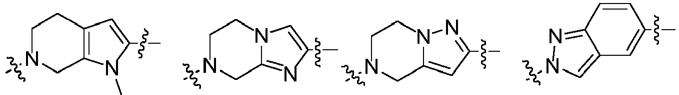

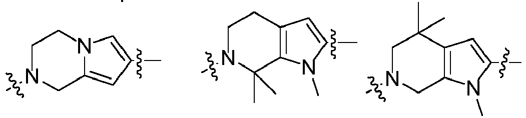

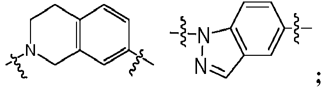

According to an embodiment of the present disclosure, the ring D is the following group unsubstituted or optionally substituted with one, two or more Re: 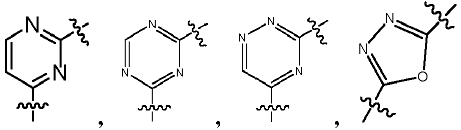 or 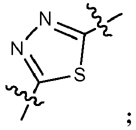 or is 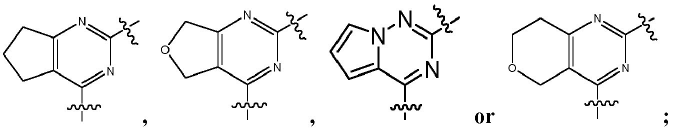 wherein Re is as defined above.

The ring D may be further selected from: 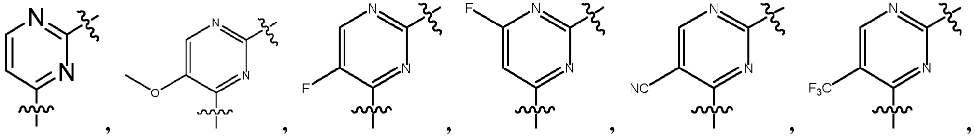 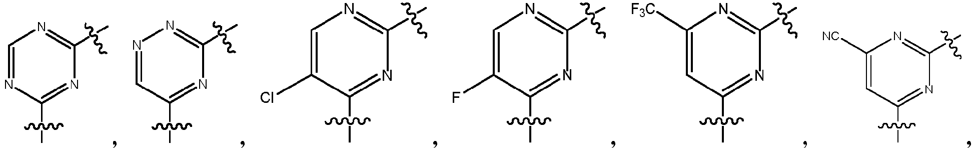 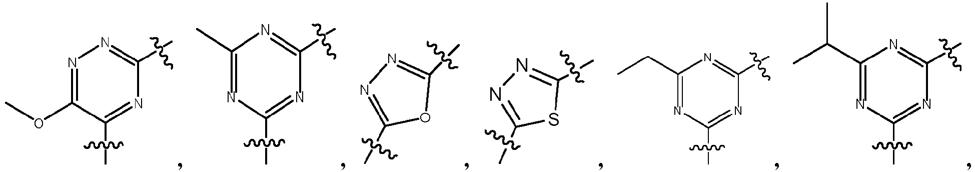 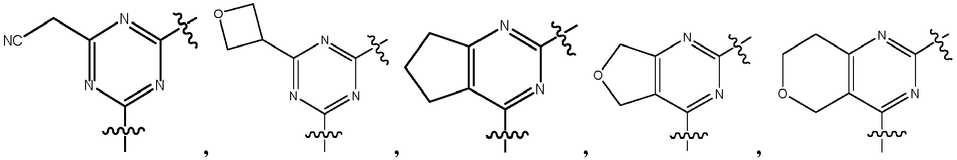 and

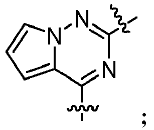

According to an embodiment of the present disclosure, Z is -NH-;
According to an embodiment of the present disclosure,
the ring E is

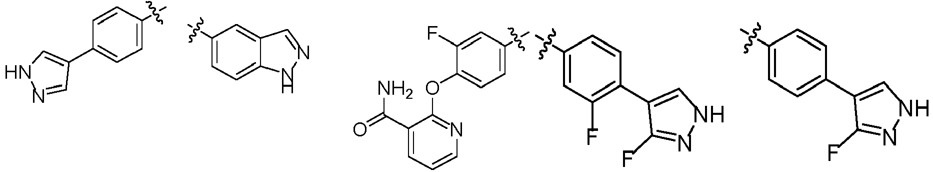

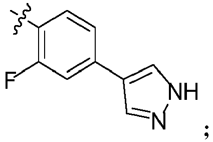

According to an embodiment of the present disclosure, 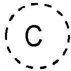 may be selected from the following structures:

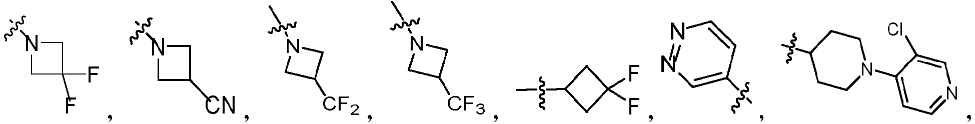

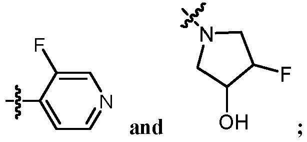

According to an embodiment of the present disclosure, Re is selected from halogen, CF₃, CN, methoxy, ethoxy, propoxy, butoxy, methyl, ethyl, propyl, butyl, CHF₂, -CNCH₂-, glycidyl, oxetanyl and oxolanyl; or, when at least two Re substitutions are present on the ring D, adjacent two Re, together with an atom on the ring D to which they are connected to via a bond, form C₅₋₆ cycloalkyl, 5-6 membered heterocyclyl or 5-6 membered heteroaryl, such as cyclopentyl, oxolanyl, oxacyclohexyl and pyrrolyl;

In some embodiments, the present disclosure provides a compound represented by formula (II) below or a racemate, a stereoisomer, a tautomer, an isotopically labeled compound, a nitrogen oxide, a solvate, a polymorph, a metabolite, an ester, a pharmaceutically acceptable salt or a prodrug thereof: 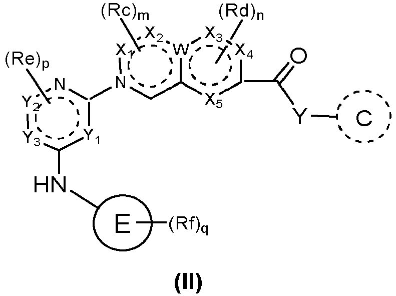
wherein, W is selected from N and C;
a double line comprising a solid line and a dotted line represents a single bond or a double bond;
X₁, X₃ and X₅ are each independently selected from N and C, and X₁, X₃ and X₅ are not N at the same time; X₂ and X₄ are selected from C and a chemical bond;
X₃ is selected from N and C;
Y₁ is selected from N, O and S;
Y₂ is selected from C, N and a chemical bond;
Y₃ is selected from N and C;
Y₂ and Y₃ are not both N at the same time;
m is 0, 1, 2, 3 or 4;
n is 0, 1, 2 or 3;
p is 0, 1, 2, 3 or 4;
q is 0, 1, 2, 3 or 4;
the other groups are as defined above.

In some embodiments, the present disclosure provides a compound represented by formula (III) below or a racemate, a stereoisomer, a tautomer, an isotopically labeled compound, a nitrogen oxide, a solvate, a polymorph, a metabolite, an ester, a pharmaceutically acceptable salt or a prodrug thereof: 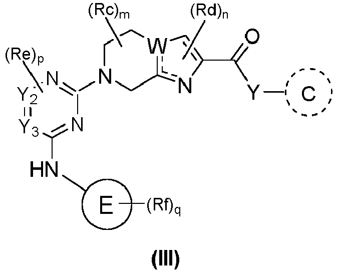
wherein, W is selected from N and C;
a double line comprising a solid line and a dotted line represents a single bond or a double bond;
Y₂ and Y₃ are each independently C or N, and Y₂ and Y₃ are not both N at the same time;
m is 0, 1, 2, 3 or 4;
n is 0, 1, 2 or 3;
p is 0, 1, 2, 3 or 4;
q is 0, 1, 2, 3 or 4;
the other groups are as defined above.

In some embodiments, the present disclosure provides a compound represented by formula (IV) below or a racemate, a stereoisomer, a tautomer, an isotopically labeled compound, a nitrogen oxide, a solvate, a polymorph, a metabolite, an ester, a pharmaceutically acceptable salt or a prodrug thereof: 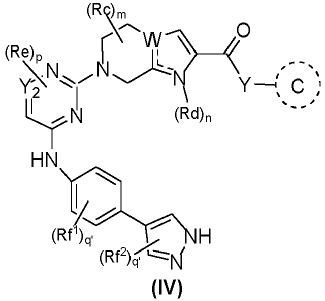
wherein, W is selected from N and C;
Y₂ is selected from N and C;
a double line comprising a solid line and a dotted line represents a single bond or a double bond;
Y is a chemical bond, -NH-, or -N(C₁₋₆ alkyl)- or -NH(C₁₋₆ alkyl);
the C₁₋₆ alkoxy in the "-N(C₁₋₆ alkyl)- or -NH(C₁₋₆ alkyl)" is unsubstituted or optionally substituted with one, two or more halogens;
when Y is a chemical bond, 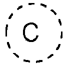 is selected from the following groups: 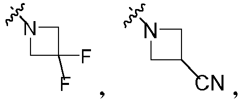 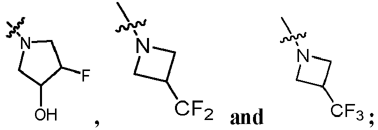
when Y is -NH- or -N(C₁₋₆ alkyl)-, 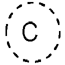 is selected from the following group: 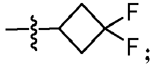
when Y is -N(C₁₋₆ alkyl), 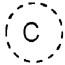 is absent;
Rc and Rd are identical or different and are each independently selected from C₁₋₆ alkyl;
Re is selected from halogen, CN, OH, and the following groups unsubstituted or optionally substituted with one, two or more Rs: C₁₋₆ alkyl, C₃₋₆ cycloalkyl, C₁₋₆ alkoxy, C₆₋₁₂ aryl, 3-6 membered heterocyclyl and 5-12 membered heteroaryl;
or when at least two Re are present, adjacent two Re, together with a ring atom to which they are connected via a bond, form C₃₋₆ cycloalkyl, 3-6 membered heterocyclyl or 5-12 membered heteroaryl;
Rs is selected from halogen, CN, OH, C₁₋₆ alkyl, C₁₋₆ alkoxy, C₆₋₁₂ aryl and 5-12 membered heteroaryl;
Rf¹ and Rf² are identical or different and are each independently selected from Rf as defined above, preferably each independently selected from halogen, CN and OH;
m is 0, 1 or 2;
n is 0, 1 or 2;
p is 0, 1 or 2;
q', when present, is each independently 0, 1 or 2.

In some embodiments, the present disclosure provides a compound or a racemate, a stereoisomer, a tautomer, an isotopically labeled compound, a nitrogen oxide, a solvate, a polymorph, a metabolite, an ester, a pharmaceutically acceptable salt or a prodrug thereof, wherein 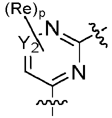 is preferably the following group:

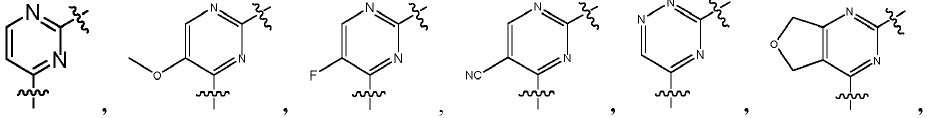

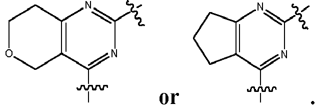

In some embodiments, the present disclosure provides a compound represented by formula (V) below or a racemate, a stereoisomer, a tautomer, an isotopically labeled compound, a nitrogen oxide, a solvate, a polymorph, a metabolite, an ester, a pharmaceutically acceptable salt or a prodrug thereof: 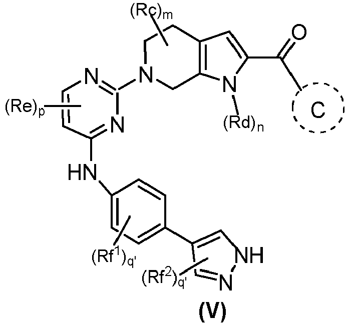 wherein, 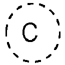 is selected from the following groups: 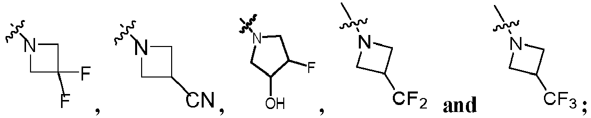
Rc and Rd are identical or different and are each independently selected from C₁₋₆ alkyl;
Re is selected from halogen, CN, OH, and the following groups unsubstituted or optionally substituted with one, two or more Rs: C₁₋₆ alkyl, C₃₋₆ cycloalkyl, C₁₋₆ alkoxy, C₆₋₁₂ aryl, 3-6 membered heterocyclyl and 5-12 membered heteroaryl;
or when at least two Re are present, adjacent two Re, together with a ring atom to which they are connected via a bond, form C₃₋₆ cycloalkyl, 3-6 membered heterocyclyl or 5-12 membered heteroaryl;
Rs is selected from halogen, CN, OH, C₁₋₆ alkyl, C₁₋₆ alkoxy, C₆₋₁₂ aryl and 5-12 membered heteroaryl;
Rf¹ and Rf² are identical or different and are each independently selected from Rf as defined above, preferably each independently selected from halogen, CN and OH;
m is 0, 1 or 2;
n is 0, 1 or 2;
p is 0, 1 or 2;
q', when present, is each independently 0, 1 or 2.

According to a preferred embodiment of the present disclosure,
in the formula I, the 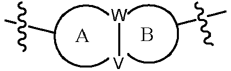 is selected from the following structures: 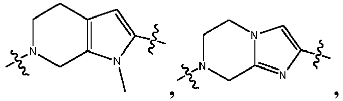
X is -C(=O)-;
when Y is a chemical bond, 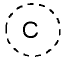 is selected from the following groups: 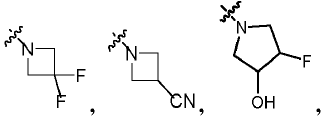 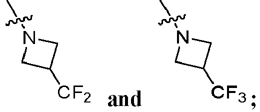
when Y is -NH- or -N(C₁₋₂₀ alkyl)-, 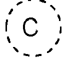 is selected from the following group: 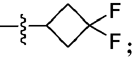
or when 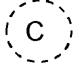 is absent, Y is -NH-CH₂CF₃;
ring D is selected from the following structures: 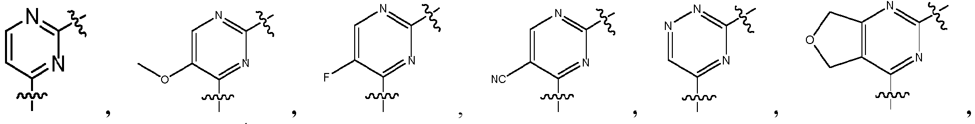 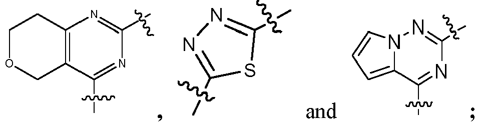
Z is -NH-;
ring E is selected from the following structures:

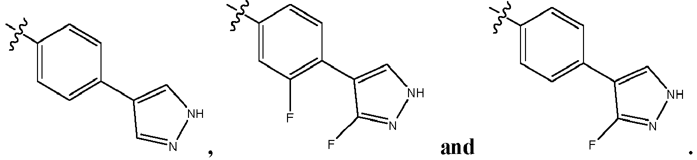

According to a preferred embodiment of the present disclosure,
in the formula I, the 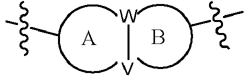 is selected from the following structures: 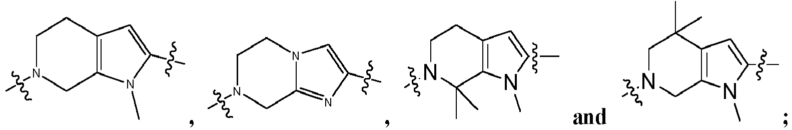
X is -C(=O)-;
when Y is a chemical bond, 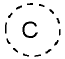 is selected from the following group: 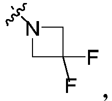
when Y is -NH- or -N(C₁₋₂₀ alkyl)-, 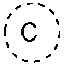 is selected from the following group: 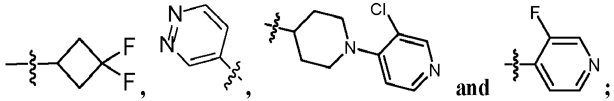
or when 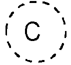 is absent, Y is -OCH₂CF₃;
ring D is selected from the following structures: 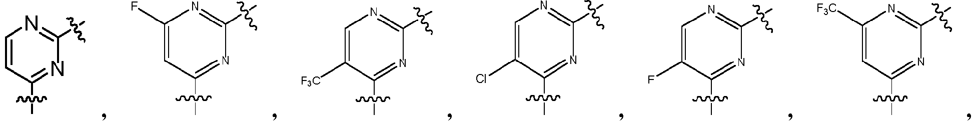 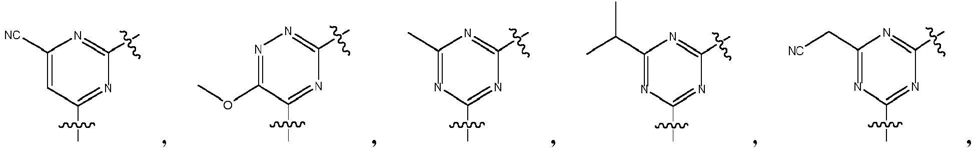 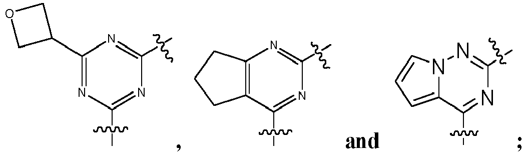
Z is -NH-;
ring E is selected from the following structures:

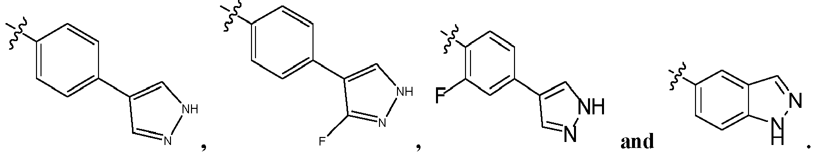

As an example, in the compound represented by formula (I) or the racemate, the stereoisomer, the tautomer, the isotopically labeled compound, the nitrogen oxide, the solvate, the polymorph, the metabolite, the ester, the pharmaceutically acceptable salt or the prodrug thereof, the compound represented by formula (I) may be selected from the following structures:

| **Example No.** | **Compound No.** | **Structural formula** |
|---|---|---|
| **1** | **L001** | 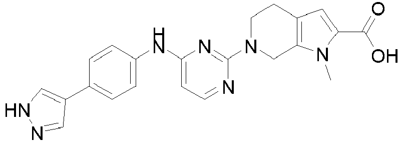 |
| **2** | **L002** | 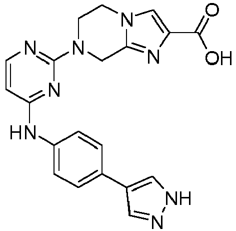 |
| **3** | **L003** | 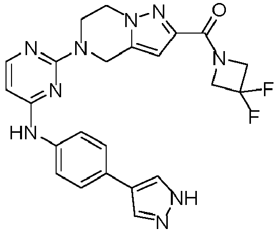 |
| **4** | **L004** | 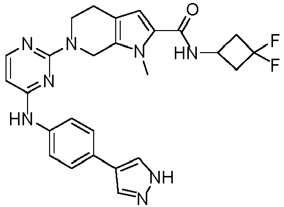 |
| **5** | **L005** | 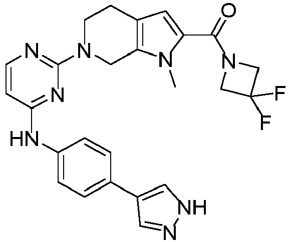 |
| **6** | **L006** | 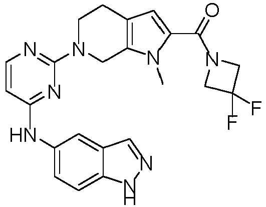 |
| **7** | **L007** | 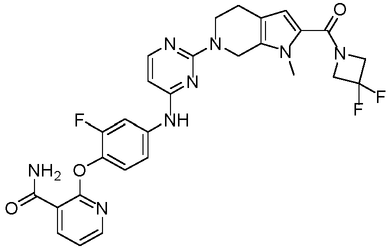 |
| **8** | **L008** | 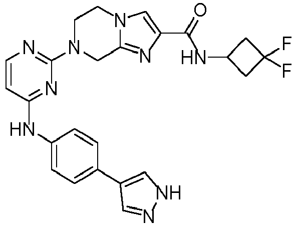 |
| **9** | **L009** | 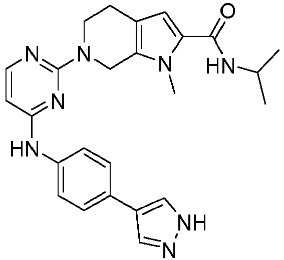 |
| **10** | **L010** | 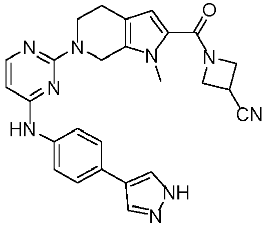 |
| **11** | **L011** | 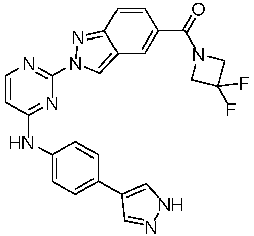 |
| **12** | **L012** | 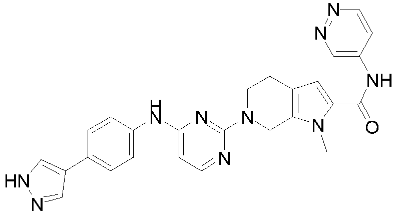 |
| **13** | **L013** | 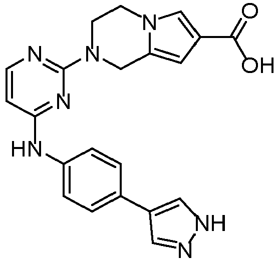 |
| **14** | **L014** | 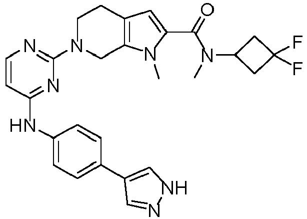 |
| **15** | **L015** | 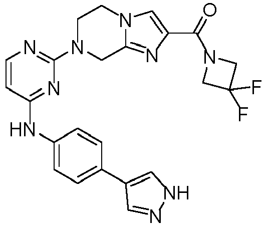 |
| **16** | **L016** | 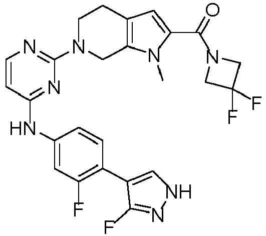 |
| **17** | **L017** | 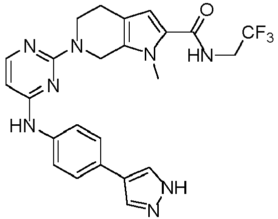 |
| **18** | **L018** | 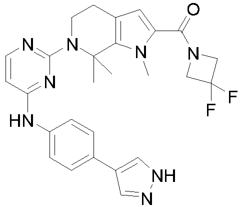 |
| **19** | **L019** | 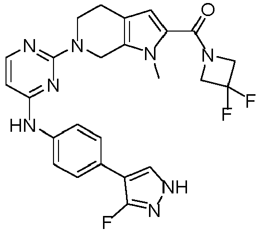 |
| **20** | **L020** | 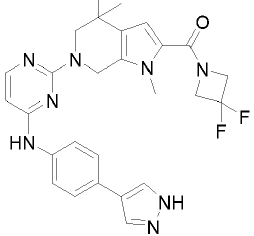 |
| **21** | **L021** | 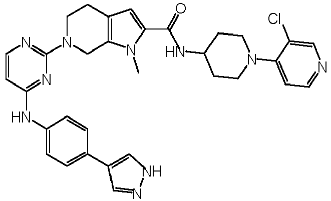 |
| **22** | **L022** | 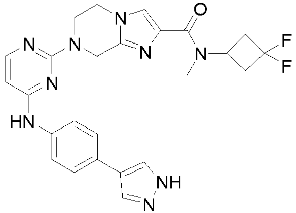 |
| **23** | **L023** | 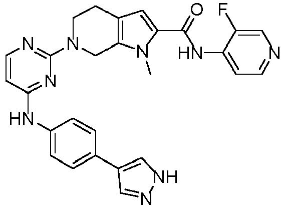 |
| **24** | **L024** | 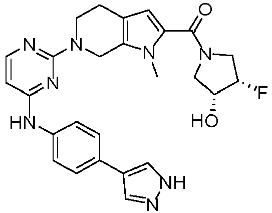 |
| **25** | **L025** | 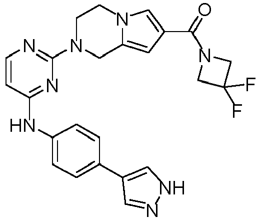 |
| **26** | **L026** | 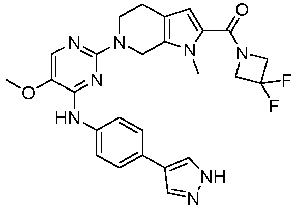 |
| **27** | **L027** | 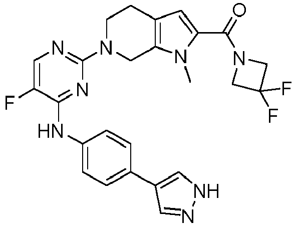 |
| **28** | **L028** | 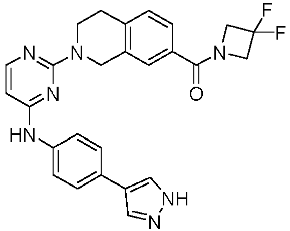 |
| **29** | **L029** | 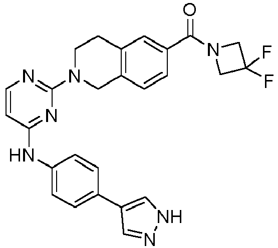 |
| **30** | **L030** | 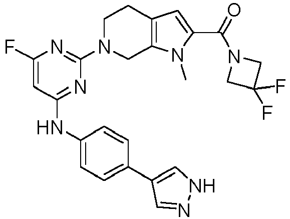 |
| **31** | **L031** | 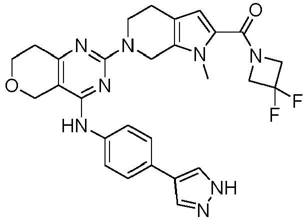 |
| **32** | **L032** | 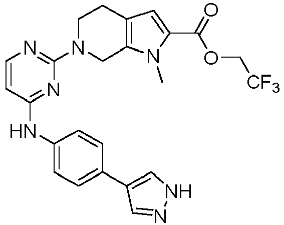 |
| **33** | **L033** | 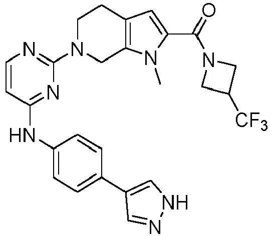 |
| **34** | **L034** | 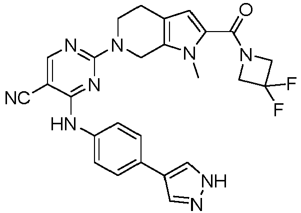 |
| **35** | **L035** | 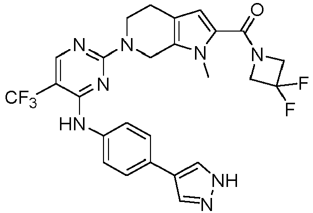 |
| **36** | **L036** | 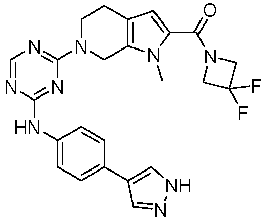 |
| **37** | **L037** | 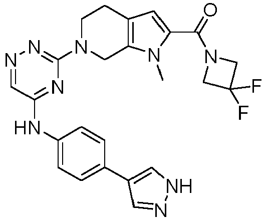 |
| **38** | **L038** | 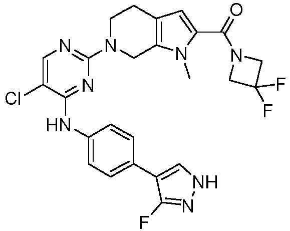 |
| **39** | **L039** | 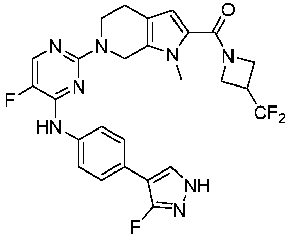 |
| **40** | **L040** | 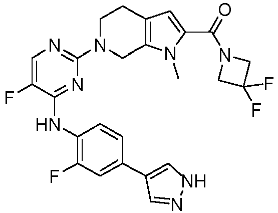 |
| **41** | **L041** | 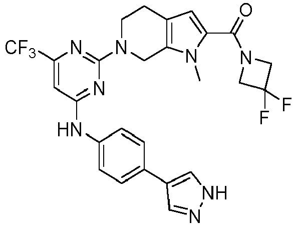 |
| **42** | **L042** | 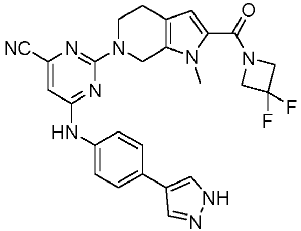 |
| **43** | **L043** | 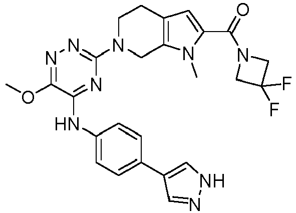 |
| **44** | **L044** | 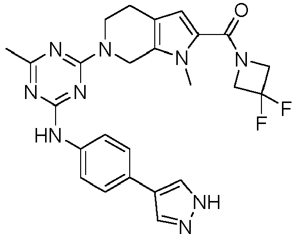 |
| **45** | **L045** | 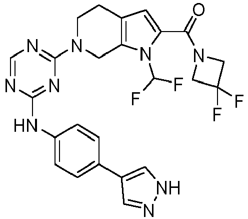 |
| **46** | **L046** | 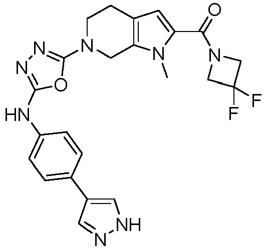 |
| **47** | **L047** | 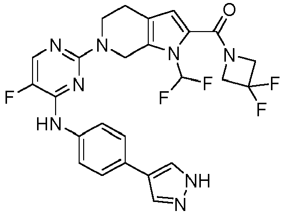 |
| **48** | **L048** | 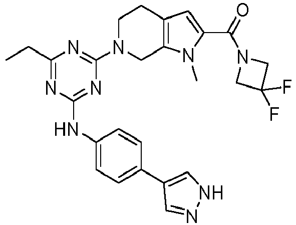 |
| **49** | **L049** | 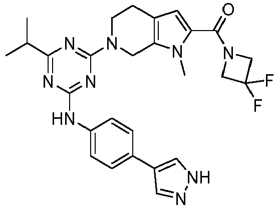 |
| **50** | **L050** | 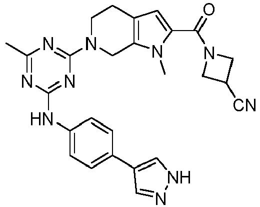 |
| **51** | **L051** | 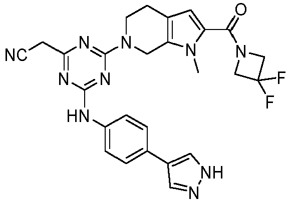 |
| **52** | **L052** | 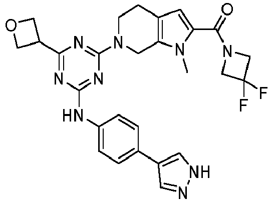 |
| **53** | **L053** | 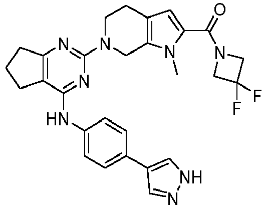 |
| **54** | **L054** | 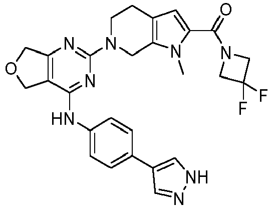 |
| **55** | **L055** | 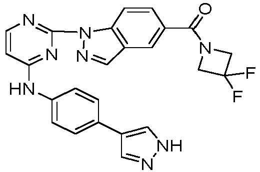 |
| **56** | **L056** | 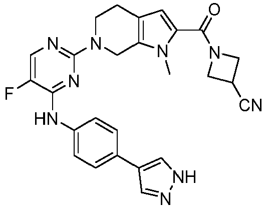 |
| **57** | **L057** | 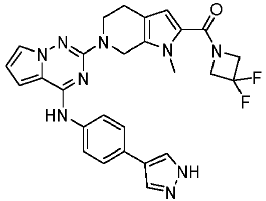 |
| **58** | **L058** | 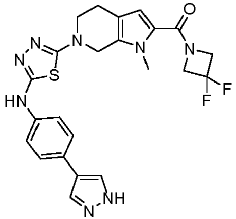 |
| **59** | **L059** | 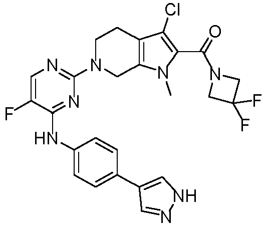 |
| **60** | **L060** | 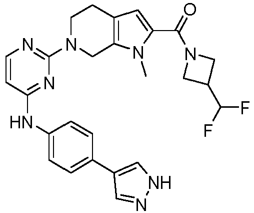 |

The present disclosure also provides a method for preparing the compound represented by formula (I), which comprises at least one of the following schemes: 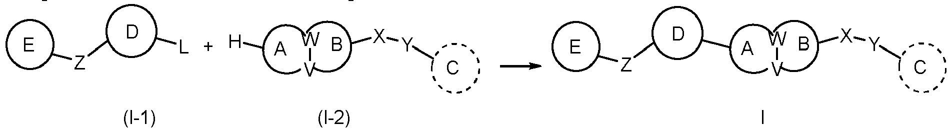 reacting a compound represented by formula (I-1) with a compound represented by formula (I-2) to give the compound represented by formula (I);
wherein, ring A, ring B, ring D, 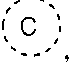 ring E, W, V, X, Y and Z are as defined above;
L is selected from leaving groups, such as halogen.

The present disclosure also provides intermediate compounds for preparing the compound represented by formula (I) of the present disclosure, including formula I-1, formula I-2 and the like.

In a preferred embodiment, formula (I-1) is selected from the following structures: 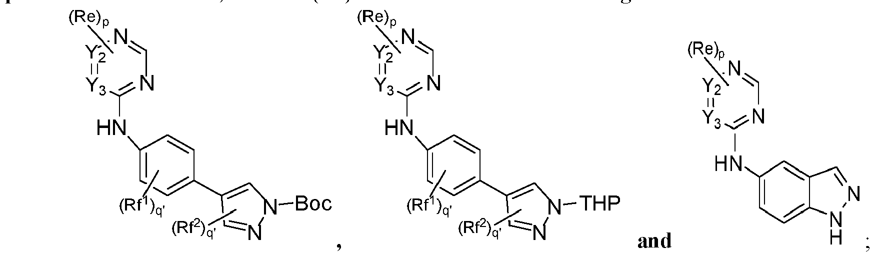 formula (I-2) is selected from the following structures: 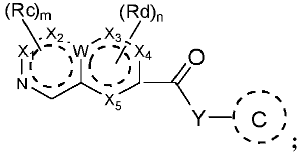 wherein each group is as defined above.

Further preferably, the formula (I-1) is selected from the following structures:

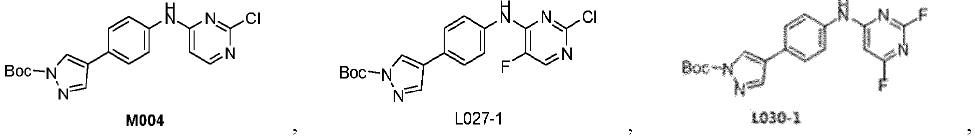

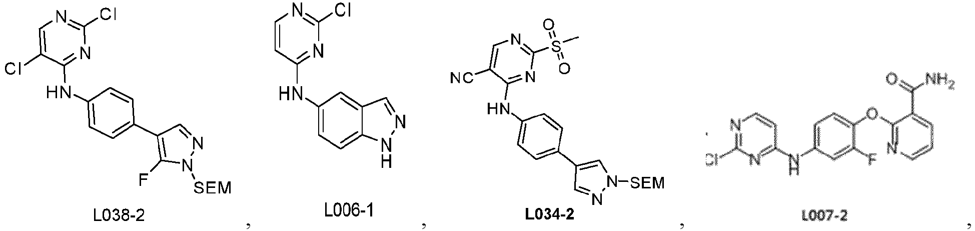

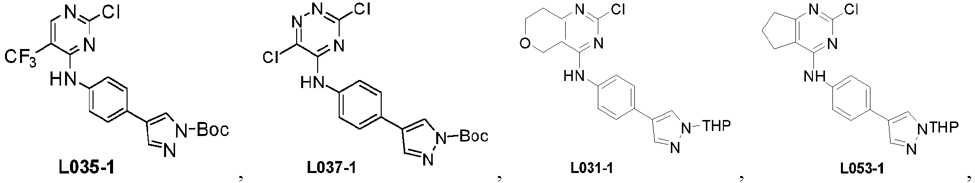

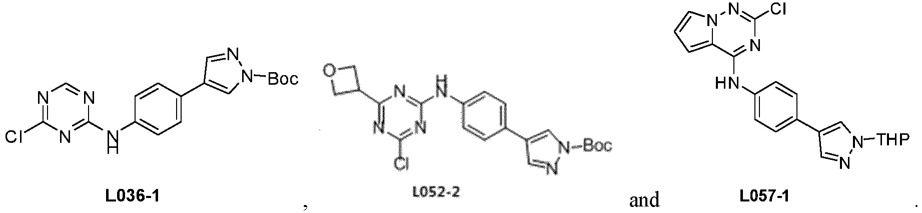

Further preferably, the formula (I-2) may be selected from the following structures:

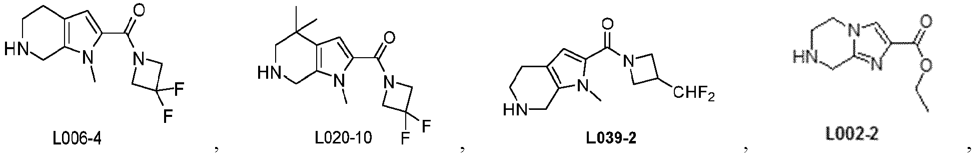

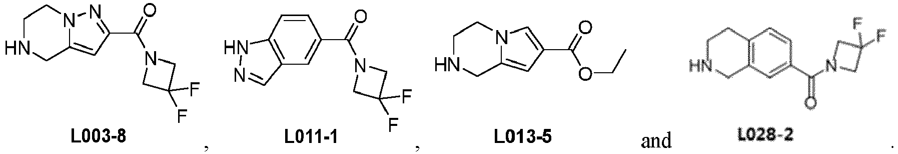

In some embodiments, the present disclosure also provides an intermediate compound for preparing the compound represented by formula (I) of the present disclosure, and the intermediate may be selected from

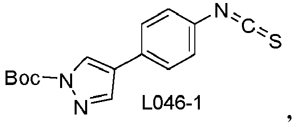

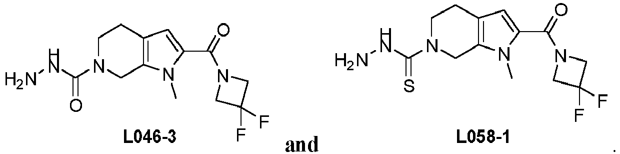

It will be understood by those skilled in the art that the compound represented by formula (I) or the racemate, the stereoisomer, the tautomer, the nitrogen oxide or the isotopically labeled compound thereof can be used as a starting material or an intermediate to prepare the prodrug or the pharmaceutically acceptable salt of the compound represented by formula (I) or the racemate, the stereoisomer, the tautomer, the nitrogen oxide or the isotopically labeled compound thereof. Therefore, the present disclosure also provides use of the compound represented by formula (I) or the racemate, the stereoisomer, the tautomer, the nitrogen oxide or the isotopically labeled compound thereof in preparing the prodrug or the pharmaceutically acceptable salt of the compound represented by formula (I) or the racemate, the stereoisomer, the tautomer, the nitrogen oxide or the isotopically labeled compound thereof.

The present disclosure also provides the following compounds:

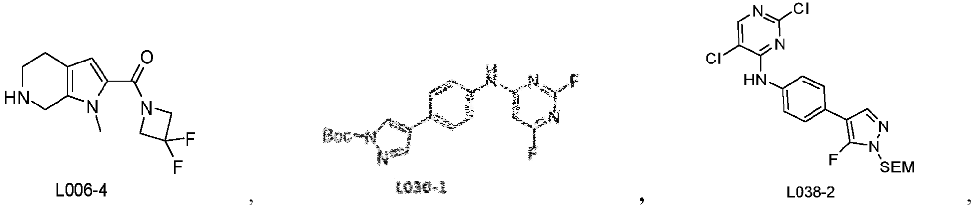

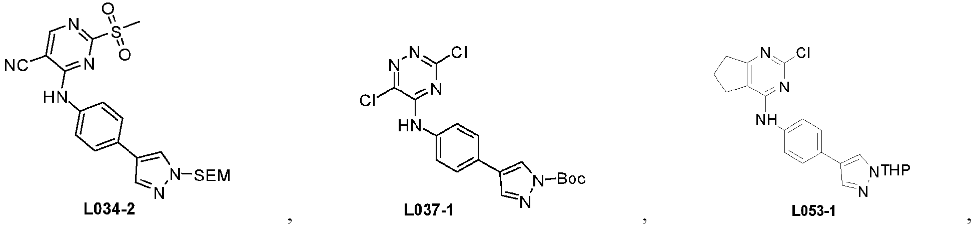

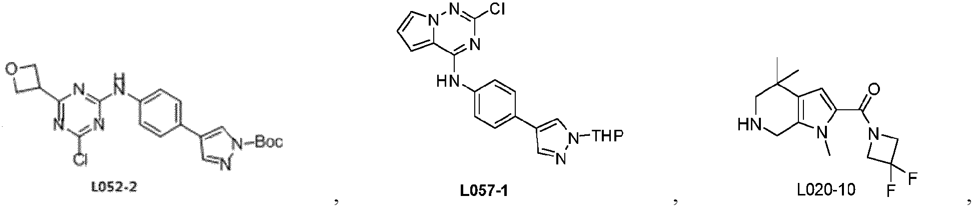

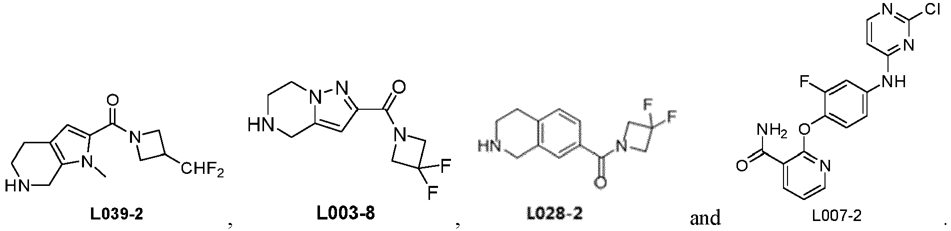

The present disclosure also provides use of at least one of the compound represented by formula (I) and the racemate, the stereoisomer, the tautomer, the nitrogen oxide, the isotopically labeled compound, the solvate, the polymorph, the metabolite, the ester, the pharmaceutically acceptable salt and the prodrug thereof in preparing a medicament, wherein the medicament is an inhibitor of protein kinase.

In particular, the medicament has the function of regulating Rho-kinase.

The medicament can be used for preventing or treating one or more diseases caused by high expression of ROCK or excessive activation of ROCK, such as cardiovascular and cerebrovascular diseases, neurological diseases, fibrosis diseases, ocular diseases, tumors, arterial thrombotic disorders, radiation damage, respiratory diseases, and autoimmune diseases, including atherosclerosis, acute coronary syndrome, hypertension, cerebral vasospasm, cerebral ischemia, ischemic stroke, restenosis, heart disease, heart failure, cardiac hypertrophy, myocardial ischemia-reperfusion injury, diabetes, diabetic nephropathy, cancer, neuronal degeneration (peripheral or central), nerve injury diseases, spinal cord injury, erectile dysfunction, platelet aggregation, leukocyte aggregation, glaucoma, ocular hypertension, asthma, osteoporosis, pulmonary fibrosis (such as idiopathic pulmonary fibrosis), hepatic fibrosis, renal fibrosis, COPD, kidney dialysis (epithelial stability), glomerulosclerosis, neuronal degeneration inflammation, and the like.

The present disclosure also provides a pharmaceutical composition comprising a therapeutically effective amount of at least one of the compound represented by formula (I) and the racemate, the stereoisomer, the tautomer, the nitrogen oxide, the isotopically labeled compound, the solvate, the polymorph, the metabolite, the ester, the pharmaceutically acceptable salt and the prodrug thereof.

Preferably, the pharmaceutical composition may optionally further comprise a pharmaceutically acceptable auxiliary material, such as a carrier or an excipient. As an example, the auxiliary material may be selected from at least one of the following: a disintegrant, a glidant, a lubricant, a diluent, a filler, an adhesive and a colorant.

The pharmaceutical composition of the present disclosure has the function of regulating Rho-kinase. The pharmaceutical composition can be used for preventing or treating one or more diseases caused by high expression of ROCK or excessive activation of ROCK, such as cardiovascular and cerebrovascular diseases, neurological diseases, fibrosis diseases, ocular diseases, tumors, arterial thrombotic disorders, radiation damage, respiratory diseases, and autoimmune diseases, including atherosclerosis, acute coronary syndrome, hypertension, cerebral vasospasm, cerebral ischemia, ischemic stroke, restenosis, heart disease, heart failure, cardiac hypertrophy, myocardial ischemia-reperfusion injury, diabetes, diabetic nephropathy, cancer, neuronal degeneration (peripheral or central), nerve injury diseases, spinal cord injury, erectile dysfunction, platelet aggregation, leukocyte aggregation, glaucoma, ocular hypertension, asthma, osteoporosis, pulmonary fibrosis (such as idiopathic pulmonary fibrosis), hepatic fibrosis, renal fibrosis, COPD, kidney dialysis (epithelial stability), glomerulosclerosis, neuronal degeneration inflammation, and the like.

The present disclosure also provides a method for regulating Rho-kinase function, which comprises administering to an individual in need thereof an effective amount of the compound represented by formula (I) or the racemate, the stereoisomer, the tautomer, the nitrogen oxide, the isotopically labeled compound, the solvate, the polymorph, the metabolite, the ester, the pharmaceutically acceptable salt or the prodrug thereof or the pharmaceutical composition. The method can be used for preventing or treating one or more diseases caused by high expression of ROCK or excessive activation of ROCK.

The present disclosure also provides a method for preventing or treating one or more diseases caused by high expression of ROCK or excessive activation of ROCK, which comprises administering to an individual in need thereof an effective amount of the compound represented by formula (I) or the racemate, the stereoisomer, the tautomer, the nitrogen oxide, the isotopically labeled compound, the solvate, the polymorph, the metabolite, the ester, the pharmaceutically acceptable salt or the prodrug thereof or the pharmaceutical composition. The disease is, for example, cardiovascular and cerebrovascular diseases, neurological diseases, fibrosis diseases, ocular diseases, tumors, arterial thrombotic disorders, radiation damage, respiratory diseases, autoimmune diseases, etc., including atherosclerosis, acute coronary syndrome, hypertension, cerebral vasospasm, cerebral ischemia, ischemic stroke, restenosis, heart disease, heart failure, cardiac hypertrophy, myocardial ischemia-reperfusion injury, diabetes, diabetic nephropathy, cancer, neuronal degeneration (peripheral or central), nerve injury diseases, spinal cord injury, erectile dysfunction, platelet aggregation, leukocyte aggregation, glaucoma, ocular hypertension, asthma, osteoporosis, pulmonary fibrosis (such as idiopathic pulmonary fibrosis), hepatic fibrosis, renal fibrosis, COPD, kidney dialysis (epithelial stability), glomerulosclerosis, neuronal degeneration inflammation, etc. In another aspect, the present disclosure provides a compound represented by formula (I) for use in regulating Rho-kinase function, and the modulating comprises administering to an individual in need thereof an effective amount of one or more compounds disclosed herein or a pharmaceutical composition comprising the compound.

In yet another aspect, the present disclosure provides a compound represented by formula (I) for use in a method for preventing or treating one or more diseases caused by high expression of ROCK or excessive activation of ROCK, and the method comprises administering to an individual in need thereof an effective amount of the compound of formula (I) or the racemate, the stereoisomer, the tautomer, the nitrogen oxide, the isotopically labeled compound, the solvate, the polymorph, the metabolite, the ester, the pharmaceutically acceptable salt or the prodrug thereof or the pharmaceutical composition. The disease is, for example, cardiovascular and cerebrovascular diseases, neurological diseases, fibrosis diseases, ocular diseases, tumors, arterial thrombotic disorders, radiation damage, respiratory diseases, autoimmune diseases, etc., including atherosclerosis, acute coronary syndrome, hypertension, cerebral vasospasm, cerebral ischemia, ischemic stroke, restenosis, heart disease, heart failure, cardiac hypertrophy, myocardial ischemia-reperfusion injury, diabetes, diabetic nephropathy, cancer, neuronal degeneration (peripheral or central), nerve injury diseases, spinal cord injury, erectile dysfunction, platelet aggregation, leukocyte aggregation, glaucoma, ocular hypertension, asthma, osteoporosis, pulmonary fibrosis (such as idiopathic pulmonary fibrosis), hepatic fibrosis, renal fibrosis, COPD, kidney dialysis (epithelial stability), glomerulosclerosis, neuronal degeneration inflammation, etc.

### Definitions and Description

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should fall within the scope of the present specification. In the description and claims of the present application, " 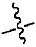 " indicates a connection site.

In the description and claims of the present application, "more" refers to three or more. The term "halogen" refers to F, Cl, Br and I. In other words, F, Cl, Br and I may be described as "halogen" in the specification. The term "C₁₋₂₀ alkyl" preferably refers to a linear or branched saturated monovalent hydrocarbyl having 1-20 carbon atoms, and is preferably C₁₋₆ alkyl. The term "C₁₋₁₂ alkyl" preferably refers to a linear or branched saturated monovalent hydrocarbyl having 1, 2, 3, 4, 5 or 6 carbon atoms. The alkyl is, for example, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof.

The term "C₁₋₂₀ alkoxy" refers to "-O-C₁₋₂₀ alkyl", wherein C₁₋₂₀ alkyl is defined as above. It is preferably "C₁₋₆ alkoxy".

The term "C₃₋₂₀ cycloalkyl" refers to a saturated monovalent monocyclic or bicyclic hydrocarbon ring having 3-20 carbon atoms, and is preferably "C₃₋₆ cycloalkyl". The term "C₃₋₆ cycloalkyl" refers to a saturated monovalent monocyclic or bicyclic hydrocarbon ring having 3, 4, 5 or 6 carbon atoms. The C₃₋₆ cycloalkyl may be a monocyclic hydrocarbon ring, such as cyclopropyl, cyclobutyl, cyclopentyl or cyclohexyl.

The term "3-20 membered heterocyclyl" refers to a saturated monovalent monocyclic or bicyclic hydrocarbon ring containing 1-5 heteroatoms independently selected from N, O and S, and is preferably "3-6 membered heterocyclyl". The term "3-6 membered heterocyclyl" refers to a saturated monovalent monocyclic or bicyclic hydrocarbon ring containing 1-5, preferably 1-3, heteroatoms selected from N, O and S. The heterocyclyl may be connected to the rest of the molecule through any of the carbon atoms or the nitrogen atom (if present). In particular, the heterocyclyl may include, but is not limited to: 4-membered rings such as azetidinyl or oxetanyl; 5-membered rings such as tetrahydrofuranyl, dioxolyl, pyrrolidinyl, imidazolidinyl, pyrazolidinyl or pyrrolinyl; or 6-membered rings such as tetrahydropyranyl, piperidyl, morpholinyl, dithianyl, thiomorpholinyl, piperazinyl or trithianyl. According to the present disclosure, the heterocyclyl is non-aromatic.

The term "C₆₋₂₀ aryl" refers to an aromatic or partially aromatic monovalent monocyclic, bicyclic or tricyclic hydrocarbon ring having 6-20 carbon atoms, and is preferably "C₆₋₁₂ aryl". The term "C₆₋₁₂ aryl" preferably refers to an aromatic or partially aromatic monovalent monocyclic, bicyclic or tricyclic hydrocarbon ring having 6, 7, 8, 9, 10, 11 or 12 carbon atoms ("C₆₋₁₂ aryl"), in particular a ring having 6 carbon atoms ("C₆ aryl"), such as phenyl or biphenyl, a ring having 9 carbon atoms ("C₉ aryl"), such as indanyl or indenyl, or a ring having 10 carbon atoms ("C₁₀ aryl"), such as tetrahydronaphthyl, dihydronaphthyl or naphthyl. The term "5-20 membered heteroaryl" refers to a monovalent aromatic monocyclic, bicyclic or tricyclic ring system that has 5-20 ring atoms and comprises 1-5 heteroatoms independently selected from N, O and S, such as "5-12 membered heteroaryl". The term "5-12 membered heteroaryl" refers to a monovalent aromatic monocyclic, bicyclic or tricyclic ring system that has 5, 6, 7, 8, 9, 10, 11 or 12 ring atoms, in particular 5, 6, 9 or 10 carbon atoms, contains 1-5, preferably 1-3, heteroatoms independently selected from N, O and S, and may be benzo-fused in each case. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4*H-*pyrazolyl and the like and benzo derivatives thereof, such as benzofuranyl, benzothienyl, benzoxazolyl, benzoisoxazolyl, benzimidazolyl, benzotriazolyl, indazolyl, indolyl, and isoindolyl; or pyridinyl, pyridazinyl, pyrimidinyl, pyrazinyl, triazinyl and the like and benzo derivatives thereof, such as quinolyl, quinazolinyl, and isoquinolyl; or azocinyl, indolizinyl, purinyl and the like and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like.

Unless otherwise stated, the heterocyclyl, heteroaryl or heteroarylene includes all possible isomeric forms thereof, e.g., positional isomers thereof. Accordingly, for some illustrative non-limiting examples, pyridinyl or pyridinylene includes pyridin-2-yl, pyridinylene-2-yl, pyridin-3-yl, pyridinylene-3-yl, pyridin-4-yl, and pyridinylene-4-yl; thienyl or thienylene includes thien-2-yl, thien-2-ylene, thien-3-yl, and thien-3-ylene.

It should be noted that when a range of values is referred to in the definitions of above groups, for example, a range of values in C₁₋₂₀, 3-20, C₆₋₂₀, 5-20 or the like indicate both endpoints of the range and any integer between the two endpoints, and is not limited to including only two endpoints.

Unless otherwise indicated, the term "leaving group", as used herein, shall refer to a charged or uncharged atom or group that is liberated during a substitution or replacement reaction. Suitable examples include, but are not limited to, H, F, Br, Cl, I, mesylate group, tosylate group, and the like.

In any method for preparing the compound disclosed herein, it may be necessary and/or desirable to protect sensitive or reactive groups on any molecule concerned. This can be achieved by conventional protective groups, as described in textbooks or in reference books in the art. The protective group may be removed at a convenient subsequent stage using methods known in the art. Those skilled in the art will recognize that, other reagents, including but not limited to Pd/C, Pd(OH)₂, PdCl₂, Pd(OAc)₂/Et₃SiH, Raney nickel, an appropriately selected acid, an appropriately selected base, fluoride, and the like, may be used in this deprotection step depending on the particular protective group.

The target compound may be isolated according to known methods, for example by extraction, filtration or column chromatography.

According to the molecular structure, the compounds disclosed herein may be chiral and may therefore exist in various enantiomeric forms. These compounds may therefore exist in racemic or optically active form. The compounds disclosed herein or intermediates thereof may be separated into enantiomers by chemical or physical methods well known to those skilled in the art, or used in this form for synthesis. In the case of racemic amines, diastereoisomers are manufactured from mixtures by reaction with optically active resolving agents. Examples of suitable resolving agents are optically active acids such as *R*- or *S*-tartaric acid, diacetyltartaric acid, dibenzoyltartaric acid, mandelic acid, malic acid, lactic acid, suitable *N*-protected amino acids (e.g., *N*-benzoylproline or *N*-benzenesulfonylproline) or various optically active camphorsulfonic acids. Enantiomeric resolution by chromatography can be advantageously performed with the aid of optically active resolving agents, such as dinitrobenzoylphenylglycine, cellulose triacetate or other carbohydrate derivatives or chirally derivatized methacrylate polymers immobilized on silica gel. Suitable eluents for this purpose are mixtures of solvent containing water or alcohol, for example, hexane/isopropanol/acetonitrile.

Those skilled in the art will appreciate that not all nitrogen-containing heterocycles can form N-oxides, as nitrogen needs to have available lone pairs of electrons used for oxidation to oxides; those skilled in the art will identify nitrogen-containing heterocycles capable of forming N-oxides. Those skilled in the art will also recognize that tertiary amines are capable of forming N-oxides. Synthetic methods for preparing N-oxides of heterocycles and tertiary amines are well known to those skilled in the art and include oxidation by peroxy acids such as peroxyacetic acid and *m*-chloroperbenzoic acid (MCPBA), hydrogen peroxide, alkyl hydroperoxides such as *tert*-butyl hydroperoxide, sodium perborate, and dioxiranes such as dimethyldioxirane. These methods for preparing N-oxides have been widely described and reviewed in the literature.

A pharmaceutically acceptable salt may be, for example, acid addition salts of the compounds disclosed herein having a nitrogen atom in the chain or ring and having sufficient basicity, for example, acid addition salts formed with the following inorganic acids: hydrochloric acid, hydrofluoric acid, hydrobromic acid, hydroiodic acid, sulfuric acid, pyrosulfuric acid, phosphoric acid or nitric acid; hydrosulfates; or acid addition salts formed with the following organic acids: formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, peroxosulfuric acid, 3-phenylpropionic acid, picric acid, pivalic acid, 2-hydroxyethanesulfonic acid, itaconic acid, sulfamic acid, trifluoromethanesulfonic acid, dodecylsulfuric acid, ethanesulfonic acid, benzenesulfonic acid, *p*-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, *D*-gluconic acid, mandelic acid, ascorbic acid, glucoheptoic acid, glycerophosphoric acid, aspartic acid, sulfosalicylic acid, hemisulfuric acid, or thiocyanic acid.

In addition, another suitable pharmaceutically acceptable salt of the compounds disclosed herein having sufficient acidity is an alkali metal salt (e.g., sodium salt or potassium salt), an alkaline earth metal salt (e.g., calcium salt or magnesium salt), an ammonium salt, or a salt formed with an organic base which provides a physiologically acceptable cation, for example, a salt formed with: a sodium ion, a potassium ion, *N-*methylglucamine, dimethylglucamine, ethylglucamine, lysine, dicyclohexylamine, 1,6-hexanediamine, ethanolamine, glucosamine, meglumine, sarcosine, serinol, trihydroxymethylaminomethane, aminopropanediol, or 1-amino-2,3,4-butanetriol. As an example, the pharmaceutically acceptable salts include salts formed from the group -COOH with the following: a sodium ion, a potassium ion, a calcium ion, a magnesium ion, N-methylglucamine, dimethylglucamine, ethylglucamine, lysine, dicyclohexylamine, 1,6-hexanediamine, ethanolamine, glucosamine, meglumine, sarcosine, serinol, trihydroxymethylaminomethane, aminopropanediol, or 1-amino-2,3,4-butanetriol.

In addition, the basic nitrogen-containing groups may be quaternized with the following agents: lower alkyl halides such as methyl, ethyl, propyl and butyl chlorides, bromides and iodides; dialkyl sulfates such as dimethyl sulfate, diethyl sulfate, dibutyl sulfate, and dipentyl sulfate; long chain halides such as decyl, lauryl, myristyl and stearyl chlorides, bromides and iodides; and aralkyl halides such as benzyl and phenethyl bromides. As an example, pharmaceutically acceptable salts include hydrochloride, sulfate, nitrate, bisulfate, hydrobromide, acetate, oxalate, citrate, mesylate, formate, meglumine, and the like.

Since the compounds disclosed herein may have a plurality of salt-forming sites, the "pharmaceutically acceptable salt" includes not only a salt formed at 1 salt-forming site of the compounds disclosed herein but also salts formed at 2, 3 or all of the salt-forming sites thereof. For this purpose, in the "pharmaceutically acceptable salt", the molar ratio of the compound of formula (I) to a radical ion (anion) of an acid or a cation of a base required for salt formation may vary within a wide range, and may be, for example, 4:1 to 1:4, such as 3:1, 2:1, 1:1, 1:2, 1:3 or the like.

According to the present disclosure, the pharmaceutically acceptable anions include anions selected from those generated by the ionization of inorganic or organic acids. The "inorganic acid" includes, but is not limited to, hydrochloric acid, hydrofluoric acid, hydrobromic acid, hydroiodic acid, sulfuric acid, pyrosulfuric acid, phosphoric acid, or nitric acid. The "organic acid" includes, but is not limited to, formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, peroxosulfuric acid, 3-phenylpropionic acid, picric acid, pivalic acid, 2-hydroxyethanesulfonic acid, itaconic acid, sulfamic acid, trifluoromethanesulfonic acid, dodecylsulfuric acid, ethanesulfonic acid, benzenesulfonic acid,*p*-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, *D*-gluconic acid, mandelic acid, ascorbic acid, glucoheptoic acid, glycerophosphoric acid, aspartic acid, sulfosalicylic acid, hemisulfuric acid, or thiocyanic acid.

The term "tautomer" refers to functional isomers resulting from the rapid movement of an atom in a molecule between two positions. The compounds disclosed herein may exhibit the tautomerism. Tautomeric compounds may exist in two or more interconvertible forms. Prototropic tautomers result from the migration of a covalently bonded hydrogen atom between two atoms. Tautomers generally exist in an equilibrium form. Trying to separate a single tautomer usually leads to a mixture, the physicochemical properties of which are consistent with the mixture of the compound. The position of the equilibrium depends on the chemical properties of the molecule. For example, in many aliphatic aldehydes and ketones such as acetaldehyde, the keto form predominates; whereas in phenol, the enol form predominates. The present disclosure comprises all tautomeric forms of the compound.

The term "effective amount" or "therapeutically effective amount" refers to an amount of the compounds disclosed herein sufficient to effect the intended use, including but not limited to the treatment of a disease as defined below. The therapeutically effective amount may vary depending on the following factors: the intended use *(in vitro* or *in vivo),* or the subject and diseases or conditions being treated, such as weight and age of the subject, severity of the diseases or conditions and mode of administration, which can be readily determined by one of ordinary skill in the art. The specific dosage will vary depending on the following factors: the selected particular compound, the dosage regimen to be followed, whether to administer in combination with other compounds, the schedule of administration, the tissue to be administered and the physical delivery system carried.

The term "auxiliary material" refers to a pharmaceutically acceptable inert ingredient. Examples of types of excipients include, without limitation, adhesives, disintegrants, lubricants, glidants, stabilizers, fillers, diluents, and the like. Excipients are capable of enhancing the handling characteristics of the pharmaceutical formulation, i.e., making the formulation more amenable to direct compression by increasing flowability and/or adhesiveness. Examples of typical pharmaceutically acceptable carriers suitable for use in the above formulations include: saccharides, such as lactose, sucrose, mannitol, and sorbitol; starches, such as corn starch, tapioca starch and potato starch; cellulose and its derivatives, such as sodium carboxymethylcellulose, ethyl cellulose and methyl cellulose; calcium phosphates, such as dicalcium phosphate and tricalcium phosphate; sodium sulfate; calcium sulfate; polyvinylpyrrolidone; polyvinyl alcohol; stearic acid; alkaline earth metal stearate, such as magnesium stearate and calcium stearate; vegetable oils such as peanut oil, cottonseed oil, sesame oil, olive oil and corn oil; nonionic, cationic and anionic surfactants; a glycol polymer; fatty alcohols; and grain hydrolysis solids and other nontoxic compatible auxiliary materials commonly available in pharmaceutical formulations, such as fillers, adhesives, disintegrants, buffers, preservatives, antioxidants, lubricants, and colorants.

### Beneficial effects of the present disclosure:

The compound provided herein has excellent **ROCK** inhibitory activity. Particularly, the compound has better selective inhibition on **ROCK2** kinase, and in addition, the compound disclosed herein has relatively good safety and metabolic stability and high bioavailability. Finally, the compound disclosed herein is simple in preparation and easy to purify, and therefore has good application prospects.

### DETAILED DESCRIPTION

The compounds of the general formulas disclosed herein and the preparation method and use thereof will be described in detail with reference to the following examples. The following examples are merely exemplary illustration and explanation of the present disclosure, and should not be construed as limiting the protection scope of the present disclosure. All techniques implemented based on the content of the present disclosure described above are encompassed within the protection scope of the present disclosure.

Unless otherwise stated, the starting materials and reagents used in the following examples are all commercially available products or can be prepared using known methods.

### Nouns and reagents denoted

The nouns used in the following detailed description of the experiments represent (unless otherwise stated) the following reagents, respectively:
DMF: *N*,*N*-dimethylformamide; DCM: dichloromethane; TEA: triethylamine; DMAP: 4-dimethylaminopyridine; DIEA: 7V,7V-diisopropylethylamine; THF: tetrahydrofuran; DME: ethylene glycol dimethyl ether; CDI: *N*,*N*'-carbonyldiimidazole; MsCl: methylsulfonyl chloride; DIPEA: *N,N-*diisopropylethylamine; HATU: 2-(7-azabenzotriazol)-*N*,*N*,*N*',*N*'-tetramethyluronium hexafluorophosphate;
DMSO: dimethyl sulfoxide; EA: ethyl acetate; MeOH: methanol; EtOH: ethanol; MeCN: acetonitrile; NCS: A-chlorosuccinimide; *n*-BuOH: n-butanol; DCE: 1,2-dichloroethane; NMAC: *N*,*N*-dimethylacetamide;
EDCI: 1-ethyl-(3-dimethylaminopropyl)carbodiimide hydrochloride; IPA: isopropanol; mCPBA, *m-*chloroperoxybenzoic acid; nBuLi: n-butyl lithium; NMP: A-methylpyrrolidone; TFA: trifluoroacetic acid;
*t*-BuOK: potassium tert-butoxide; *t*-BuOMe: methyl *tert-butyl* ether; xantphos: 5-bis(diphenylphosphino)-9,9-dimethylxanthene; and DHP: 3,4-dihydro-2*H*-pyran.

### <Preparation Examples>

### Preparation of Intermediate 1 3,3-difluoro-N-methylcyclobutan-1-amine hydrochloride (M001)

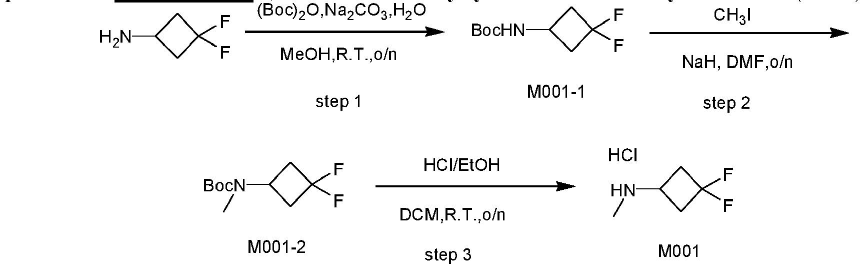

### (1)Preparation of compound tert-butyl (3,3-difluorocyclobutan)carbamate (M001-1)

3,3-difluorocyclobutan-1-amine (1.00 g, 9.34 mmol)was dissolved in a mixed solvent of methanol (10 mL) and saturated aqueous sodium carbonate solution (10 mL), and di-*tert*-butyl dicarbonate (2.45 g, 11.20 mmol) was added to the system at room temperature. The reaction system was stirred overnight at room temperature, and the product was precipitated from the system. The mixture was filtered, and the filter cake was concentrated by rotary evaporation to give *tert*-butyl (3,3-difluorocyclobutan)carbamate in the form of a white solid (1.35 g, 70.2% yield, crude product).

¹H NMR (301 MHz, CD₃OD) δ 3.89 (m, 1H), 2.93 - 2.74 (m, 2H), 2.58 - 2.38 (m, 2H), 1.42 (s, 9H).

### (2)Preparation of compound tert-butyl (3,3-difluorocyclobutan)(methyl)carbamate (M001-2)

*Tert*-butyl (3,3-difluorocyclobutan)carbamate (900 mg, 4.34 mmol) was dissolved in anhydrous *N,N-*dimethylformamide (10 mL) under nitrogen atmosphere, and the mixture was cooled to 0 °C. Sodium hydride (60%, 208 mg, 5.21 mmol) was added in batches, and the resulting mixture was stirred at temperature for 0.5 h and placed in an ice water bath, followed by dropwise addition of iodomethane (1.23 g, 8.69 mmol). The mixture was stirred overnight at room temperature. Water (30 mL) was added slowly to quench the reaction, and ethyl acetate (30 mL × 3) was added for extraction. The organic phases were combined, dried over anhydrous sodium sulfate, filtered and concentrated to give *tert-butyl* (3,3-difluorocyclobutan)(methyl)carbamate in the form of a light yellow solid (1.10 g, crude product).

¹H NMR (301 MHz, CD₃OD) δ 4.35 - 4.27 (m, 1H), 2.82 (s, 3H), 2.75 (dt, J = 15.5,8.5 Hz, 4H), 1.45 (s, 9H). Tert-butyl (3,3-difluorocyclobutan)(methyl)carbamate (1.10 g, crude product) was dissolved in dichloromethane (10 mL), and a solution of hydrogen chloride in ethanol (33%, 3 mL) was added dropwise. The mixture was stirred overnight at room temperature. The mixture was then concentrated under reduced pressure to give 3,3-difluoro-*N-*methylcyclobutane-1-amine hydrochloride in the form of a brown-yellow solid (800 mg, the crude product being overweight). LC-MS [M+H]⁺: 122.1.

### Preparation of Intermediate 2 tert-butyl 4-(4-aminophenyl)-1H-pyrazole-1-carboxylate (M002)

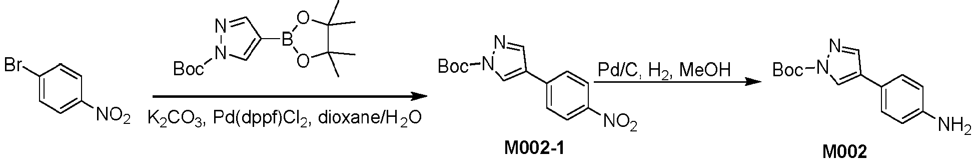

### 1) Preparation of tert-butyl 4-(4-nitrophenyl)-1H-pyrazole-1-carboxylate (M002-1)

Tert-butyl (4-(4,4,5-trimethyl-1,3,2-dioxaborolan-2-yl)pyrazol-1-yl)carboxylate (58.8 g, 0.2 mol), 1-bromo-4-nitrobenzene (20 g, 0.1 mol), anhydrous potassium carbonate (41.5 g, 0.3 mol) and (1,1'-bis(diphenylphosphino)ferrocene)dichloropalladium(II) (7.3 g, 10 mmol) were added to 1,4-dioxane: water = 30:1 (207 mL), and the reaction system was purged 3 times with nitrogen. The mixture was then stirred for 5 h at 100 °C in an oil bath. After the reaction was completed as monitored by TLC, the reaction was stopped. The mixture was filtered under reduced pressure, and the filtrate was concentrated. The residue was purified by column chromatography (petroleum ether:ethyl acetate = 4:1) to give a yellow solid (16 g, 46% yield), MS (M+1-100)⁺ = 190.05, which was product M002-1 according to the hydrogen spectrum.

### 2) Preparation of tert-butyl 4-(4-aminophenyl)-1H-pyrazole-1-carboxylate (M002)

*Tert*-butyl 4-(4-nitrophenyl)-1*H*-pyrazole-1-carboxylate (16 g, 55.3 mol) was dissolved in methanol (160 mL), and palladium on carbon (3.2 g) was added. A hydrogen balloon was provided, and the reaction system was purged with hydrogen and reacted at 45 °C for 16 h. After the reaction was completed as detected by TLC, celite was added and suction filtration was carried out under reduced pressure, and the filtrate was concentrated to give a yellow crude product, which was purified by column chromatography (petroleum ether:ethyl acetate = 3:1) to give an off-white solid (9.6 g, 60% yield). MS(M +1)⁺ = 260.15.

### Intermediate 3 4-(1-(tetrahydro-2H-pyran-2-yl)-1H-pyrazol-4-yl)aniline (M003)

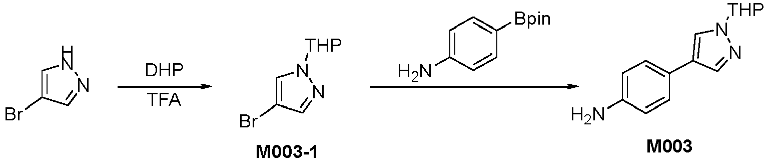

### 1)Preparation of 4-bromo-1-(tetrahydro-2Hpyran-2-yl)-1Hpyrazole (M003-1)

3-bromopyrazole (5 g, 34.02 mmol) was dissolved in 3,4-dihydro-2*H*-pyran (25 mL), and trifluoroacetic acid (387.9 mg, 3.4 mmol) was slowly added dropwise. After the dropwise addition was completed, the mixture was warmed to 80 °C and reacted overnight. Samples were taken and sent for detection by LCMS, and the starting material yielded a large amount of product. Post-treatment: saturated aqueous NaHCO₃ solution was added to adjust pH to about 8, and then EA (20 mL × 3) was added for extraction. The organic phases were combined, washed with saturated brine, dried, filtered and concentrated to give a yellow oil, which was directly used in the next step. LC-MS [M+H]⁺ = 233.00.

### 2) Preparation of 4-(1-(tetrahydro-2H-pyran-2-yl)-1H-pyrazol-4-yl)aniline (M003)

4-bromo-1-(tetrahydro-2*H*-pyran-2-yl)-1*H*-pyrazole (7.86 g, 34.01 mmol), 4-nitro borate (8.94 g, 40.81 mmol), potassium carbonate (9.4 g, 68.02 mmol) and Pd(dppf)Cl₂ (2.49 g, 3.4 mmol) were dissolved in dioxane/water (70 mL/10 mL), and the mixture was reacted overnight at 80 °C under nitrogen atmosphere. Samples were taken and sent for detection by LCMS, which showed that the starting material was completely reacted. After the reaction was completed, aqueous ammonium chloride solution (50 mL) was added, and ethyl acetate (50 mL × 3) was added for extraction. The organic phase was washed with saturated brine (30 mL × 2), dried over anhydrous sodium sulfate, filtered and concentrated. The resulting crude product was separated by silica gel column chromatography to give a dark green solid (5 g, 60% yield). LC-MS [M+H]⁺ = 244.15.

### Preparation of intermediates 4 tert-butyl-4-(4-((2-chloropyrimidin-4-yl)amino)phenyl)-1H-pyrazole-1-carboxylate (M004) and N-(4-(1H-pyrazol-4-yl)phenyl)-2-chloropyrimidin-4-amine (M004')

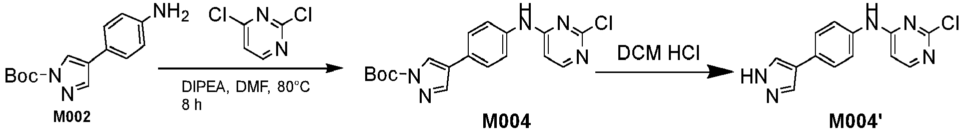

### 1) Preparation of compound tert-butyl 4-(4-((2-chloropyrimidin-4-yl)amino)phenyl)-1H-pyrazole-1-carboxylate (M004):

*Tert*-butyl 4-(4-aminophenyl)-1*H*-pyrazole-1-carboxylate (5.64 g, 21.69 mmol)and 2,4-dichloropyrimidine (4.85 g, 1.44 mmol) were dissolved in *N*,*N*-dimethylformamide (90 mL), and *N*,*N*-diisopropylethylamine (8.39 g, 65.07 mmol) was added. The mixture was reacted at 80 °C for 8 h under nitrogen atmosphere. The reaction liquid was poured into water (200 mL), and ethyl acetate (100 mL × 2) was added for extraction. The organic phases were combined, washed with saturated brine (80 mL ×3), dried over anhydrous sodium sulfate, filtered and concentrated. The resulting crude product was separated by silica gel column chromatography (dichloromethane:ethyl acetate = 50:1-5:1) to give a light yellow solid (4.40 g, 54% yield). LC-MS [M+H]⁺: 371.7.

### 2) Preparation of N-(4-(1H-pyrazol-4-yl)phenyl)-2-chloropyrimidin-4-amine (M004')

*Tert*-butyl 4-(4-((2-chloropyrimidin-4-yl)amino)phenyl)-1*H*-pyrazole-1-carboxylate was dissolved in anhydrous dichloromethane, and a solution of hydrogen chloride in ethanol (33%) was added. The mixture was stirred at room temperature for 2 h. Then the mixture was concentrated under reduced pressure to give *N*-(4-(1*H*-pyrazol-4-yl)phenyl)-2-chloropyrimidin-4-amine, MS (M+1)⁺ = 272.4.

### Example 1. Preparation of Compound 1 6-(4-((4-(1H pyrazol-4-yl)phenyl)amino)pyrimidin-2-yl)-1-methyl-4,5,6,7-tetrahydro-1H-pyrrolo[2,3-c]pyridine-2-carboxylic acid (L001)

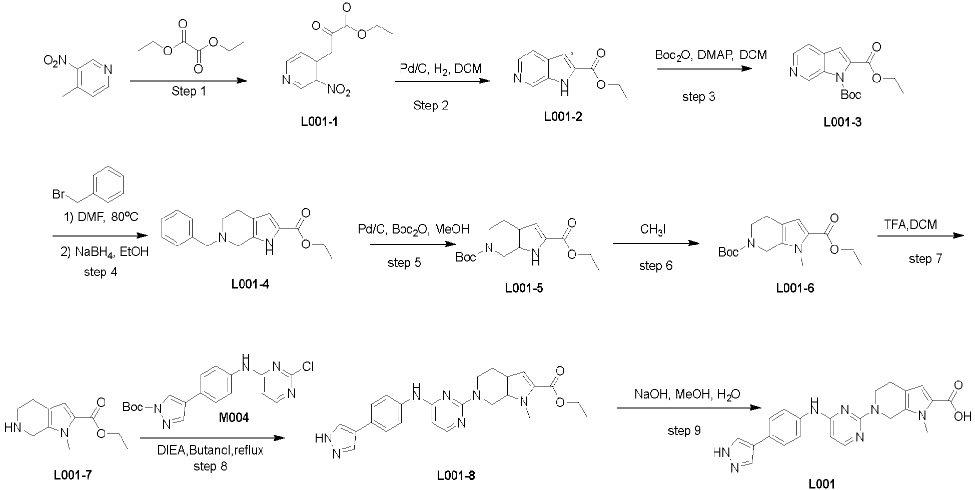

### 1.1 Preparation of compound ethyl 3-(3-nitropyridin-4-yl)-2-oxopropionate (L001-1)

4-methyl-3-nitropyridine (10.0 g, 72.0 mmol) was dissolved in diethyl oxalate (50 mL) under nitrogen atmosphere, and DBU (12.7 g, 83.0 mmol) was added to the reaction liquid. The mixture was stirred overnight at room temperature. Ice water (30.0 g) was added to the reaction system to quench the reaction, and 1 N diluted hydrochloric acid was added to adjust the pH to 4-5. The resulting mixture was filtered, and the filter cake was washed with ethyl acetate (100 mL × 2) and dried in an oven to give a red solid (18.0 g), and the crude product was directly used in the next step. LC-MS [M+H]⁺: 239.1.

### 1.2 Preparation of compound ethyl 1H-pyrrolo[2,3-c]pyridine-2-carboxylate (L001-2)

Ethyl 3-(3-nitropyridin-4-yl)-2-oxopropionate (17.0 g, 70.0 mmol) was dissolved in anhydrous dichloromethane (200 mL) under hydrogen atmosphere, and palladium on carbon (3.4 g) was added to the reaction liquid. The mixture was reacted overnight at room temperature. Then the mixture was filtered, and the filtrate was concentrated under reduced pressure. The residue was separated by silica gel column chromatography (PE/EA = 1/1) to give a yellow oily liquid (6.0 g, 43.8% two-step yield). LC-MS [M+H]⁺: 191.1.

### 1.3 Preparation of compound ethyl 1-tert-butoxycarbonyl-pyrrolo[2,3-c]pyridine-2-carboxylate (L001-3)

Ethyl 1*H*-pyrrolo[2,3-*c*]pyridine-2-carboxylate (6.0 g, 31.5 mmol) was dissolved in anhydrous dichloromethane (50 mL) under nitrogen atmosphere, and Boc anhydride (7.6 g, 34.7 mmol) and DMAP (384 mg, 3.2 mmol) were added to the reaction liquid. The mixture was stirred overnight at room temperature. Then the mixture was concentrated under reduced pressure, and the crude product was separated by silica gel column chromatography (PE/EA = 3/1) to give a yellow oily liquid (7.5 g, 82.4% yield). LC-MS [M+H]⁺: 291.0.

### 1.4 Preparation of compound ethyl 6-benzyl-4,5,6,7-tetrahydro-1H-pyrrolo[2,3-c]pyridine-2-carboxylate (L001-4)

Ethyl 1-*tert*-butoxycarbonyl-pyrrolo[2,3-e]pyridine-2-carboxylate (7.2 g, 24.7 mmol) was dissolved in anhydrous DMF (50 mL) under nitrogen atmosphere, and benzyl bromide (4.2 g, 24.7 mmol) was added. The mixture was reacted at 80 °C for 4 h, and then concentrated under reduced pressure. The resulting solid was dissolved in ethanol (50 mL), and sodium borohydride (934 mg, 24.7 mmol) was added to the reaction liquid. The mixture was reacted at room temperature for 3 h. Water (100 mL) was added to quench the reaction, and ethyl acetate (100 mL × 3) was added for extraction. The organic phase was washed with saturated brine (100 mL), dried over anhydrous sodium sulfate, filtered and concentrated, and the resulting crude product was separated by silica gel column chromatography (PE/EA = 3/1)to give a yellow oily liquid (3.5 g, 49.8% yield). LC-MS [M+H]⁺: 285.1.

### 1.5 Preparation of compound ethyl 6-tert-butoxycarbonyl-4,5,7-trihydro-1H-pyrrolo[2,3-e]pyridine-2-carboxylate (L001-5)

Ethyl 6-benzyl-4,5,6,7-tetrahydro-1*H-*pyrrolo[2,3-*c*]pyridine-2-carboxylate (3.5 g, 12.3 mmol) was dissolved in methanol (40 mL), and Boc anhydride (4.0 g, 18.5 mmol) and palladium on carbon (700 mg) were added. The mixture was reacted overnight at room temperature under hydrogen atmosphere. Then the mixture was filtered and concentrated, and the residue was separated by silica gel column chromatography (PE/EA = 5/1) to give a yellow oily liquid (1.8 g, 50.0% yield). MS [M/2+H]⁺ = 295.1.

### 1.6 Preparation of compound ethyl 6-tert-butoxycarbonyl-1-methyl-4,5,7-trihydro-1H-pyrrolo[2,3-c]pyridine-2-carboxylate (L001-6)

Ethyl 6-tert-butoxycarbonyl-4,5,7-trihydro-1*H*-pyrrolo[2,3-e]pyridine-2-carboxylate (1.8 g, 6.1 mmol) was dissolved in tetrahydrofuran (20 mL) under nitrogen atmosphere, and the reaction system was then cooled to 0 °C. Sodium hydride (364 mg, 9.1 mmol, 60%) was added to the reaction system, and the resulting mixture was stirred at this temperature for 30 min, followed by dropwise addition of iodomethane (1.7 g, 12.2 mmol). The mixture was reacted at room temperature for 2 h. Saturated ammonium chloride (20 mL) was added to quench the reaction, and ethyl acetate (30 mL × 3) was added for extraction. The organic phases were combined, washed with saturated brine (50 mL), dried over anhydrous sodium sulfate, filtered and concentrated to give a yellow oily liquid (1.8 g, crude product). LC-MS [M+H]⁺: 308.9.

### 1.7 Preparation of compound ethyl 1-methyl-4,5,6,7-tetrahydro-1H-pyrrolo[2,3-e]pyridine-2-carboxylate (L001-7)

Ethyl 6-*tert*-butoxycarbonyl-4,5,7-trihydro-1*H*-pyrrolo[2,3-e]pyridine-2-carboxylate (1.8 g, 5.8 mmol) was dissolved in anhydrous dichloromethane (20 mL), and trifluoroacetic acid (5 mL) was added. The mixture was reacted at room temperature for 3 h. Then the mixture was concentrated under reduced pressure, saturated sodium bicarbonate solution was slowly added dropwise to adjust pH to 8-9, and dichloromethane (20 mL × 3) was added for extraction. The organic phase was washed with saturated brine (50 mL), dried over anhydrous sodium sulfate, filtered and concentrated, and the resulting crude product was purified by silica gel column chromatography (DCM/MeOH = 15/1) to give a yellow oily liquid (900 mg, 71% two-step yield). LC-MS [M+H]⁺: 209.1.

### 1.8 Preparation of compound ethyl 6-(4-((4-(1H-pyrazol-4-yl)phenyl)amino)pyrimidin-2-yl)-1-methyl-4,5,6,7-tetrahydro-1H-pyrrolo[2,3-c]pyridine-2-carboxylate (L001-8)

Tert-butyl 4-(4-((2-chloropyrimidin-4-yl)amino)phenyl)-1*H-*pyrazole-1-carboxylate (1342 mg, 3.6 mmol) was dissolved in *n*-butanol (10 mL), and ethyl-1-methyl-4,5,6,7-tetrahydro-1*H*-pyrrolo[2,3-*c*]pyridine-2-carboxylate (500 mg, 2.4 mmol) and DIEA (930 mg, 7.2 mmol) were added to the reaction liquid. The mixture was warmed to 120 °C and reacted overnight. Water (40 mL) was added to quench the reaction, and ethyl acetate (30 mL × 3) was added for extraction. The organic phases were combined, washed with saturated brine (50 mL), dried over anhydrous sodium sulfate, filtered and concentrated. The resulting crude product was purified by silica gel column chromatography (PE/EA = 1/1) to give a yellow oily liquid (900 mg, 84.5% yield). LC-MS [M+H]⁺: 444.1.

### 1.9 Preparation of compound 6-(4-((4-(1H-pyrazol-4-yl)phenyl)amino)pyrimidin-2-yl)-1-methyl-4,5,6,7-tetrahydro-1H-pyrrolo[2,3-c]pyridine-2-carboxylic acid (L001)

Ethyl 6-(4-((4-(1*H*-pyrazol-4-yl)phenyl)amino)pyrimidin-2-yl)-1-methyl-4,5,6,7-tetrahydro-1*H*-pyrrolo[2,3-c]pyridine-2-carboxylate (800 mg, 1.5 mmol) was dissolved in tetrahydrofuran/methanol/water (3/1/1, 10 mL in total), and sodium hydroxide (292 mg, 7.3 mmol) was added. The mixture was warmed to 50 °C and reacted for 1 h. Then the mixture was concentrated under reduced pressure, and 1 N diluted hydrochloric acid was added to adjust the pH to 5-6. The resulting mixture was concentrated under reduced pressure to give a white solid (1.0 g, crude product, containing sodium chloride). LC-MS [M+H]⁺: 416.0.

### Example 2. Preparation of Compound 7-(4-((4-(1H-pyrazol-4-yl)phenyl)amino)pyrimidin-2-yl)-5,6,7,8-tetrahydroimidazo[1,2-a]pyrazine-2-carboxylic acid (L002)

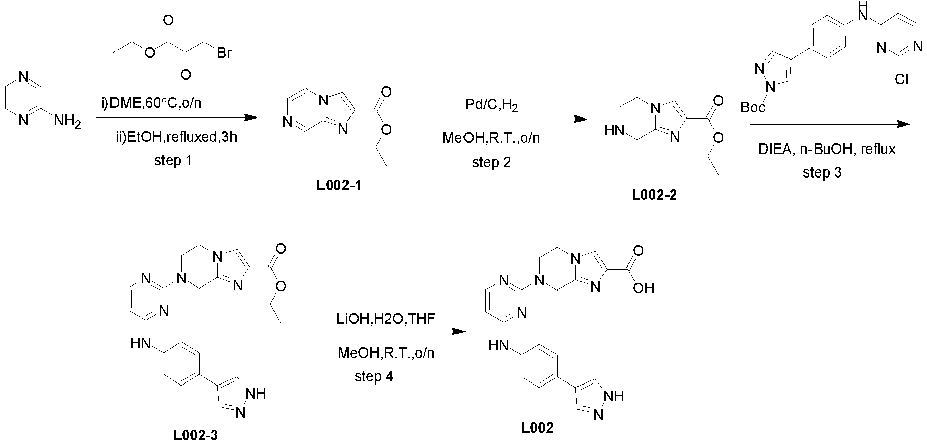

### 2.1 Preparation of compound ethyl imidazo[1,2-a]pyrazine-2-carboxylate (L002-1)

Pyrazine-2-amine (3.5 g, 36.82 mmol) and ethyl 3-bromo-2-oxopropionate (7.0 g, 44.18 mmol) were dissolved in ethylene glycol dimethyl ether (60 mL), and the mixture was heated at 60 °C overnight. Then the mixture was cooled to room temperature and filtered to collect the filter cake. Ethanol (40 mL) was added, and the mixture was heated at 80 °C for 3 h. Then the mixture was concentrated under reduced pressure, saturated sodium bicarbonate solution (30 mL) was added, and ethyl acetate (30 mL × 3) was added for extraction. The organic phases were combined, dried over anhydrous sodium sulfate and concentrated under reduced pressure. The resulting crude product was purified by silica gel column chromatography (dichloromethane:ethyl acetate = 1:1) to give a yellow oily product (1.15 g, 16.4% yield). LC-MS [M+H]⁺: 192.0.

### 2.2 Preparation of compound ethyl 5,6,7,8-tetrahydroimidazo[1,2-a]pyrazine-2-carboxylate (L002-2)

Ethyl imidazo[1,2-*a*]pyrazine-2-carboxylate (1.1 g, 5.75 mmol) was dissolved in absolute methanol (30 mL), and the catalyst palladium on carbon (10%, 600 mg) was added. Hydrogen was introduced, and the mixture was stirred overnight at room temperature. Then the mixture was filtered, and the filtrate was concentrated by rotary evaporation to give ethyl-5,6,7,8-tetrahydroimidazo[1,2-*a*]pyrazine-2-carboxylate in the form of a brown-yellow oil (800 mg, 71.2% yield). LC-MS [M+H]⁺: 196.0.

### 2.3 Preparation of compound ethyl 7-(4-((4-(1H-pyrazol-4-yl)phenyl)amino)pyrimidin-2-yl)-5,6,7,8-tetrahydroimidazo [1,2-a]pyrazine-2-carboxylate (L002-3)

Ethyl 5,6,7,8-tetrahydroimidazo[1,2-*a*]pyrazine-2-carboxylate (310 mg, 1.59 mmol), *tert-butyl* 4-(4-((2-chloropyrimidin-4-yl)amino)phenyl)-1*H*-pyrazole-1-carboxylate (589 mg, 1.59 mmol) and *N,N-*diisopropylethylamine (570 mg, 4.42 mmol) were dissolved in n-butanol (5 mL), and the mixture was stirred at 120 °C for 5 h. Water (20 mL) was added for dilution, and ethyl acetate (15 mL × 3) was added for extraction. The organic phases were combined, dried over anhydrous sodium sulfate, filtered and concentrated. The resulting crude product was purified by silica gel column chromatography (dichloromethane:methanol = 5: 1) to give ethyl 7-(4-((4-(1*H-*pyrazol-4-yl)phenyl)amino)pyrimidin-2-yl)-5,6,7,8-tetrahydroimidazo[1,2-*a*]pyrazine-2-carboxylate in the form of a brown oil (430 mg, 62.9% yield, crude product). LC-MS [M+H]⁺: 431.1.

### 2.4 Preparation of compound 7-(4-((4-(1H-pyrazol-4-yl)phenyl)amino)pyrimidin-2-yl)-5,6,7,8-tetrahydroimidazo[1,2-a]pyrazine-2-carboxylic acid (L002)

Ethyl 7-(4-((4-(1*H-*pyrazol-4-yl)phenyl)amino)pyrimidin-2-yl)-5,6,7,8-tetrahydroimidazo[1,2-*a*]pyrazine-2-carboxylate (430 mg, 1.00 mmol) was dissolved in tetrahydrofuran (3 mL), methanol (1 mL) and water (1 mL), and lithium hydroxide monohydrate (126 mg, 3.00 mmol) was added. The mixture was stirred overnight at room temperature. 1 N hydrochloric acid was added to adjust the pH to 2, and ethyl acetate (15 mL × 3) was added for extraction. The organic phases were combined, dried over anhydrous sodium sulfate, filtered and concentrated to give the product 7-(4-((4-(1*H-*pyrazol-4-yl)phenyl)amino)pyrimidin-2-yl)-5,6,7,8-tetrahydroimidazo[1,2-*a*]pyrazine-2-carboxylic acid in the form of a grey solid (350 mg, 87.1% yield, crude product). LC-MS [M+H]⁺: 403.0.

### Example 3. Preparation of Compound (5-(4-((4-(1H-pyrazol-4-yl)phenyl)amino)pyrimidin-2-yl)-4,5,6,7-tetrahydropyrazolo[1,5-a]pyrazin-2-yl)(3,3-difluoroazetidin-1-yl)methanone (L003)

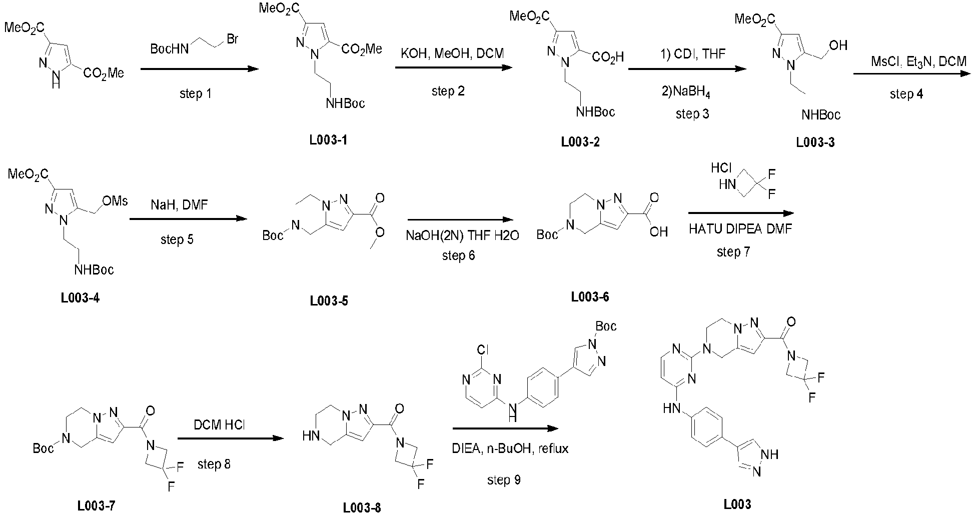

### 3.1 Preparation of compound dimethyl 1-(2-((tert-butoxycarbonyl)amino)ethyl)-1H-pyrazole-3,5-dicarboxylate (L003-1)

Dimethyl 1*H*-pyrazole-3,5-dicarboxylate (5.00 g, 27.10 mmol) was added to anhydrous *N,N-*dimethylformamide (60 mL), and potassium carbonate (5.61 g, 40.60 mmol) was added. The mixture was placed in an ice water bath under nitrogen atmosphere, and then *tert*-butyl (2-bromoethyl)carbamate (6.64 g, 29.80 mmol) was added. The mixture was stirred at room temperature for 3 h. Water (100 mL) was added for dilution, and a large amount of solid was precipitated out. Suction filtration was carried out, and the filter cake was washed with water (50 mL). The solid was collected and dried to give the crude product in the form of a white solid (8.70 g, 70% pure, 68% yield). MS [M+H]⁺ = 350.2.

### 3.2 Preparation of compound 1-(2-((tert-butoxycarbonyl)amino)ethyl)-3-(methoxycarbonyl)-1H-pyrazole-5-carboxylic acid (L003-2)

Dimethyl 1-(2-((*tert*-butoxycarbonyl)amino)ethyl)-1*H*-pyrazole-3,5-dicarboxylate (5.60 g, 17.10 mmol) was dissolved in dichloromethane (30 mL) and methanol (30 mL), and a solution of potassium hydroxide in methanol (2.2 M, 5.4 mL, 11.9 mmol) was added dropwise. The mixture was reacted at room temperature for 1 h. Water (60 mL) was added for dilution, and dichloromethane (50 mL × 2) was added for back-extraction. The aqueous phase was adjusted to pH = 3 with diluted hydrochloric acid (1 N), and dichloromethane (50 mL × 3) was added for extraction. The organic phases were combined, washed with saturated brine, dried over anhydrous sodium sulfate, filtered and concentrated to give a white solid (4.20 g, 78% yield, crude product). MS [M+H]⁺ = 336.2.

### 3.3 Preparation of compound methyl 1-(2-((tert-butoxycarbonyl)amino)ethyl)-5-(hydroxymethyl)-1H pyrazole-3-carboxylate (L003-3)

1-(2-((*tert*-butoxycarbonyl)amino)ethyl)-3-(methoxycarbonyl)-1*H*-pyrazole-5-carboxylic acid (2.00 g, 6.30 mmol) was dissolved in anhydrous tetrahydrofuran (30 mL), and *N*,*N*'-carbonyldiimidazole (2.04 g, 12.6 mmol)(CDI) was added. After stirring at 45 °C for 1 h, sodium borohydride (957 mg, 25.20 mmol) was added. The mixture was stirred overnight at room temperature. Saturated ammonium chloride solution (20 mL) was added to quench the reaction, water (20 mL) was added for dilution, and ethyl acetate (20 mL × 3) was added for extraction. The organic phases were combined, washed with saturated brine, dried over anhydrous sodium sulfate, filtered and concentrated, and the resulting crude product was purified by silica gel column chromatography (petroleum ether:ethyl acetate = 3:1) to give a colorless clear oily liquid (400 mg, 21% yield). MS [M+H]⁺ = 322.1.

### 3.4 Preparation of compound methyl 1-(2-((tert-butoxycarbonyl)amino)ethyl)-5-(((methylsulfonyl)oxo)meth yl)-1H-pyrazole-3-carboxylate (L003-4)

Methyl 1-(2-((*tert*-butoxycarbonyl)amino)ethyl)-5-(hydroxymethyl)-1*H*-pyrazole-3-carboxylate (400 mg, 1.33 mmol) was dissolved in anhydrous dichloromethane (5 mL), triethylamine (201 mg, 2.00 mmol) was added, and then methylsulfonyl chloride (182 mg, 1.59 mmol) was added dropwise at 0 °C. The mixture was stirred at room temperature for 1.5 h. Saturated sodium bicarbonate (5 mL) was added to quench the reaction, water (10 mL) was added for dilution, and ethyl acetate (10 mL × 3) was added for extraction. The organic phases were combined, washed with saturated brine, dried over anhydrous sodium sulfate, filtered and concentrated to give a colorless clear oily liquid (420 mg, 83% yield, crude product). MS (M -99)⁺= 278.1. 3.5 Preparation of compound methyl 5-*tert*-butoxycarbonyl-6,7-dihydropyrazolo[1,5-*a*]pyrazine-2,5(4*H*)-2-carboxylate (L003-5)

Methyl 1-(2-((*tert*-butoxycarbonyl)amino)ethyl)-5-(((methylsulfonyl)oxo)methyl)-1*H*-pyrazole-3-carboxylate (420 mg, 1.11 mmol) was dissolved in anhydrous *N*,*N-*dimethylformamide (10 mL), and sodium hydride (76 mg, 1.89 mmol, 60%)was added at 0 °C under nitrogen atmosphere. The mixture was stirred at room temperature for 1.5 h. After the reaction was completed, saturated ammonium chloride (10 mL) was added to quench the reaction, water (10 mL) was added for dilution, and ethyl acetate (10 mL × 3) was added for extraction. The organic phases were combined, washed with saturated brine (30 mL), dried over anhydrous sodium sulfate, filtered and concentrated, and the residue was purified by silica gel column chromatography (petroleum ether:ethyl acetate = 1:1) to give a colorless clear oily liquid (200 mg, 64% yield). MS[M+H]⁺ = 282.2.

### 3.6 Preparation of compound 5-(tert-butoxycarbonyl)-4,5,6,7-tetrahydropyrazol[1,5-a]pyrazine-2-carboxylic acid (L003-6)

Methyl 5-*tert*-butoxycarbonyl-6,7-dihydropyrazolo[1,5-*a*]pyrazine-2,5(4*H*)-2-carboxylate (200 mg, 0.71 mmol) was dissolved in tetrahydrofuran (3 mL) and water (1 mL), and aqueous sodium hydroxide solution (2 N, 1.4 mL, 2.80 mmol) was added dropwise. The mixture was reacted at room temperature for 1 h. Water (5 mL) was added for dilution, and ethyl acetate (5 mL × 2) was added for back-extraction. The aqueous phase was adjusted to pH = 3 with diluted hydrochloric acid (1 N), and ethyl acetate (5 mL × 3) was added for extraction. The organic phases were combined, washed with saturated brine, dried over anhydrous sodium sulfate, filtered and concentrated to give a white solid (130 mg, 68% yield, crude product). MS(M+1)⁺ = 268.2.

### 3.7 Preparation of compound tert-butyl 2-(3,3-difluoroazetidin-1-carbonyl)-6,7-dihydropyrazolo[1,5-a]pyrazine-5(4H)-carboxylate (L003-7)

5-(*tert*-butoxycarbonyl)-4,5,6,7-tetrahydropyrazol[1,5-*a*]pyrazine-2-carboxylic acid (130 mg, 0.49 mmol) was dissolved in anhydrous *N*,*N*-dimethylformamide (3 mL), and 2-(7-azabenzotriazol)-*N*,*N*,*N',N*'-tetramethyluronium hexafluorophosphate (205 mg, 0.54 mmol), *N*,*N*-diisopropylethylamine (255 mg,1.96 mmol) and 3,3-difluoroazetidine hydrochloride (70 mg, 0.54 mmol) were added. The mixture was stirred at room temperature for 2 h. Water (10 mL) was added for dilution, and ethyl acetate (10 mL × 3) was added for extraction. The organic phases were combined, washed with saturated brine, dried over anhydrous sodium sulfate, filtered and concentrated, and the resulting crude product was purified by silica gel column chromatography (petroleum ether:ethyl acetate = 3:1) to give a white solid (110 mg, 65% yield). MS (M+1)⁺ = 343.2.

### 3.8 Preparation of compound (3, 3-difluoroazetidin-1-yl)(4,5,6,7-tetrahydropyrazolo[1,5-a]pyrazin-2-yl)methanone (L003-8)

Tert-butyl 2-(3,3-difluoroazetidin-1-carbonyl)-6,7-dihydropyrazolo[1,5-*a*]pyrazine-5(4*H*)-carboxylate (110 mg, 0.32 mmol) was dissolved in anhydrous dichloromethane (3 mL), and a solution of hydrogen chloride in ethanol (33%, 1 mL) was added. The mixture was stirred at room temperature for 2 h. Then the mixture was concentrated under reduced pressure to give a white solid (90 mg, crude product). MS (M+1)⁺ = 242.9.

### 3.9 Preparation of compound (5-(4-((4-(1H-pyrazol-4-yl)phenyl)amino)pyrimidin-2-yl)-4,5,6,7-tetrahydropyrazolo[1,5-a]pyrazin-2-yl)(3,3-difluoroazetidin-1-yl)methanone (L003)

(3,3-difluoroazetidin-1-yl)(4,5,6,7-tetrahydropyrazolo[1,5-a]pyrazin-2-yl)methanone hydrochloride (90 mg, 0.32 mmol) was dissolved in *n*-butanol (3 mL), and *tert*-butyl 4-(4-((2-chloropyrimidin-4-yl)amino)phenyl)-1*H*-pyrazole-1-carboxylate (119 mg, 0.32 mmol) and N*,N-*diisopropylethylamine (255 mg, 1.96 mmol) were added. The mixture was stirred overnight under reflux, water (10 mL) was added for dilution, and ethyl acetate (10 mL × 3) was added for extraction. The organic phases were combined, washed with saturated brine, dried over anhydrous sodium sulfate, filtered and concentrated, and the residue was purified by preparative chromatography to give a white solid (19.6 mg, 12% two-step yield). MS (M+1)⁺ = 478.2.

¹H NMR (400 MHz, DMSO) δ 12.91 (brs, 1H), 9.42 (s, 1H), 8.08-7.96 (m, 3H), 7.63-7.58(m, 4H), 6.68 (s, 1H), 6.15 (d, J = 5.7 Hz, 1H), 4.97 (s, 2H), 4.85 (t, J = 12.4 Hz, 2H), 4.44 (t, J = 12.4 Hz, 2H), 4.25 (s, 4H).

### Example 4. Preparation of Compound 6-(4-((4-(1H-pyrazol-4-yl)phenyl)amino)pyrimidin-2-yl)-N-(3,3-difluorocyclobutyl)-1-methyl-4,5,6,7-tetrahydro-1H-pyrrolo[2,3-c]pyridine-2-carboxamide (L004)

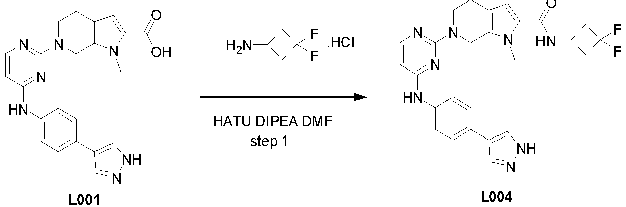

4.1 Preparation of compound 6-(4-((4-(1*H*-pyrazol-4-yl)phenyl)amino)pyrimidin-2-yl)-*N*-(3,3-difluorocyclobutyl)-1-methyl-4,5,6,7-tetrahydro-1*H*-pyrrolo[2,3-*c*]pyridine-2-carboxamide (L004) 6-(4-((4-(1H pyrazol-4-yl)phenyl)amino)pyrimidin-2-yl)-1-methyl-4,5,6,7-tetrahydro-1H pyrrolo[2,3-c]pyridine-2-carboxylic acid (L001) (150 mg, 0.36 mmol) was dissolved in anhydrous *N,N-*dimethylformamide (5 mL), and *N*,*N-*diisopropylethylamine (185 mg, 1.44 mmol), 3,3-difluorocyclobutane-1-amine hydrochloride (57 mg, 0.40 mmol) and 2-(7-azabenzotriazol)-*N*,*N*,*N*',*N*'-tetramethyluronium hexafluorophosphate (150 mg, 0.40 mmol) were added. The mixture was reacted at room temperature for 4 h under nitrogen atmosphere. After the reaction was completed, water (15 mL) was added to quench the reaction, and ethyl acetate (10 mL × 3) was added for extraction. The organic phases were combined, washed with saturated brine (20 mL), dried over anhydrous sodium sulfate, filtered and concentrated. The resulting crude product was purified by silica gel column chromatography (eluent: dichloromethane:methanol = 20:1) to give a white solid (40.8 mg, 22% yield). MS [M+H]⁺ = 504.9.

¹H NMR (400 MHz, MeOD) δ 7.93 (brs, 2H), 7.88 (d, J = 6.0 Hz, 1H), 7.62 (d, J = 8.6 Hz, 2H), 7.55 (d, J = 8.7 Hz, 2H), 6.63 (s, 1H), 6.09 (d, J = 6.0 Hz, 1H), 4.77 (s, 2H), 4.28 -4.22 (m, 1H), 4.02 - 3.96 (m, 2H), 3.80(s, 3H), 2.95-2.87 (m, 2H), 2.65-2.62 (m, 4H).

### Example 5. Preparation of Compound (6-(4-((4-(1H-pyrazol-4-yl)phenyl)amino)pyrimidin-2-yl)-1-methyl-4,5,6,7-tetrahydro-1H-pyrrolo[2,3-c]pyridin-2-yl)(3,3-difluoroazetidin-1-yl)methanone (L005)

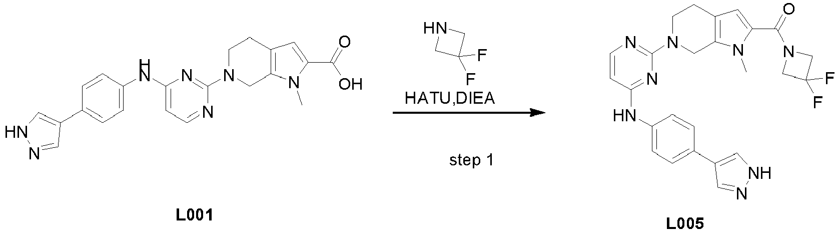

3,3-difluoroazetidine (67 mg, 0.7 mmol) was dissolved in DMF (10 mL), and 6-(4-((4-(1*H*-pyrazol-4-yl)phenyl)amino)pyrimidin-2-yl)-1-methyl-4,5,6,7-tetrahydro-1*H*-pyrrolo[2,3-*c*]pyridine-2-carboxylic acid (L001) (200 mg, 0.5 mmol), HATU (547 mg, 1.4 mmol) and DIEA (186 mg, 1.4 mmol) were added. The mixture was reacted at room temperature for 2 h. Water (30 mL) was added to quench the reaction, and ethyl acetate (30 mL × 3) was added for extraction. The organic phase was washed with saturated brine (30 mL), dried over anhydrous sodium sulfate and concentrated, and the residue was separated by preparative chromatography to give a white solid (60 mg). LC-MS [M+H]⁺: 490.7.

¹H NMR (400 MHz, MeOD) δ 7.96 (s, 2H), 7.90 (d, J = 6.1 Hz, 1H), 7.64 (d, J = 8.7 Hz, 2H), 7.59 (d, J = 8.7 Hz, 2H), 6.48 (s, 1H), 6.15 (d, J = 6.1 Hz, 1H), 4.82 (s, 2H), 4.64-4.52 (m, 4H), 4.03 (t, J = 5.7 Hz, 2H), 3.83 (s, 3H), 2.69 (t, J = 5.5 Hz, 2H).

### Example 6. Preparation of Compound (6-(4-((1H-indazol-5-yl)amino)pyrimidin-2-yl)-1-methyl-4,5,6,7-tetrahydro-1H pyrrol[2,3-c]pyridin-2-yl)(3,3-difluoroazetidin-1-yl)methanone (L006)

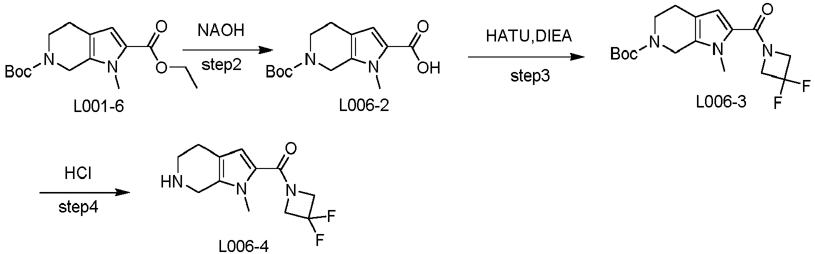

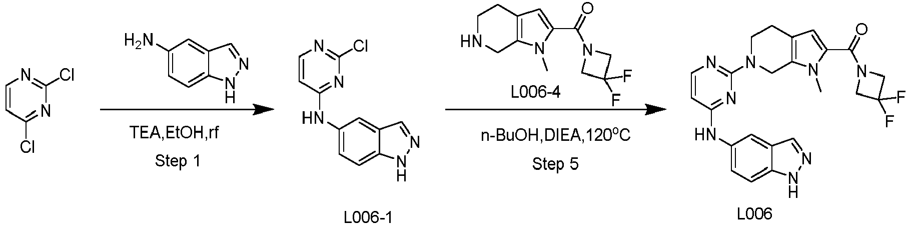

6.1 Preparation of compound *N*-(2-chloropyrimidin-4-yl)-1*H-*indazol-5-amine (L006-1) 2,4-dichloropyrimidine (1.48 g, 10.0 mmol) and 1*H*-indazol-5-amine (1.33 g, 10.0 mmol) were dissolved in absolute ethanol (10 mL) under nitrogen atmosphere, and triethylamine (1.01 g, 10.0 mmol) was added. The mixture was heated under reflux and reacted for 16 h. After the reaction was completed, the mixture was concentrated under reduced pressure. Water (20 mL) was added for dilution, and ethyl acetate (20 mL × 3) was added for extraction. The organic phases were combined, dried over anhydrous sodium sulfate, filtered and concentrated by rotary evaporation to give *N*-(2-chloropyrimidin-4-yl)-1*H*-indazol-5-amine in the form of a pink solid (1.30 g, 53% yield). MS [M+H]⁺ = 246.0.

### 6.2 Preparation of compound 6-(tert-butoxycarbonyl)-1-methyl-4,5,6,7-tetrahydro-1H-pyrrolo[2,3-c]pyridine-2-carboxylic acid (L006-2)

Ethyl 6-*tert*-butoxycarbonyl-1-methyl-4,5,7-trihydro-1H pyrrolo[2,3-c]pyridine-2-carboxylate (4.0 g, 12.9 mmol) was dissolved in methanol (40 mL), and sodium hydroxide solution (38.7 mL, 1 N) was added. The mixture was reacted at 50 °C for 2 h. Diluted hydrochloric acid (1N) was added to adjust the pH to 5-6, and ethyl acetate (50 mL × 3) was added for extraction. The organic phase was washed with saturated brine (50 mL), dried over anhydrous sodium sulfate and concentrated by rotary evaporation under reduced pressure to give a purple oily liquid (3.2 g). LC-MS [M+H]⁺: 281.1.

### 6.3 Preparation of compound tert-butyl 2-(3,3-difluoroazetidin-1-carbonyl)-1-methyl-4,5-dihydro-1H-pyrrolo[2,3-c]pyridine-6(7H)-carboxylate (L006-3):

6-(tert-butoxycarbonyl)-1-methyl-4,5,6, 7-tetrahydro-1*H*-pyrrolo[2,3-e]pyridine-2-carboxylic acid (2.4 g, 8.5 mmol) was dissolved in anhydrous DMF (20 mL), and 3,3-difluoroazetidine hydrochloric acid (1.2 g, 12.8 mmol), HATU (4.9 g, 12.8 mmol) and DIEA (3.3 g, 25.6 mmol) were added. The mixture was reacted at room temperature for 1 h. Water (30 mL) was added to quench the reaction, and ethyl acetate (30 mL × 3) was added for extraction. The organic phase was washed with saturated brine (50 mL), dried over anhydrous sodium sulfate and concentrated by rotary evaporation under reduced pressure, and the residue was subjected to silica gel column chromatography (PE/EA = 3/1) to give a yellow oily liquid (2.5 g, 83.3% yield).

LC-MS[ M+H]⁺: 355.8.

### 6.4 Preparation of compound (3,3-difluoroazetidin-1-yl) (1-methyl-4,5,6,7-tetrahydro-1H-pyrrolo[2,3-c]pyridin-2-yl)methanone (L006-4):

*Tert-butyl* 2-(3,3-difluoroazetidin-1-carbonyl)-1-methyl-4,5-dihydro-1*H*-pyrrolo[2,3-*c*]pyridine-6(7*H*)-carboxylate (1.8 g, 5.8 mmol) was dissolved in absolute ethanol (20 mL), and a solution of hydrochloric acid in ethanol (5 mL, mass fraction 33%) was added. The mixture was reacted at room temperature for 3 h. Then the mixture was concentrated by rotary evaporation under reduced pressure to give a yellow solid (2.0 g).

LC-MS [M+H]⁺: 256.0.

### 6.5 Preparation of compound (6-(4-((1H-indazol-5-yl)amino)pyrimidin-2-yl)-1-methyl-4,5,6,7-tetrahydro-1H-pyrrol[2,3-c]pyridin-2-yl)(3,3-difluoroazetidin-1-yl)methanone (L006)

(3,3-difluoroazetidin-1-yl)(1-methyl-4,5,6,7-tetrahydro-1*H*-pyrrolo[2,3-*c*]pyridin-2-yl)methanone (50 mg, 0.20 mmol) and *N*-(2-chloropyrimidin-4-yl)-1*H*-indazol-5-amine (73.7 mg, 0.30 mmol) were dissolved in *n-*butanol (1.0 mL), and diisopropylethylamine (53 mg, 0.41 mmol) was added. The mixture was reacted at 120 °C for 15 h. After the reaction was completed, the mixture was cooled to room temperature and concentrated by rotary evaporation under reduced pressure. The residue was purified by thin layer chromatography (petroleum ether:ethyl acetate = 1:5) to give a white solid (20 mg, 22% yield). MS [M+H] ⁺ = 485.0.

¹H NMR (400 MHz, DMSO) δ 12.97 (s, 1H), 9.28 (s, 1H), 8.13 (s, 1H), 8.00 (s, 1H), 7.94 (d, J = 5.7 Hz, 1H), 7.51 (d, J = 8.9 Hz, 1H), 7.44 (dd, J = 8.9,1.6 Hz, 1H), 6.46 (s, 1H), 6.06 (d, J = 5.8 Hz, 1H), 4.80 (s, 2H), 4.62-4.48 (m, 4H), 3.97 (t, J = 5.6 Hz, 2H), 3.76 (s, 3H), 2.56 (t, J = 5.5 Hz, 2H).

### Example 7. Preparation of Compound 2-(4-((2-(2-(3,3-difluoroazetidin-1-carbonyl)-1-methyl-1,4,5,7-tetrahydro-6H-pyrrolo[2,3-c]pyridin-6-yl)pyrimidin-4-yl)amino)-2-fluorophenoxy)nicotinamide (L007)

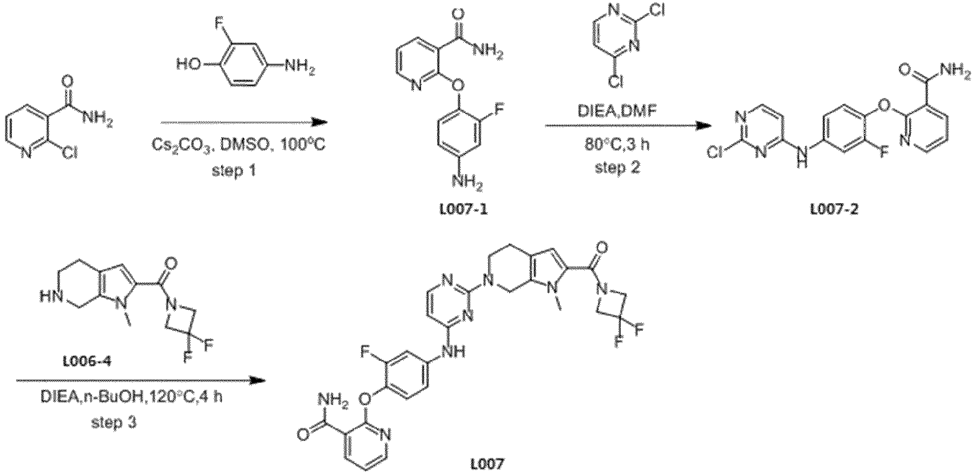

### 7.1 Preparation of compound 2-(4-amino-2-fluorophenoxy)nicotinamide (L007-1):

2-chloronicotinamide (3.00 g, 19.16 mmol) and 4-amino-2-fluorophenol (2.43 g, 19.16 mmol) were dissolved in anhydrous dimethyl sulfoxide (50 mL), and cesium carbonate (18.68 g, 57.48 mmol) was added. The mixture was stirred at 100 °C for 12 h. After the reaction was completed, water (100 mL) was added, and

ethyl acetate (100 mL × 8) was added for extraction. The organic phases were combined, dried over anhydrous sodium sulfate, filtered and concentrated by rotary evaporation. The resulting crude product was purified by silica gel column chromatography (dichloromethane:methanol = 10:1) to give 2-(4-amino-2-fluorophenoxy)nicotinamide in the form of a brown solid (1.60 g, 34% yield). LC-MS [M+H]⁺: 248.1.

7.2 Preparation of compound 2-(4-((2-chloropyrimidin-4-yl)amino)-2-fluorophenoxy)nicotinamide (L007-2): 2-(4-amino-2-fluorophenoxy)nicotinamide (300 mg, 1.21 mmol) was dissolved in anhydrous *N,N-*dimethylformamide (10 mL), and 2,4-dichloropyrimidine (180 mg, 1.21 mmol) and *N,N-*diisopropylethylamine (470 mg, 3.63 mmol) were added. The system was warmed to 80 °C and reacted for 3 h. Water (30 mL) was added to quench the reaction, and ethyl acetate (30 mL × 3) was added for extraction. The organic phases were combined, washed with saturated brine (30 mL), dried over anhydrous sodium sulfate, filtered and concentrated by rotary evaporation. The resulting crude product was purified by silica gel column chromatography (dichloromethane:ethyl acetate = 1:1) to give 2-(4-((2-chloropyrimidin-4-yl)amino)-2-fluorophenoxy)nicotinamide in the form of a light yellow solid (150 mg, 31% yield).

### 7.3 Preparation of compound 2-(4-((2-(2-(3,3-difluoroazetidin-1-carbonyl)-1-methyl-1,4,5,7-tetrahydro-6H-pyrrolo [2,3-c]pyridin-6-yl)pyrimidin-4-yl)amino)-2-fluorophenoxy)nicotinamide (L007):

2-(4-((2-chloropyrimidin-4-yl)amino)-2-fluorophenoxy)nicotinamide (85 mg, 0.24 mmol) was dissolved in *n-*butanol (3 mL), and (3,3-difluoroazetidin-1-yl)(1-methyl-4,5,6,7-tetrahydro-1*H*-pyrrolo[2,3-*c*]pyridin-2-yl)methanone hydrochloride (60 mg, 0.24 mmol) and *N*,*N*-diisopropylethylamine (155 mg, 1.20 mmol) were added to the system. The system was warmed to 120 °C and stirred for 4 h. After the reaction was completed as detected, water (10 mL) was added to quench the reaction, and ethyl acetate (10 mL × 3) was added for extraction. The organic phases were combined, washed with saturated brine (10 mL), dried over anhydrous sodium sulfate, filtered and concentrated by rotary evaporation, and the resulting crude product was purified by silica gel column chromatography (dichloromethane:methanol = 10:1) to give 2-(4-((2-(2-(3,3-difluoroazetidin-1-carbonyl)-1-methyl-1,4,5,7-tetrahydro-6*H-*pyrrolo[2,3-*c*]pyridin-6-yl)pyrimidin-4-yl)amino)-2-fluorophenoxy)nicotinamide in the form of a white solid (12.1 mg, 8.8% yield). LC-MS [M+H]⁺: 579.0.

¹H NMR (301 MHz, DMSO) δ 9.58 (s, 1H), 8.23 - 8.13 (m, 2H), 8.05 - 7.98 (m, 1H), 7.92 (d, J = 13.6 Hz, 1H), 7.82 (s, 2H), 7.38 - 7.29 (m, 2H), 7.26 - 7.20 (m, 1H), 6.44 (s, 1H), 6.09 (d, J = 5.7 Hz, 1H), 4.78 (s, 2H), 4.53 (t, J=7.1 Hz, 4H), 3.99-3.92 (m, 2H), 3.70 (s, 3H), 2.58-2.50 (m, 2H).

### Example 8. Preparation of Compound 7-(4-((4-(1H-pyrazol-4-yl)phenyl)amino)pyrimidin-2-yl)-N-(3,3-difluorocyclobutyl)-5,6,7,8-tetrahydroimidazo[1,2-a]pyrazine-2-carboxamide (L008)

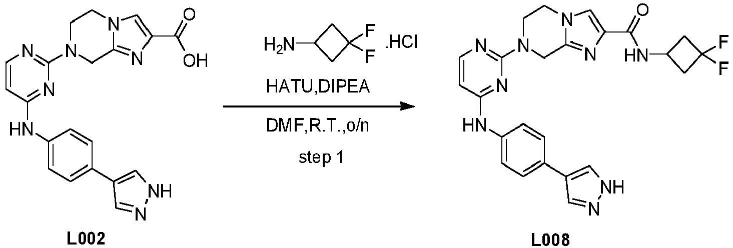

7-(4-((4-(1*H*-pyrazol-4-yl)phenyl)amino)pyrimidin-2-yl)-5,6,7,8-tetrahydroimidazo[1,2-*a*]pyrazine-2-carboxylic acid (L002) (100 mg, 0.25 mmol) was dissolved in *N,N*-dimethylformamide (5 mL), and HATU (140 mg, 0.37 mmol), 3,3-difluorocyclobutane-1-amine hydrochloride (53 mg, 0.37 mmol) and *N,N-*diisopropylethylamine (160 mg, 1.25 mmol) were added. The mixture was stirred overnight at room temperature under nitrogen atmosphere. Water (15 mL) was added to quench the reaction, and ethyl acetate (15 mL × 3) was added for extraction. The organic phases were combined, washed with saturated brine (15 mL × 3), dried over anhydrous sodium sulfate, filtered and concentrated. The resulting crude product was purified by silica gel column chromatography (dichloromethane:methanol = 10:1), and the product was collected, concentrated and lyophilized to give 7-(4-((4-(1*H*-pyrazol-4-yl)phenyl)amino)pyrimidin-2-yl)-*N-*(3,3-difluorocyclobutyl)-5,6,7,8-tetrahydroimidazo[1,2-*a*]pyrazine-2-carboxamide in the form of a white solid (24.7 mg, 20.1% yield). LC-MS [M+H]⁺: 492.0.

¹H NMR (400 MHz, DMSO) δ 12.87 (s, 1H), 9.41 (s, 1H), 8.49 (d, J = 7.7 Hz, 1H), 8.20 - 7.85 (m, 3H), 7.66 - 7.56 (m, 5H), 6.14 (d, J = 5.8 Hz, 1H), 4.91 (s, 2H), 4.30 - 4.25 (m, 1H), 4.20 - 4.08 (m, 4H), 2.87 - 2.77 (m, 4H).

### Example 9. Preparation of Compound 6-(4-((4-(1H-pyrazol-4-yl)phenyl)amino)pyrimidin-2-yl)-N-isopropyl-1-methyl-4,5,6,7-tetrahydro-1H-pyrrolo[2,3-c]pyridine-2-carboxamide (L009)

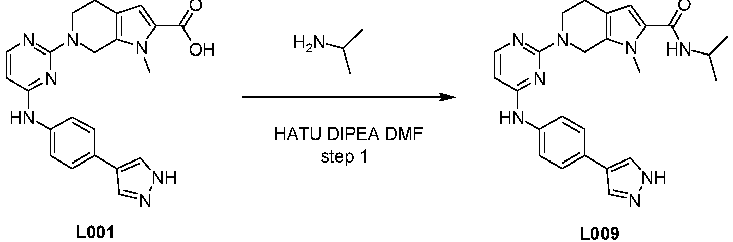

6-(4-((4-(1*H*-pyrazol-4-yl)phenyl)amino)pyrimidin-2-yl)-1-methyl-4,5,6,7-tetrahydro-1*H-*pyrrolo[2,3-c]pyridine-2-carboxylic acid (L001) (150 mg, 0.36 mmol) was dissolved in anhydrous *N,N-*dimethylformamide (5 mL), and N,N diisopropylethylamine (185 mg, 1.44 mmol), isopropylamine (57 mg, 0.40 mmol) and 2-(7-azabenzotriazol)-*N,N,N',N'*-tetramethyluronium hexafluorophosphate (24 mg, 0.40 mmol) were added. The mixture was reacted at room temperature for 4 h under nitrogen atmosphere. After the reaction was completed, water (15 mL) was added to quench the reaction, and ethyl acetate (10 mL × 3) was added for extraction. The organic phases were combined, washed with saturated brine (20 mL), dried over anhydrous sodium sulfate, filtered and concentrated. The resulting crude product was purified to give a white solid (30.7 mg, 18% yield). MS [M+H]⁺ = 457.2.

¹H NMR (400 MHz, DMSO) δ 12.82 (s, 1H), 9.31 (s, 1H), 8.01 (s, 2H), 7.96 (d, J = 5.7 Hz, 1H), 7.65 (d, J = 8.6 Hz, 2H), 7.58 - 7.55 (m, 3H), 6.62 (s, 1H), 6.07 (d, J = 5.8 Hz, 1H), 4.76 (s, 2H), 4.05 - 3.98 (m, 3H), 3.78 (s, 3H), 2.54 - 2.52 (m, 2H), 1.10 (d, J = 6.6 Hz, 6H).

### Example 10. Preparation of Compound 1-(6-(4-((4-(1H-pyrazol-4-yl)phenyl)amino)pyrimidin-2-yl)-1-methyl-4,5,6,7-tetrahydro-1H-pyrrolo[2,3-c]pyridin-2-carbonyl)azetidine-3-carbonitrile (L010)

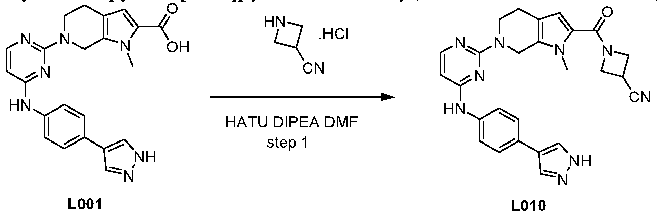

6-(4-((4-(1*H-*pyrazol-4-yl)phenyl)amino)pyrimidin-2-yl)-1-methyl-4,5,6,7-tetrahydro-1*H*-pyrrolo[2,3-c]pyridine-2-carboxylic acid (L001) (150 mg, 0.36 mmol) was dissolved in anhydrous *N,N-*dimethylformamide (5 mL), and *N*,*N*-diisopropylethylamine (185 mg, 1.44 mmol), 3-cyano-azetidine hydrochloride (47 mg, 0.40 mmol) and 2-(7-azabenzotriazol)-*N*,*N*,*N'*,*N'*-tetramethyluronium hexafluorophosphate (150 mg, 0.40 mmol) were added. The mixture was reacted at room temperature for 4 h under nitrogen atmosphere. After the reaction was completed, water (15 mL) was added to quench the reaction, and ethyl acetate (10 mL × 3) was added for extraction. The organic phases were combined, washed with saturated brine (20 mL), dried over anhydrous sodium sulfate, filtered and concentrated. The resulting crude product was purified by silica gel column chromatography (eluent: dichloromethane:methanol = 20:1) to give a white solid (58.6 mg, 34% yield). MS [M+H]⁺ = 479.9.

¹H NMR (400 MHz, MeOD) δ 7.92 (brs, 2H), 7.87 (d, J = 6.0,1H), 7.61 (d, J = 8.6 Hz, 2H), 7.54 (d, J = 8.6 Hz, 2H), 6.38 (s, 1H), 6.09 (d, J = 6.0 Hz, 1H), 4.75 (s, 2H), 4.60 - 4.28 (m, 4H), 4.00 - 3.96 (m, 2H), 3.77 (s, 3H), 3.75 - 3.68 (m, 1H), 2.66 - 2.60 (m, 2H).

### Example 11. Preparation of Compound (2-(4-((4-(1H-pyrazol-4-yl)phenyl)amino)pyrimidin-2-yl)-2H indazol-5-yl)(3,3-difluoroazetidin-1-yl)methanone (L011)

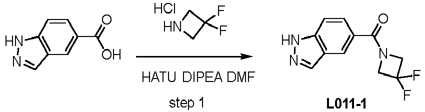

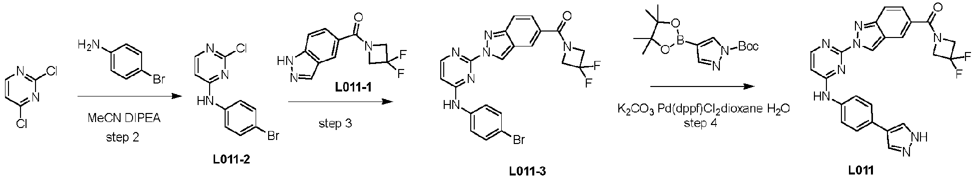

11.1 Preparation of compound (3,3-difluoroazetidin-1-yl)(1H indazol-5-yl)methanone (L011-1) 1*H*-indazole-5-carboxylic acid (1.50 g, 9.26 mmol) was dissolved in anhydrous *N*,*N*-dimethylformamide (30 mL), and 3,3-difluoroazetidine hydrochloride (1.31 g, 10.18 mmol), 2-(7-azabenzotriazol)-*N*,*N*,*N'*,*N'-*tetramethyluronium hexafluorophosphate (3.86 g, 10.18 mmol) and 7V,7V-diisopropylethylamine (4.78 g, 37.04 mmol) were added. The mixture was stirred at room temperature for 4 h. Water (50 mL) was added for dilution, and ethyl acetate (30 mL × 2) was added for extraction. The organic phases were combined, washed with saturated brine (30 mL × 3), dried over anhydrous sodium sulfate, filtered and concentrated, and the resulting crude product was purified by silica gel column chromatography (EA) to give a white solid (1.40 g, 63% yield). LC-MS [M+H]⁺: 237.9.

### 11.2 Preparation of compound N-(4-bromophenyl)-2-chloropyrimidin-4-amine (L011-2)

2,4-dichloropyrimidine (500 mg, 3.38 mmol) was dissolved in acetonitrile (5 mL), and 4-bromoaniline (577 mg, 3.38 mmol) and 7V,7V-diisopropylethylamine (872 mg, 6.76 mmol) were added. The mixture was heated under reflux and reacted for 24 h. Then the mixture was concentrated under reduced pressure, and the resulting crude product was separated by silica gel column chromatography (PE/EA = 2/1) to give a white solid (800 mg, 83% yield). LC-MS [M+H]⁺: 283.9.

### 11.3 Preparation of compound (2-(4-((4-bromophenyl)amino)pyrimidin-2-yl)-2H-indazol-5-yl)(3,3-difluoroazetidin-1-yl)methanone (L011-3)

7V-(4-bromophenyl)-2-chloropyrimidin-4-amine (800 mg, 2.82 mmol) was dissolved in acetonitrile (5 mL), and (3,3-difluoroazetidin-1-yl)(1*H*-indazol-5-yl)methanone (735 mg, 3.10 mmol) and acetic acid (0.1 mL) were added. The mixture was stirred under microwave at 140 °C for 1 h. Water (20 mL) was added to quench the reaction, and ethyl acetate (20 mL × 3) was added for extraction. The organic phases were combined, washed with saturated brine (20 mL), dried over anhydrous sodium sulfate, filtered and concentrated, and the resulting crude product was separated by silica gel column chromatography (EA) to give the compound L011-3 in the form of a colorless oily liquid (250 mg, 18% yield). LC-MS [M+H]⁺: 484.8.

### 11.4 Preparation of compound (2-(4-((4-(1H-pyrazol-4-yl)phenyl)amino)pyrimidin-2-yl)-2H-indazol-5-yl)(3,3-difluoroazetidin-1-yl)methanone (L011)

(2-(4-((4-bromophenyl)amino)pyrimidin-2-yl)-2H indazol-5-yl)(3,3-difluoroazetidin-1-yl)methanone (250 mg, 0.51 mmol) was dissolved in dioxane (6 mL) and water (1 mL) under nitrogen atmosphere, and *tert-butyl* 4-(4,4,5,5-tetramethyl-1,3,2-dioxaborolan-2-yl)-1H pyrazole-1-carboxylate (180 mg, 0.61 mmol), potassium carbonate (140 mg,10.2 mmol) and 1,1'-bis(diphenylphosphino)ferrocene dichloropalladium(II) (37 mg, 0.051 mmol) were added. The mixture was stirred at 80 °C for 3 h. Then the mixture was cooled to room temperature, water (20 mL) was added to quench the reaction, and ethyl acetate (10 mL × 3) was added for extraction. The organic phases were combined, washed with saturated brine (20 mL), dried over anhydrous sodium sulfate, and separated by preparative chromatography to give a white solid (23 mg, 10% yield). LC-MS [M+H]⁺: = 472.8.

¹H NMR (400 MHz, DMSO) δ 12.93 (s, 1H), 10.16 (s, 1H), 9.31 (s, 1H), 8.41 (d, J = 5.9 Hz, 1H), 8.30 (s, 1H), 8.19 (s,1H), 7.94 (s,1H),7.83-7.76 (m, 3H), 7.68 (d, J = 8.6 Hz, 2H), 7.63 (dd, J = 9.2, 1.5 Hz, 1H), 6.86 (d, J = 5.9 Hz, 1H), 4.72 (brs, 4H).

### Example 12. Preparation of Compound 6-(4-((4-(1H-pyrazol-4-yl)phenyl)amino)pyrimidin-2-yl)-1-methyl-N-(pyridazin-4-yl)-4,5,6,7-tetrahydro-1H-pyrrolo[2,3-c]pyridine-2-carboxamide (L012)

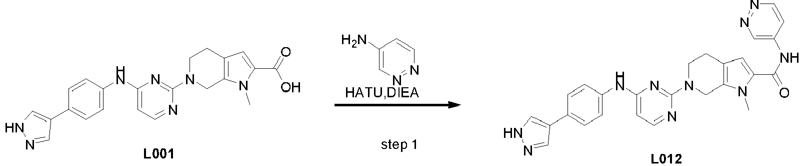

Pyridazin-4-amine (34 mg, 0.36 mmol) was dissolved in DMF (4 mL), and 6-(4-((4-(1*H*-pyrazol-4-yl)phenyl)amino)pyrimidin-2-yl)-1-methyl-4,5,6,7-tetrahydro-1*H*-pyrrolo[2,3-c]pyridine-2-carboxylic acid (L001) (100 mg, 0.24 mmol), HATU (137 mg, 0.36 mmol) and DIEA (93 mg, 0.72 mmol) were added. The mixture was reacted at room temperature for 2 h under nitrogen atmosphere. Water (15 mL) was added to quench the reaction, and ethyl acetate (10 mL × 3) was added for extraction. The organic phases were combined, washed with saturated brine (15 mL), dried over anhydrous sodium sulfate, filtered and concentrated. The resulting crude product was purified by preparative chromatography to give a white solid (2 mg).

LC-MS [M+H]⁺: 492.7.

¹H NMR (400 MHz, DMSO) δ 12.98 (s, 1H), 10.23 (s, 1H), 9.47 (s, 1H), 9.35 (s, 1H), 8.99 (d, J = 5.8 Hz, 1H), 8.34 (s, 1H), 8.08 - 7.94 (m, 3H), 7.66 (d, J = 8.2 Hz, 2H), 7.58 (d, J = 8.1 Hz, 2H), 7.04 (s, 1H), 6.09 (d, J = 6.0 Hz, 1H), 4.85 (s, 2H), 4.05 - 3.97 (m, 2H), 3.85 (s, 3H), 3.54 - 3.46 (m, 2H).

### Example 13. Preparation of Compound 2-(4-((4-(1H-pyrazol-4-yl)phenyl)amino)pyrimidin-2-yl)-1,2,3,4-tetrahydropyrrolo[1,2-a]pyrazine-7-carboxylic acid (L013)

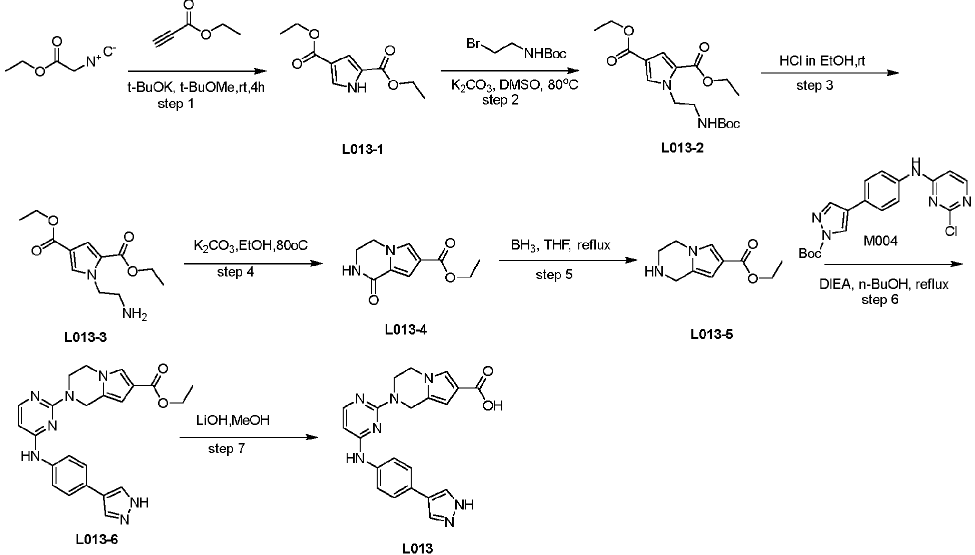

### 13.1 Preparation of compound diethyl 1H pyrrole-2,4-dicarboxylate (L013-1)

Ethyl 2-isocyanoacetate (5.0 g, 44.2 mmol) was dissolved in methyl *tert-butyl* ether (50 mL) under nitrogen atmosphere, and ethyl propiolate (4.3 g, 44.2 mmol) was added to the reaction liquid. Potassium tert-butoxide (6.9 g, 61.9 mmol) was added to the reaction system under an ice salt bath, and then the reaction system was warmed to room temperature and stirred for 2 h. Water (50 mL) was added to quench the reaction, and methyl *tert-butyl* ether (50 mL × 2) was added for extraction. The organic phases were combined, dried over anhydrous sodium sulfate, filtered and concentrated under reduced pressure, and the resulting crude product was purified by silica gel column chromatography (PE/EA = 3/1) to give a yellow oily liquid (2.5 g, 26.9% yield). LC-MS [M+H]⁺: 212.0.

### 13.2 Preparation of compound diethyl 1-(2-((tert-butoxycarbonyl)amino)ethyl)-1H-pyrrole-2,4-dicarboxylate (L013-2)

Diethyl 1*H*-pyrrole-2,4-dicarboxylate (2.5 g, 11.8 mmol) was dissolved in anhydrous DMSO (20 mL) under hydrogen atmosphere, and tert-butyl (2-bromoethyl)carbamate (7.9 g, 35.4 mmol) and potassium carbonate (4.9 g, 35.4 mmol) were added. The mixture was stirred overnight at 80 °C. Water (50 mL) was added to quench the reaction, and ethyl acetate (30 mL) was added for extraction. The organic phases were combined, dried over anhydrous sodium sulfate, filtered and concentrated under reduced pressure, and the resulting crude product was separated by silica gel column chromatography (PE/EA = 5/1) to give a yellow oily liquid (2.0 g, 47.9% yield). LC-MS [M+Na]⁺: 376.9.

### 13.3 Preparation of compound diethyl 1-(2-aminoethyl)-1H-pyrrole-2,4-dicarboxylate (L013-3)

Diethyl 1-(2-((*tert*-butoxycarbonyl)amino)ethyl)-1*H-*pyrrole-2,4-dicarboxylate (2.0 g, 5.6 mmol) was dissolved in absolute ethanol (10 mL) under nitrogen atmosphere, and a solution of hydrogen chloride in ethanol (33%, 5 mL) was added. The mixture was stirred at room temperature for 2 h. Then the mixture was concentrated to give a yellow oily liquid (2.2 g), and the crude product was used directly in the next step. LC-MS [M+H]⁺: 255.1.

13.4 Preparation of compound ethyl 1-oxo-1,2,3,4-tetrahydropyrrolo[1,2-*a*]pyrazine-7-carboxylate (L013-4) Diethyl 1-(2-aminoethyl)-1H pyrrole-2,4-dicarboxylate (1.5 g, 5.9 mmol) was dissolved in absolute ethanol (15 mL) under nitrogen atmosphere, and potassium carbonate (4.1 g, 29.5 mmol) was added. The mixture was reacted under reflux for 12 h. Water (50 mL) was added to quench the reaction, and ethyl acetate (20 mL × 3) was added for extraction. The organic phases were combined, washed with saturated brine (20 mL), dried over anhydrous sodium sulfate and concentrated, and the residue was separated by silica gel column chromatography (PE/EA = 1/1) to give a colorless oily liquid (600 mg, 48.9% yield). LC-MS [M+H]⁺: 209.1.

### 13.5 Preparation of compound ethyl 1,2,3,4-tetrahydropyrrolo[1,2-a]pyrazine-7-carboxylate (L013-5)

Ethyl 1-oxo-1,2,3,4-tetrahydropyrrolo[1,2-*a*]pyrazine-7-carboxylate (600 mg, 3.1 mmol) was dissolved in anhydrous tetrahydrofuran (10 mL) under hydrogen atmosphere, and borane (12.4 mL, 12.4 mmol) was added. The mixture was stirred at 80 °C for 3 h. After the mixture was cooled to room temperature, methanol (2 mL) was slowly added dropwise to quench the reaction. After stirring for 20 min, 1 N diluted hydrochloric acid (20 mL) was slowly added dropwise, followed by stirring for 20 min. Water (10 mL) was added, and ethyl acetate (20 mL) was added for extraction. The aqueous phase was adjusted to pH = 9 with 1 N NaOH solution and extracted with ethyl acetate (20 mL × 3). The organic phases were combined, washed with saturated brine (15 mL), dried over anhydrous sodium sulfate, filtered and concentrated under reduced pressure, and the resulting crude product was separated by silica gel column chromatography (DCM/MeOH = 10/1) to give a colorless oil (200 mg, 33.1% yield). MS[ M/2 + H]+ = 195.1.

### 13.6 Preparation of compound ethyl 2-(4-((4-(1H-pyrazol-4-yl)phenyl)amino)pyrimidin-2-yl)-1,2,3,4-tetrahydropyrrolo[1,2-a]pyrazine-7-carboxylate (L013-6)

Ethyl 1,2,3,4-tetrahydropyrrolo[1,2-a]pyrazine-7-carboxylate (200 mg, 1.0 mmol) was dissolved in n-butanol (5 mL) under nitrogen atmosphere, and tert-butyl 4-(4-((2-chloropyrimidin-4-yl)amino)phenyl)-1*H-*pyrazole-1-carboxylate (408 mg, 1.1 mmol) and DIEA (400 mg, 3.1 mmol) were added. The mixture was stirred at 120 °C for 12 h. Water (20 mL) was added to quench the reaction, and ethyl acetate (20 mL × 3) was added for extraction. The organic phases were combined, washed with saturated brine (30 mL), dried over anhydrous sodium sulfate, filtered and concentrated by rotary evaporation under reduced pressure, and the resulting crude product was purified by silica gel column chromatography (DCM/MeOH = 20/1) to give a light yellow solid (200 mg, 46.5% yield). LC-MS[M+H]⁺: 430.1.

### 13.7 Preparation of compound 2-(4-((4-(1H-pyrazol-4-yl)phenyl)amino)pyrimidin-2-yl)-1,2,3,4-tetrahydropyrrolo[1,2-a]pyrazine-7-carboxylic acid (L013)

Ethyl 2-(4-((4-(1*H*-pyrazol-4-yl)phenyl)amino)pyrimidin-2-yl)-1,2,3,4-tetrahydropyrrolo[1,2-*a*]pyrazine-7-carboxylate (200 mg, 0.5 mmol) was dissolved in methanol/water/tetrahydrofuran (1/1/3 = 10 mL), and lithium hydroxide (100 mg, 2.5 mmol) was added. The mixture was reacted overnight at room temperature. Then the mixture was concentrated under reduced pressure, and 1 N diluted hydrochloric acid was added to adjust the pH to 5-6. The resulting mixture was concentrated under reduced pressure to give a yellow solid (400 mg, crude product). LC-MS [M+H]⁺: 402.0.

### Example 14. Preparation of Compound 6-(4-((4-(1H pyrazol-4-yl)phenyl)amino)pyrimidin-2-yl)-N (3,3-difluorocyclobutyl)-N,1-dimethyl-4,5,6,7-tetrahydro-1H-pyrrolo[2,3-c]pyridine-2-carboxamide (L014)

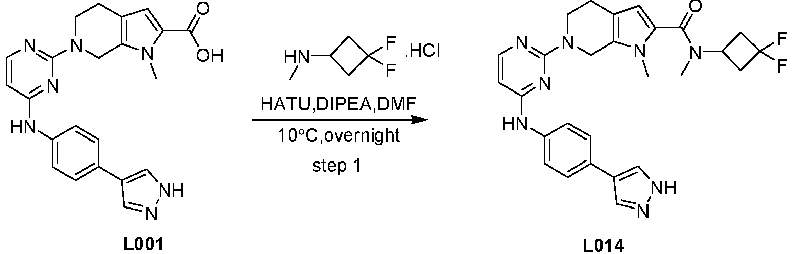

6-(4-((4-(1*H*-pyrazol-4-yl)phenyl)amino)pyrimidin-2-yl)-1-methyl-4,5,6,7-tetrahydro-1*H*-pyrrolo[2,3-c]pyridine-2-carboxylic acid (L001) (200 mg, 0.71 mmol) was dissolved in *N*,*N*-dimethylformamide (6 mL), and HATU (274 mg, 0.72 mmol), 3,3-difluoro-N-methylcyclobutane-1-amine hydrochloride (M001) (87 mg, 0.72 mmol) and 7V,7V-diisopropylethylamine (248 mg, 1.92 mmol) were added. The mixture was stirred overnight at room temperature. Water (20 mL) was added to quench the reaction, and ethyl acetate (10 mL × 3) was added for extraction. The organic phases were combined, washed with saturated brine (10 mL × 3), dried over anhydrous sodium sulfate, filtered and concentrated. The resulting crude product was purified by silica gel column chromatography (dichloromethane:ethyl acetate = 1:1), and the product was collected and lyophilized to give 6-(4-((4-(1*H*-pyrazol-4-yl)phenyl)amino)pyrimidin-2-yl)-*N*-(3,3-difluorocyclobutyl)-*N*,1-dimethyl-4,5,6,7-tetrahydro-1*H*-pyrrolo[2,3-*c*]pyridine-2-carboxamide in the form of a white solid (24.4 mg, 9.8% yield). LC-MS [M+H]⁺: 519.1.

¹H NMR (400 MHz, DMSO) δ 12.85 (brs, 1H), 10.20 (brs, 1H) 8.05 (s, 2H), 7.94 (d, J = 6.5 Hz, 1H), 7.64 (s, 4H), 6.28 - 6.24 (m, 2H), 4.83 (s, 2H), 4.70 - 4.60 (m, 1H), 4.02 - 3.96 (m, 2H), 3.58 (s, 3H), 3.00 (s, 3H), 2.95 - 2.80 (m, 4H), 2.66 - 2.60 (m, 2H).

### Example 16. Preparation of Compound (3,3-difluoroazetidin-1-yl)(6-(4-((3-fluoro-4-(3-fluoro-1H pyrazol-4-yl)phenyl)amino)pyrimidin-2-yl)-1-methyl-4,5,6,7-tetrahydro-1H-pyrrolo[2,3-c]pyridin-2-yl)methanone

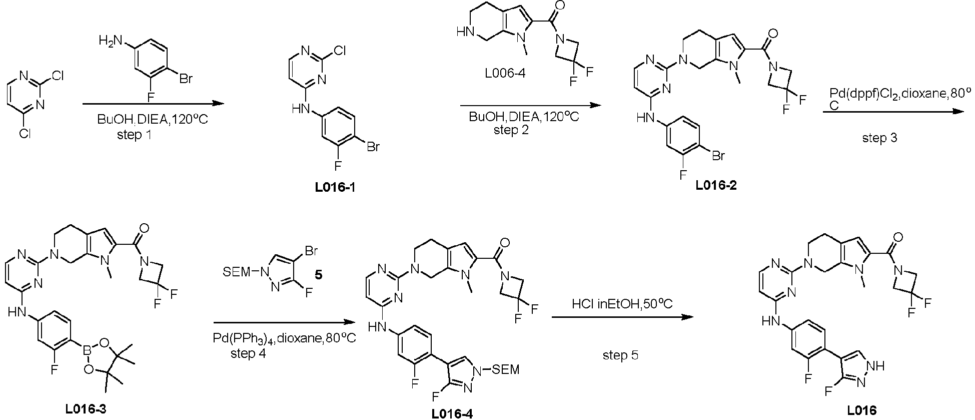

### 16.1 Preparation of compound N-(4-bromo-3-fluorophenyl)-2-chloropyrimidin-4-amine (L016-1):

2,4-dichloropyrimidine (1000 mg, 6.7 mmol) was dissolved in n-butanol (5 mL) under nitrogen atmosphere, and 4-bromo-3-fluoroaniline (1274 mg, 6.7 mmol) and DIEA (2601 mg, 20.1 mmol) were added. The mixture was warmed to 120 °C and reacted for 8 h. Water (20 mL) was added to quench the reaction, and ethyl acetate (20 mL × 3) was added for extraction. The organic phases were combined, washed with saturated brine (20 mL × 2), dried over anhydrous sodium sulfate, filtered and concentrated. The resulting crude product was separated by silica gel column chromatography (petroleum ether/ethyl acetate = 5/1) to give a yellow solid (600 mg, 29.6% yield). LC-MS [M+H]⁺: 301.9/303.9.

### 16.2 Preparation of compound (6-(4-((4-bromo-3-fluorophenyl)amino)pyrimidin-2-yl)-1-methyl-4,5,6,7-tetrahydro-1H-pyrrolo[2,3-c]pyridin-2-yl)(3,3-difluoroazetidin-1-yl)methanone (L016-2):

*N*-(4-bromo-3-fluorophenyl)-2-chloropyrimidin-4-amine (500 mg, 1.6 mmol) was dissolved in n-butanol (5 mL), and (3,3-difluoroazetidin-1-yl)(1-methyl-4,5,6,7-tetrahydro-1*H*-pyrrolo[2,3-*c*]pyridin-2-yl)methanone (421 mg, 1.6 mmol) and DIEA (639 mg, 4.9 mmol) were added. The mixture was warmed to 120 °C and reacted for 8 h. Water (20 mL) was added to quench the reaction, and ethyl acetate (20 mL × 3) was added for extraction. The organic phases were combined, washed with saturated brine (20 mL × 2), dried over anhydrous sodium sulfate, filtered and concentrated. The resulting crude product was separated by silica gel column chromatography (petroleum ether/ethyl acetate = 2/1) to give a yellow solid (450 mg). LC-MS [M+H]⁺: 520.7.

### 16.3 Preparation of compound (3,3-difluoroazetidin-1-yl)(6-(4-((3-fluoro-4-(4,4,5,5-tetramethyl-1,3,2-dioxaborolan-2-yl)phenyl)amino)pyrimidin-2-yl)-1-methyl-4,5,6,7-tetrahydro-1H-pyrrolo[2,3-c]pyridin-2-yl)methanone (L016-3):

(6-(4-((4-bromo-3-fluorophenyl)amino)pyrimidin-2-yl)-1-methyl-4,5,6,7-tetrahydro-1H pyrrolo[2,3-e]pyridin-2-yl)(3,3-difluoroazetidin-1-yl)methanone (500 mg, 0.9 mmol) was dissolved in 1,4-dioxane (10 mL), and tert-butyl 4-(4,4,5,5-tetramethyl-1,3,2-dioxaborolan-2-yl)-1*H*-pyrazole-1-carboxylate (366 mg, 1.4 mmol), Pd(dppf)Cl₂ (70 mg, 0.09 mmol) and potassium acetate (282 mg, 2.9 mmol) were added. The mixture was reacted for 12 h at 80 °C under nitrogen atmosphere. Water (10 mL) was added for dilution, and ethyl acetate (20 mL × 3) was added for extraction. The organic phases were combined, washed with saturated brine (20 mL), dried over anhydrous sodium sulfate, filtered and concentrated, and the resulting crude product was separated by silica gel column chromatography (wet loading, PE/EA = 5/1) to give a brown oily liquid (500 mg). MS [M+H]⁺ = 569.2.

### 16.4 Preparation of compound (3,3-difluoroazetidin-1-yl)(6-(4-((3-fluoro-4-(3-fluoro-1-((2-(trimethylsilyl)ethoxy)methyl)-1H pyrazol-4-yl)phenyl)amino)pyrimidin-2-yl)-1-methyl-4,5,6,7-tetrahydro-1H-pyrrolo[2,3-c]pyridin-2-yl)methanone (L016-4):

(3,3-difluoroazetidin-1-yl)(6-(4-((3-fluoro-4-(4,4,5,5-tetramethyl-1,3,2-dioxaborolan-2-yl)phenyl)amino)pyrimidin-2-yl)-1-methyl-4,5,6,7-tetrahydro-1H pyrrolo[2,3-c]pyridin-2-yl)methanone (500 mg, 0.9 mmol) was dissolved in 1,4-dioxane (8 mL) and water (2 mL), and 4-bromo-5-fluoro-1-((2-(trimethylsilyl)ethoxy)methyl)-1*H*-pyrazole (426 mg, 1.4 mmol), Pd(PPh₃)₄ (111 mg, 0.09 mmol) and potassium carbonate (398 mg, 2.9 mmol) were added. The mixture was reacted at 90 °C for 4 h under nitrogen atmosphere. Water (20 mL) was added for dilution, and ethyl acetate (20 mL × 3) was added for extraction.

The organic phases were combined, washed with saturated brine (20 mL), dried over anhydrous sodium sulfate, filtered and concentrated, and the resulting crude product was separated by silica gel column chromatography (PE/EA = 1/1) to give a yellow oily liquid (100 mg). MS [M+H]⁺ = 657.0.

### 16.5 Preparation of compound (3,3-difluoroazetidin-1-yl)(6-(4-((3-fluoro-4-(3-fluoro-1H pyrazol-4-yl)phenyl)amino)pyrimidin-2-yl)-1-methyl-4,5,6,7-tetrahydro-1H-pyrrolo[2,3-c]pyridin-2-yl)methanone (L016-5):

(3,3-difluoroazetidin-1-yl)(6-(4-((3-fluoro-4-(3-fluoro-1-((2-(trimethylsilyl)ethoxy)methyl)-1H pyrazol-4-yl)phenyl)amino)pyrimidin-2-yl)-1-methyl-4,5,6,7-tetrahydro-1*H*-pyrrolo[2,3-*c*]pyridin-2-yl)methanone

(150 mg, 0.2 mmol) was dissolved in ethanol (5 mL), and a solution of hydrochloric acid in ethanol (2 mL, 33% mass fraction) was added. The mixture was reacted at room temperature for 2 h under nitrogen atmosphere. Then the mixture was concentrated under reduced pressure, and the residue was separated by preparative chromatography to give a white solid (7.6 mg). MS [M+H]⁺ = 527.0.

MS [M +H]⁺ = 527.0. 1H NMR (400 MHz, DMSO) δ 12.68 (s, 1H), 9.63 (s, 1H), 8.03 (d, J = 5.7 Hz, 1H), 7.99 - 7.91 (m, 2H), 7.51 (t, J = 8.6 Hz, 1H), 7.34 (d, J = 6.9 Hz, 1H), 6.47 (s, 1H), 6.12 (d, J = 5.7 Hz, 1H), 4.81 (s, 2H), 4.65-4.65 (m, 4H), 3.99 (t, J = 5.6 Hz, 2H), 3.77 (s, 3H), 2.61-2.55 (m, 2H).

### Example 17. Preparation of Compound 6-(4-((4-(1H-pyrazol-4-yl)phenyl)amino)pyrimidin-2-yl)-1-methyl-N-(2,2,2-trifluoroethyl)-4,5,6,7-tetrahydro-1H-pyrrolo[2,3-c]pyridine-2-carboxamide (L017)

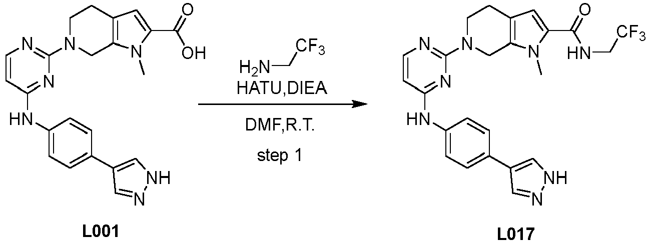

6-(4-((4-(1*H*-pyrazol-4-yl)phenyl)amino)pyrimidin-2-yl)-1-methyl-4,5,6,7-tetrahydro-1*H*-pyrrolo[2,3-c]pyridine-2-carboxylic acid (70 mg, 0.17 mmol) was dissolved in *N*,*N*-dimethylformamide (5 mL), and 2,2,2-trifluoroethane-1-amine (25 mg, 0.25 mmol), 2-(7-azabenzotriazol)-*N*,*N*,*N'*,*N'*-tetramethyluronium hexafluorophosphate (96 mg, 0.25 mmol) and 7V,7V-diisopropylethylamine (110 mg, 0.84 mmol) were added successively. The mixture was stirred and reacted overnight at room temperature. After the reaction was completed, water (20 mL) was added to quench the reaction, and ethyl acetate (15 mL × 3) was added for extraction. The organic phases were combined, washed with saturated brine (30 mL × 3), dried over anhydrous sodium sulfate, filtered and concentrated by rotary evaporation, and the resulting crude product was purified by silica gel column chromatography (4 g, dichloromethane:methanol = 20:1) and lyophilized to give 7-(4-((4-(1H pyrazol-4-yl)phenyl)amino)pyrimidin-2-yl)-N (3,3-difluorocyclobutyl)-5,6,7,8-tetrahydroimidazo[1,2-*a*]pyrazine-2-carboxamide in the form a white solid (6.7 mg, 5.39% yield). LC-MS [M+H]⁺: 497.0.

¹H NMR (301 MHz, CD3OD) δ 8.00 (s, 2H), 7.92 (d, J = 5.9 Hz, 1H), 7.74 - 7.56 (m, 4H), 6.72 (s, 1H), 6.20 (d, J = 6.1 Hz, 1H), 4.85 (s, 2H), 4.03 (dd, J = 18.8, 9.1 Hz, 4H), 3.88 (s, 3H), 2.72 (s, 2H).

### Example 19. Preparation of Compound (3,3-difluoroazetidin-1-yl)(6-(4-(4-(3-fluoro-1H pyrazol-4-yl)benzyl)pyrimidin-2-yl)-1-methyl-4,5,6,7-tetrahydro-1H-pyrrolo[2,3-c]pyridin-2-yl)methanone (L019)

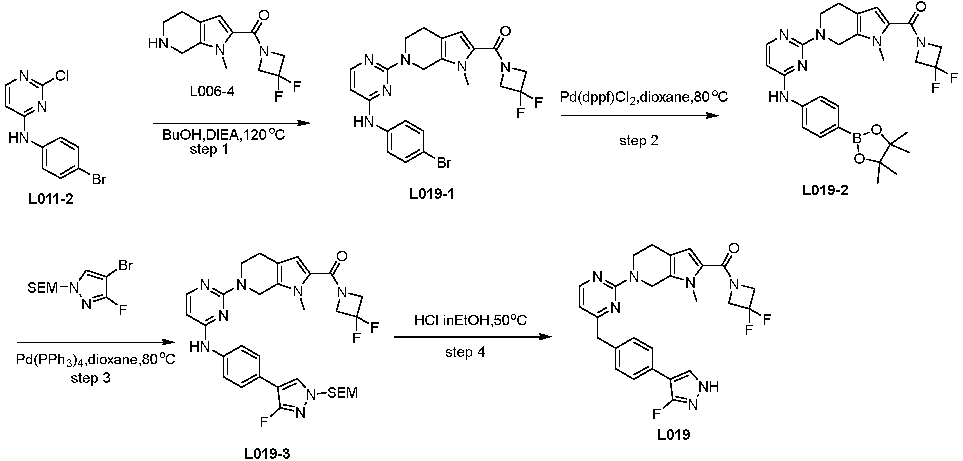

### 19.1 Preparation of compound (6-(4-((4-bromophenyl)amino)pyrimidin-2-yl)-1-methyl-4,5,6,7-tetrahydro-1H-pyrrolo[2,3-c]pyridin-2-yl)(3,3-difluoroazetidin-1-yl)methanone (L019-1):

*N*-(4-bromophenyl)-2-chloropyrimidin-4-amine (150 mg, 0.5 mmol) was dissolved in n-butanol (5 mL), and (3,3-difluoroazetidin-1-yl)(1-methyl-4,5,6,7-tetrahydro-1*H*-pyrrolo[2,3-*c*]pyridin-2-yl)methanone (127 mg, 0.5 mmol) and DIEA (129 mg, 1 mmol) were added. The mixture was warmed to 120 °C and reacted for 8 h.

Water (20 mL) was added to quench the reaction, and ethyl acetate (20 mL × 3) was added for extraction.

The organic phases were combined, washed with saturated brine (20 mL × 2), dried over anhydrous sodium sulfate, filtered and concentrated. The resulting crude product was separated by silica gel column chromatography (petroleum ether/ethyl acetate = 5/1) to give a yellow solid (500 mg). LC-MS [M+H]⁺: 503.0.

### 19.2 Preparation of compound (3,3-difluoroazetidin-1-yl)(1-methyl-6-(4-((4-(4,4,5,5-tetramethyl-1,3,2-dioxaborolan-2-yl)phenyl)amino)pyrimidin-2-yl)-4,5,6,7-tetrahydro-1H-pyrrolo[2,3-c]pyridin-2-yl)methanone (L019-2):

(6-(4-((4-bromophenyl)amino)pyrimidin-2-yl)-1-methyl-4,5,6,7-tetrahydro-1*H*-pyrrolo[2,3-*c*]pyridin-2-yl)(3,3-difluoroazetidin-1-yl)methanone (200 mg, 0.4 mmol) was dissolved in 1,4-dioxane (10 mL), and *tert-*butyl 4-(4,4,5,5-tetramethyl-1,3,2-dioxaborolan-2-yl)-1*H*-pyrazole-1-carboxylate (152 mg, 0.6 mmol), Pd(dppf)Cl2 (29 mg, 0.04 mmol) and potassium acetate (78 mg, 0.8 mmol) were added. The mixture was reacted at 80 °C for 12 h under nitrogen atmosphere. Water (10 mL) was added for dilution, and ethyl acetate (20 mL × 3) was added for extraction. The organic phases were combined, washed with saturated brine (20 mL), dried over anhydrous sodium sulfate, filtered and concentrated, and the resulting crude product was separated by silica gel column chromatography (wet loading, PE/EA = 5/1) to give a brown oily liquid (200 mg). MS [M+H]⁺ = 551.2.

### 19.3 Preparation of compound (3,3-difluoroazetidin-1-yl)(6-(4-((4-(3-fluoro-1-((2-(trimethylsilyl)ethoxy)methyl)-1H-pyrazol-4-yl)phenyl)amino)pyrimidin-2-yl)-1-methyl-4,5,6,7-tetrahydro-1H-pyrrolo[2,3-c]pyridin-2-yl)methanone (L019-3):

(3,3-difluoroazetidin-1-yl)(1-methyl-6-(4-((4-(4,4,5,5-tetramethyl-1,3,2-dioxaborolan-2-yl)phenyl)amino)pyrimidin-2-yl)-4,5,6,7-tetrahydro-1*H*-pyrrolo[2,3-*c*]pyridin-2-yl)methanone (200 mg, 0.4 mmol) was dissolved in 1,4-dioxane (8 mL) and water (2 mL), and 4-bromo-5-fluoro-1-((2-(trimethylsilyl)ethoxy)methyl)-1*H*-pyrazole (177 mg, 0.6 mmol), Pd(PPh₃)₄ (46 mg, 0.04 mmol) and potassium carbonate (110 mg, 0.8 mmol) were added. The mixture was reacted at 90 °C for 4 h under nitrogen atmosphere. Water (20 mL) was added for dilution, and ethyl acetate (20 mL × 3) was added for extraction.

The organic phases were combined, washed with saturated brine (20 mL), dried over anhydrous sodium sulfate, filtered and concentrated, and the resulting crude product was separated by silica gel column chromatography (PE/EA = 5/1) to give a yellow oily liquid (100 mg). MS [M+H]⁺ = 639.0.

### 19.4 Preparation of compound (3,3-difluoroazetidin-1-yl)(6-(4-(4-(3-fluoro-1H-pyrazol-4-yl)benzyl)pyrimidin-2-yl)-1-methyl-4,5,6,7-tetrahydro-1H-pyrrolo[2,3-c]pyridin-2-yl)methanone (L019):

(3,3-difluoroazetidin-1-yl)(6-(4-((4-(3-fluoro-1-((2-(trimethylsilyl)ethoxy)methyl)-1H pyrazol-4-yl)phenyl)amino)pyrimidin-2-yl)-1-methyl-4,5,6,7-tetrahydro-1*H*-pyrrolo[2,3-*c*]pyridin-2-yl)methanone (100 mg, 0.2 mmol) was dissolved in ethanol (5 mL), and a solution of hydrochloric acid in ethanol (2 mL, 33% mass fraction) was added. The mixture was reacted at room temperature for 2 h under nitrogen atmosphere. Then the mixture was concentrated under reduced pressure, and the residue was separated by preparative chromatography to give a white solid (3.5 mg). MS [M+H]⁺ = 509.0.

¹H NMR (400 MHz, MeOD) δ 7.95 (d, J = 2.3 Hz, 1H), 7.81 (d, J = 7.1 Hz, 1H), 7.64 (s, 4H), 6.49 (s, 1H), 6.34 (d, J = 7.0 Hz, 1H), 4.84 (s, 1H), 4.65-4.48 (m, 4H), 4.06-3.98 (m, 2H), 3.80 (s, 3H), 2.75 (t, J = 5.5 Hz, 2H).

### Example 20. Preparation of Compound (6-(4-((4-(1H-pyrazol-4-yl)phenyl)amino)pyrimidin-2-yl)-1,4,4-trimethyl-4,5,6,7-tetrahydro-1H-pyrrolo[2,3-c]pyridin-2-yl)(3,3-difluoroazetidin-1-yl)methanone (L020)

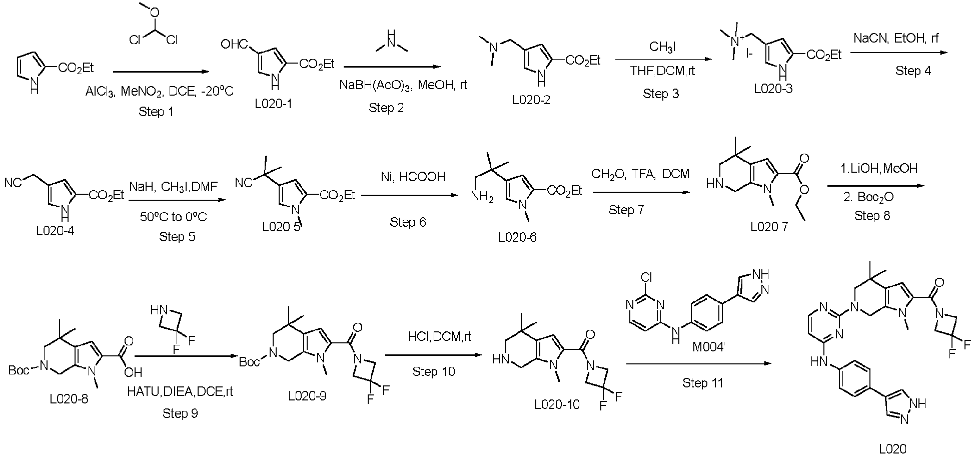

### 20.1 Preparation of compound ethyl 4-formyl-1H-pyrrole-2-carboxylate (L020-1):

Ethyl pyrrole-2-carboxylate (6.9 g, 49.6 mmol) and aluminum trichloride (13.2 g, 99.2 mmol) were added to 1,2-dichloroethane-nitromethane (1:1, 100 mL), and the mixture was cooled to -20 °C, and dichloro(methoxy)methane (11.4 g, 99.2 mmol) was added dropwise. The resulting mixture was stirred for 1 h and then left to stand at -20 °C overnight. The reaction liquid was poured into ice water (100 mL). The organic phase was separated out, and the aqueous layer was extracted with DCM (100 mL × 3). The resulting organic phases were washed with aqueous ammonia (200 mL), dried over sodium sulfate and concentrated under reduced pressure, and the resulting crude product was subjected to silica gel column chromatography (petroleum ether:ethyl acetate = 1:1) to give ethyl 4-formyl-1*H*-pyrrole-2-carboxylate in the form of a yellow solid (5.0 g, 54% yield). MS:[M+H]⁺ = 168.0.

### 20.2 Preparation of compound ethyl 4-((dimethylamino)methyl)-1H-pyrrole-2-carboxylate (L020-2):

4-formyl-1*H*-pyrrole-2-carboxylate (5.0 g, 29.9 mmol) and dimethylamine (2 N, in THF, 0.45 mL) were added to MeOH (3 mL) at room temperature, and sodium triacetoxyborohydride (381.5 mg, 1.8 mmol) was added in batches. The mixture was stirred for 16 h and concentrated under reduced pressure. Water (100 mL) was added for washing, and DCM (50 mL × 3) was added for extraction. Na²CO₃ was added to adjust the pH to 12, and then the mixture was extracted with DCM (50 mL × 3) to give ethyl 4-((dimethylamino)methyl)-1*H* pyrrole-2-carboxylate in the form of a colorless oily liquid (4.3 g). MS:[M+H]⁺ = 197.1.

20.3 Preparation of compound 1-(5-(ethoxycarbonyl)-1*H-*pyrrol-3-yl)-*N*,*N*,*N*-trimethylformamide (L020-3): Ethyl 4-((dimethylamino)methyl)-1*H*-pyrrole-2-carboxylate (4.3 g, 21.9 mmol) was dissolved in a solvent (THF:DCM = 1:1, 80 mL) at room temperature, and iodomethane (15.5 g, 11.0 mmol) was added. The mixture was stirred for 15 h and concentrated under reduced pressure. Ethyl acetate was added, and the mixture was stirred for 1 h. Suction filtration was carried out to give 1-(5-(ethoxycarbonyl)-1*H*-pyrrol-3-yl)-*N,N,N*-trimethylformamide in the form of a white solid (4.7 g, 63% yield). MS:[M+H]⁺ = 211.0.

### 20.4 Preparation of compound ethyl 4-(cyanomethyl)-1H-pyrrole-2-carboxylate (L020-4):

1-(5-(ethoxycarbonyl)-1*H*-pyrrol-3-yl)-*N,N*,*N*-trimethylformamide (4.67 g, 13.8 mmol) was added to ethanol (30 mL), and NaCN (2.6 g, 53.1 mmol) was added. The mixture was heated to 78 °C and refluxed for 15 h, and then concentrated under reduced pressure. Water (20 mL) was added for washing, and ethyl acetate (20 mL × 3) was added for extraction. The organic phase was concentrated, and the resulting crude product was subjected to silica gel column chromatography (dichloromethane:methanol = 20:1) to give ethyl 4-(cyanomethyl)-1*H*-pyrrole-2-carboxylate in the form of a colorless oily liquid (2.0 g, 81% yield). MS:[M+H]⁺ = 179.0.

### 20.5 Preparation of compound ethyl 4-(2-cyanopropan-2-yl)-1-methyl-1H-pyrrole-2-carboxylate (L020-5):

Ethyl 4-(cyanomethyl)-1*H-*pyrrole-2-carboxylate (300 mg, 1.68 mmol) was added to *N*,*N*-dimethylformamide (5 mL) under nitrogen atmosphere, and the mixture was cooled to -50 °C. Sodium hydride (336.0 g, 8.4 mmol) was added, and iodomethane (1.19 g, 8.4 mmol) was added dropwise. The resulting mixture was stirred for 0.5 h, warmed to -5 °C, and then stirred for 3 h. Saturated NH4Cl (20 mL) was added, and ethyl acetate (20 mL × 3) was added for extraction. The organic phase was concentrated, and the resulting crude product was subjected to silica gel column chromatography (PE:EtOAc = 10:1) to give ethyl 4-(2-cyanopropan-2-yl)-1-methyl-1*H*-pyrrole-2-carboxylate in the form of a colorless oily liquid (250 mg, 67% yield). MS:[M+H]⁺ = 221.0.

### 20.6 Preparation of compound ethyl 4-(1-amino-2-methylpropan-2-yl)-1-methyl-1H-pyrrole-2-carboxylate (L020-6):

Ethyl 4-(1-cyano-1-methylethyl)-1-methylpyrrole-2-carboxylate (50 mg, 0.23 mmol) was added to formic acid (2 mL), and Ni (0.3 mL) was added. The mixture was heated to 100 °C and stirred for 2 h. Then the mixture was cooled to room temperature and filtered, and the filtrate was concentrated under reduced pressure to give ethyl 4-(1-amino-2-methylpropan-2-yl)-1-methyl-1*H*-pyrrole-2-carboxylate in the form of a colorless oily liquid (50 mg). MS:[M+H]⁺ = 225.0.

### 20.7 Preparation of compound ethyl 1,4,4-trimethyl-4,5,6,7-tetrahydro-1H-pyrrolo[2,3-c]pyridine-2-carboxylate (L020-7):

Ethyl 4-(1-amino-2-methylpropan-2-yl)-1-methyl-1*H*-pyrrole-2-carboxylate (50 mg, 0.22 mmol) and trifluoroacetic acid (50.17 mg, 0.44 mmol) were added to dichloromethane (1 mL) at room temperature, and aqueous formaldehyde solution (13 mg, 0.45 mmol) was added. The mixture was stirred for 4 h and concentrated under reduced pressure to give ethyl 1,4,4-trimethyl-4,5,6,7-tetrahydro-1H pyrrolo[2,3-*c*]pyridine-2-carboxylate in the form of a colorless oily liquid (50 mg). MS: [ M+H]⁺ = 237.0.

### 20.8 Preparation of compound 6-(tert-butoxycarbonyl)-1,4,4-trimethyl-4,5,6,7-tetrahydro-1H-pyrrolo[2,3-c]pyridine-2-carboxylic acid (L020-8):

Ethyl 1,4,4-trimethyl-5*H*,6*H*,7*H*pyrrolo[2,3-*c*]pyridine-2-carboxylate (50 mg, 0.21 mmol) was dissolved in methanol (2 mL) at room temperature, and lithium hydroxide (5 mL, 1 N, dissolved in water) was added.

The mixture was stirred for 20 h. Then the mixture was concentrated under reduced pressure, and Boc2O (137.5 mg, 0.63 mmol) was added. The mixture was stirred for 12 h and extracted with ethyl acetate (2 mL × 2). The resulting mixture was adjusted to PH = 5 with hydrochloric acid and extracted with DCM (5 mL × 3). The organic phase was concentrated under reduced pressure to give 6-(*tert*-butoxycarbonyl)-1,4,4-trimethyl-4,5,6,7-tetrahydro-1*H*-pyrrolo[2,3-*c*]pyridine-2-carboxylic acid in the form of a colorless oily liquid (70 mg). MS:[M+Na]⁺ = 331.0.

### 20.9 Preparation of compound tert-butyl 2-(3,3-difluoroazetidin-1-carbonyl)-1,4,4-trimethyl-1,4,5,7-tetrahydro-6H-pyrrolo[2,3-c]pyridine-6-carboxylate (L020-9):

6-(*tert*-butyl-1'{3}-oxy)-1,4,4-trimethyl-5*H*,7*H*-pyrrolo[2,3-*c*]pyridine-2-carboxylic acid (70 mg, 0.23 mmol), 3,3-difluoroazetidine (44.50 mg, 0.345 mmol) and HATU (131.2mg) were added to 1,2-dichloroethane (3 mL) at room temperature, and DIEA (89.2 mg, 0.7 mmol) was added. The mixture was stirred for 15 h. After the reaction was completed, water (5 mL) was added for washing, and ethyl acetate (5 mL × 3) was added for extraction. The organic phase was concentrated under reduced pressure, and the residue was purified by thin layer chromatography (petroleum ether/ethyl acetate) = (1:1) to give tert-butyl 2-(3,3-difluoroazetidin-1-carbonyl)-1,4,4-trimethyl-1,4,5,7-tetrahydro-6H pyrrolo[2,3-c]pyridine-6-carboxylate in the form of a colorless oily liquid (35 mg, 49% yield). MS:[M+H]⁺ = 384.0.

### 20.10 Preparation of compound (3,3-difluoroazetidin-1-yl)(1,4,4-trimethyl-4,5,6,7-tetrahydro-1H-pyrrolo[2,3-c]pyridin-2-yl)methanone (L020-10):

Tert-butyl 2-(3,3-difluoroazetidin-1-carbonyl)-1,4,4-trimethyl-1,4,5,7-tetrahydro-6H pyrrolo[2,3-c]pyridine-6-carboxylate (35 mg, 0.09 mmol) was dissolved in dichloromethane (3 mL) at room temperature, and HCl (1 mL, 4 N, dissolved in 1,4 dioxane) was added. The mixture was stirred for 1 h and concentrated under reduced pressure to give (3,3-difluoroazetidin-1-yl)(1,4,4-trimethyl-4,5,6,7-tetrahydro-1H pyrrolo[2,3-c]pyridin-2-yl)methanone in the form of a white solid (30 mg). MS:[M+H]⁺ = 284.0.

### 20.11 Preparation of compound (6-(4-((4-(1H-pyrazol-4-yl)phenyl)amino)pyrimidin-2-yl)-1,4,4-trimethyl-4,5,6,7-tetrahydro-1H-pyrrolo[2,3-c]pyridin-2-yl)(3,3-difluoroazetidin-1-yl)methanone (L020):

(3,3-difluoroazetidin-1-yl)(1,4,4-trimethyl-4,5,6,7-tetrahydro-1*H*pyrrolo[2,3-*c*]pyridin-2-yl)methanone (30 mg, 0.11 mmol) and *N*-(4-(1*H*-pyrazol-4-yl)phenyl)-2-chloropyrimidin-4-amine (61.52 mg, 0.165 mmol) were dissolved in n-butanol (0.5 mL) under nitrogen atmosphere, and DIEA (71.08 mg, 0.55 mmol) was added.

The mixture was heated to 120 °C, stirred for 5 h and then concentrated under reduced pressure, and the residue was purified by high performance liquid chromatography to give (6-(4-((4-(177-pyrazol-4-yl)phenyl)amino)pyrimidin-2-yl)-1,4,4-trimethyl-4,5,6,7-tetrahydro-1H pyrrolo[2,3-c]pyridin-2-yl)(3,3-difluoroazetidin-1-yl)methanone in the form of a white solid (6.5 mg, 12% yield). MS: [ M+H]⁺ = 519.31.

¹H NMR (400 MHz, DMSO) δ 9.32 (s, 1H), 8.26 (s, 1H), 8.02 (s, 2H), 7.69 (d, J = 8.6 Hz, 2H), 7.56 (d, J = 8.6 Hz, 2H), 6.60 (s, 1H), 6.07 (d, J = 5.7 Hz, 1H), 4.74 (s, 2H), 4.58 (s, 4H), 3.76 (d, J = 3.4 Hz, 5H), 1.18 (s, 6H).

For the preparation of Compound L015 and Compound L022, reference was made to the methods described in Examples 2 and 8 above.

For the preparation of Compound L018, reference was made to the method described in Example 20 above. For the preparation of Compound L021, Compound L023 and Compound L024, reference was made to the methods described in Examples 1, 4, 5, 9,10,12 and 14 above.

The characterization data for each compound are shown in the table below:

| **Example No.** | **Compound No.** | **LC-MS: [M+H]⁺** |
|---|---|---|
| **Example 15** | **L015** | **478.1** |
| **Example 18** | **L018** | **519.1** |
| **Example 21** | **L021** | **609.3** |
| **Example 22** | **L022** | **506.3** |
| **Example 23** | **L023** | **510.3** |
| **Example 24** | **L024** | **503.3** |

### Example 25. Preparation of Compound (2-(4-((4-(1H-pyrazol-4-yl)phenyl)amino)pyrimidin-2-yl)-1,2,3,4-tetrahydropyrrolo[1,2-a]pyrazin-7-yl)(3,3-difluoroazetidin-1-yl)methanone (L025)

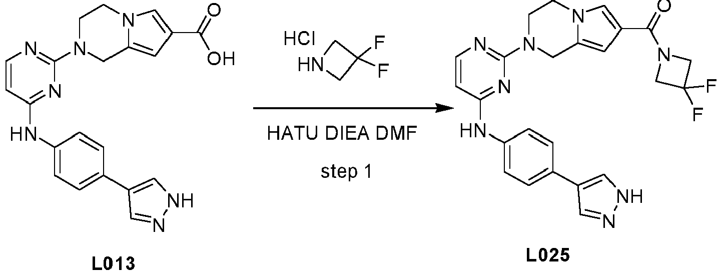

2-(4-((4-(1*H*-yrazol-4-yl)phenyl)amino)pyrimidin-2-yl)-1,2,3,4-tetrahydropyrrolo[1,2-*a*]pyrazine-7-carboxylic acid (L013) (crude product, 200 mg) was dissolved in DMF (10 mL) under nitrogen atmosphere, and 3,3-difluoroazetidine hydrochloride (39 mg, 0.3 mmol), HATU (114 mg, 0.3 mmol) and DIEA (129 mg, 1.0 mmol) were added. The mixture was reacted at room temperature for 2 h. Water (20 mL) was added to quench the reaction, and ethyl acetate (20 mL × 3) was added for extraction. The organic phases were combined, washed with saturated brine (30 mL), dried over anhydrous sodium sulfate, filtered and concentrated, and the resulting crude product was separated by column chromatography (DCM/MeOH = 10/1) to give a white solid (15 mg).

LC-MS [M+H]⁺: 476.9.

¹H NMR (400 MHz, DMSO) δ 12.86 (s, 1H), 8.04 (brs, 2H), 7.96 (d, J = 6.3 Hz, 1H), 7.75-7.57 (m, 4H), 7.32 (s, 1H), 6.30 (s, 1H), 6.24 (s, 1H), 4.86 (s, 2H), 4.71-4.41 (m, 4H), 4.17-4.05 (m, 4H).

### Example 26. Preparation of Compound (6-(4-((4-(1H pyrazol-4-yl)phenyl)amino)-5-methoxypyrimidin-2-yl)-1-methyl-4,5,6,7-tetrahydro-1H pyrrolo[2,3-c]pyridin-2-yl)(3,3-difluoroazetidin-1-yl)methanone (L026)

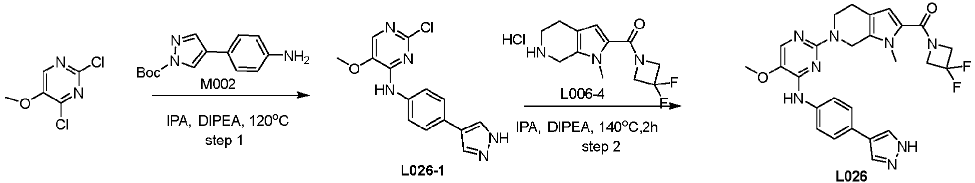

### 26.1 Preparation of compound N-(4-(1H-pyrazol-4-yl)phenyl)-2-chloro-5-methoxypyrimidin-4-amine (L026-1)

2,4-dichloro-5-methoxypyrimidine (300 mg, 1.68 mmol) was dissolved in isopropanol (5 mL), and *tert-butyl* 4-(4-aminophenyl)-1*H*-pyrazole-1-carboxylate (436 mg, 1.68 mmol) and 7V,7V-diisopropylethylamine (1.30 g,10.08 mmol) were added. The mixture was stirred at 120 °C for 16 h under nitrogen atmosphere. After the reaction was completed, water (10 mL) was added for dilution, and ethyl acetate (10 mL × 2) was added for extraction. The organic phases were combined, washed with saturated brine (20 mL), dried over anhydrous sodium sulfate and concentrated, and the residue was separated by silica gel column chromatography (DCM/MeOH = 20/1) to give a white solid (450 mg, 88% yield). LC-MS [M+H]⁺: 301.9.

26.2 Preparation of compound (6-(4-((4-(1*H*-pyrazol-4-yl)phenyl)amino)-5-methoxypyrimidin-2-yl)-1-methyl-4,5,6,7-tetrahydro-1*H*-pyrrolo[2,3-*c*]pyridin-2-yl)(3,3-difluoroazetidin-1-yl)methanone *N*-(4-(1*H*-pyrazol-4-yl)phenyl)-2-chloro-5-methoxypyrimidin-4-amine (100 mg, 0.33 mmol) was dissolved in isopropanol (0.5 mL), and (3,3-difluoroazetidin-1-yl)(1-methyl-4,5,6,7-tetrahydro-1*H*-pyrrolo[2,3*-c*]pyridin-2-yl)methanone hydrochloride (116 mg, 0.40 mmol) and 7V,7V-diisopropylethylamine (155 mg,1.20 mmol) were added. The mixture was heated to 140 °C under microwave and reacted for 2 h under nitrogen atmosphere. After the reaction was completed, water (5 mL) was added for dilution, and ethyl acetate (5 mL × 3) was added for extraction. The organic phases were combined, washed with saturated brine (10 mL × 2), dried over anhydrous sodium sulfate, filtered and concentrated, and the residue was subjected to preparative chromatography to give a white solid (26.2 mg, 15% yield). LC-MS [M+H]⁺: 520.9.

¹H NMR (400 MHz, DMSO) δ 12.88 (s, 1H), 8.72 (s, 1H), 8.15 (s, 1H), 7.90 (s, 1H), 7.83-7.78 (m, 3H), 7.56 (d, J = 8.7 Hz, 2H), 6.45 (s, 1H), 4.71 (s, 2H), 4.65-4.48 (m, 4H), 3.89 (t, J = 5.5 Hz, 2H), 3.82 (s, 3H), 3.75 (s, 3H), 2.58-2.52 (m, 2H).

### Example 27. Preparation of Compound (6-(4-((4-(1H-pyrazol-4-yl)phenyl)amino)-5-fluoropyrimidin-2-yl)-1-methyl-4,5,6,7-tetrahydro-1H-pyrrolo[2,3-c]pyridin-2-yl)(3,3-difluoroazetidin-1-yl)methanone (L027)

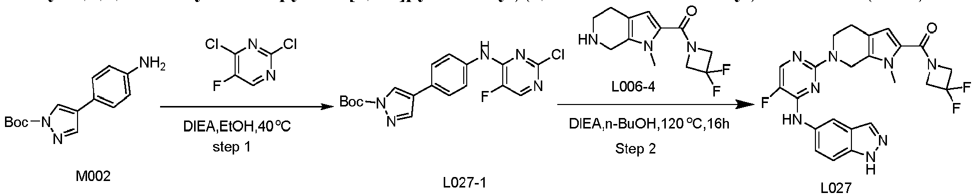

### 27.1 Preparation of compound tert-butyl 4-(4-((2-chloro-5-fhioropyrimidin-4-yl)amino)phenyl)-17/-pyrazole-1-carboxylate (L027-1):

*Tert*-butyl 4-(4-aminophenyl)-1*H*-pyrazole-1-carboxylate (M002) (1.8 g, 6.9 mmol) was dissolved in ethanol (20 mL), and 2,4-dichloro-5-fluoropyrimidine (2.3 g, 13.8 mmol) and DIEA (3.6 g, 27.6 mmol) were added successively. The mixture was stirred and reacted at 40 °C for 2 h. After the reaction was completed, the mixture was cooled to room temperature, water (20 mL) was added to quench the reaction, and ethyl acetate (15 mL × 3) was added for extraction. The organic phases were combined, washed with saturated brine (30 mL × 2), dried over anhydrous sodium sulfate, filtered and concentrated by rotary evaporation. The resulting crude product was purified by silica gel column chromatography (PE/EA = 3/1) to give a brown solid (2.3 g, 85.2% yield). LC-MS [M+H]⁺: 389.9

### 27.2 Preparation of compound (6-(4-((4-(1H-pyrazol-4-yl)phenyl)amino)-5-fluoropyrimidin-2-yl)-1-methyl-4,5,6,7-tetrahydro-1H-pyrrolo[2,3-c]pyridin-2-yl)(3,3-difluoroazetidin-1-yl)methanone (L027):

*Tert*-butyl 4-(4-((2-chloro-5-fluoropyrimidin-4-yl)amino)phenyl)-1*H*-pyrazole-1-carboxylate (1.6 g, 12.3 mmol) was dissolved in *n*-butanol (16 mL), and (3,3-difluoroazetidin-1-yl)(1-methyl-4,5,6,7-tetrahydro-1*H-*pyrrolo[2,3-*c*]pyridin-2-yl)methanone hydrochloride (784 mg, 3.1 mmol) and DIEA (1.2 g, 3.1 mmol) were added to the system. The mixture was stirred overnight at 120 °C. After the reaction was completed, the mixture was cooled to room temperature, water (20 mL) was added to quench the reaction, and ethyl acetate (20 mL × 3) was added for extraction. The organic phases were combined, washed with saturated brine (20 mL × 3), dried over anhydrous sodium sulfate, filtered and concentrated by rotary evaporation, and the resulting crude product was subjected to silica gel column chromatography (DCM/MeOH = 20/1) and lyophilized to give a white solid (600 mg). LC-MS [M+H]⁺: 508.7

¹H NMR (400 MHz, DMSO) δ 12.90 (s, 1H), 9.34 (s, 1H), 8.16 (s, 1H), 8.06 (d, J = 3.7 Hz, 1H), 7.91 (s, 1H), 7.76 (d, J = 8.7 Hz, 2H), 7.59 (d, J = 8.7 Hz, 2H), 6.46 (s, 1H), 4.73 (s, 2H), 4.56 (s, 4H), 3.92 (t, J = 5.5 Hz, 2H), 3.74 (s, 3H), 2.55 (t, J = 5.2 Hz, 2H).

### Example 28. Preparation of Compound (2-(4-((4-(1H-pyrazol-4-yl)phenyl)amino)pyrimidin-2-yl)-1,2,3,4-tetrahydroisoquinolin-7-yl)(3,3-difluoroazetidin-1-yl)methanone (L028)

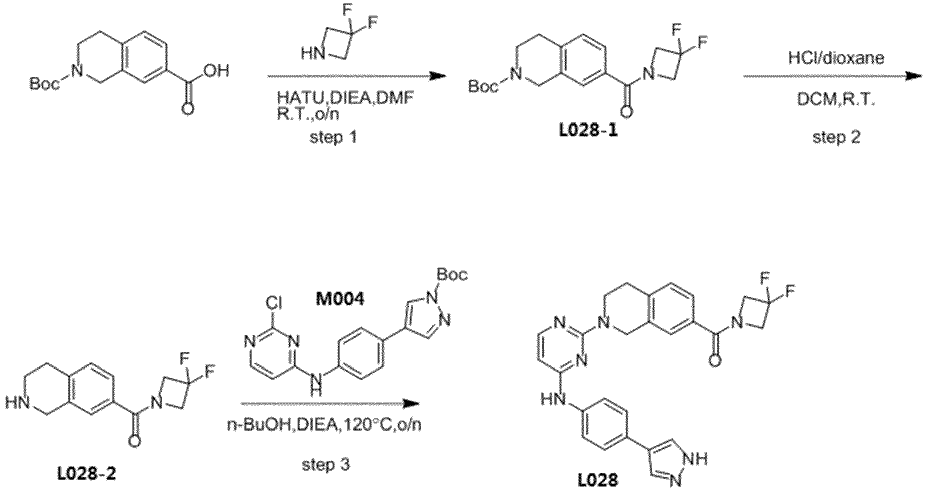

### 28.1 Preparation of compound tert-butyl 7-(3,3-difluoroazetidin-1-carbonyl)-3,4-dihydroisoquinoline-2(1H)-carboxylate (L028-1)

2-(*tert*-butoxycarbonyl)-1,2,3,4-tetrahydroisoquinoline-7-carboxylic acid (300 mg, 1.08 mmol) was dissolved in *N*,*N*-dimethylformamide (10 mL), and 3,3-difluoroazetidine (210 mg, 1.62 mmol), 2-(7-azabenzotriazol)-*N*,*N*,*N'*,*N'*-tetramethyluronium hexafluorophosphate (615 mg, 1.62 mmol) and *N*,*N*-diisopropylethylamine (557 mg, 4.32 mmol) were added successively. The mixture was stirred and reacted overnight at room temperature. After the reaction was completed, water (20 mL) was added to quench the reaction, and ethyl acetate (15 mL × 3) was added for extraction. The organic phases were combined, washed with saturated brine (30 mL × 3), dried over anhydrous sodium sulfate, filtered and concentrated by rotary evaporation, and the resulting crude product was purified by silica gel column chromatography (4 g, petroleum ether:ethyl acetate = 3:1) to give *tert*-butyl 7-(3,3-difluoroazetidin-1-carbonyl)-3,4-dihydroisoquinoline-2(1*H*)-carboxylate in the form of a white solid (350 mg, 91.80% yield). LC-MS [M+H]⁺: 375.0.

### 28.2 Preparation of compound (3,3-difluoroazetidin-1-yl)(1,2,3,4-tetrahydroisoquinolin-7-yl)methanone hydrochloride (L028-2)

*Tert*-butyl 7-(3,3-difluoroazetidin-1-carbonyl)-3,4-dihydroisoquinoline-2(1*H*)-carboxylate (300 mg, 0.85 mmol) was dissolved in dichloromethane (3 mL), and a hydrochloric acid/dioxane solution (33%, 1 mL) was added to the system. The mixture was stirred at room temperature for 3 h. After the reaction was completed as detected, the mixture was concentrated by rotary evaporation to give (3,3-difluoroazetidin-1-yl)(1,2,3,4-tetrahydroisoquinolin-7-yl)methanone hydrochloride in the form of a brown solid (270 mg, crude product), and the crude product was used directly in the next step. LC-MS [M+H]⁺: 253.0.

### 28.3 Preparation of compound (2-(4-((4-(1H-pyrazol-4-yl)phenyl)amino)pyrimidin-2-yl)-1,2,3,4-tetrahydroisoquinolin-7-yl)(3,3-difluoroazetidin-1-yl)methanone (L028)

(3, 3-difluoroazetidin-1-yl)(1,2,3,4-tetrahydroisoquinolin-7-yl)methanone hydrochloride (100 mg, 0.27 mmol) was dissolved in *n*-butanol (5 mL), and *tert*-butyl 4-(4-((2-chloropyrimidin-4-yl)amino)phenyl)-1*H-*pyrazole-1-carboxylate (81 mg, 0.32 mmol) and *N*,*N*-diisopropylethylamine (105 mg, 0.81 mmol) were added. The mixture was reacted overnight at room temperature of 120 °C. After the reaction was completed, water (20 mL) was added to quench the reaction, and ethyl acetate (15 mL × 3) was added for extraction. The organic phases were combined, washed with saturated brine (30 mL × 3), dried over anhydrous sodium sulfate, filtered and concentrated by rotary evaporation, and the resulting crude product was purified by silica gel column chromatography (4 g, dichloromethane:methanol = 20:1) and lyophilized to give (2-(4-((4-(1*H*-pyrazol-4-yl)phenyl)amino)pyrimidin-2-yl)-1,2,3,4-tetrahydroisoquinolin-7-yl)(3,3-difluoroazetidin-1-yl)methanone in the form a white solid (32.6 mg, 24.74% yield). LC-MS [M+H]⁺: 488.0.

¹H NMR (301 MHz, DMSO) δ 12.84 (s, 1H), 9.38 (s, 1H), 8.16 - 7.91 (m, 3H), 7.66 (d, J = 8.7 Hz, 2H), 7.62 - 7.52 (m, 3H), 7.49 (d, J = 7.9 Hz, 1H), 7.28 (d, J = 7.9 Hz, 1H), 6.07 (d, J = 5.8 Hz, 1H), 4.90 (s, 2H), 4.78 (s, 2H), 4.47 (s, 2H), 3.98 (t, J = 5.7 Hz, 2H), 2.92 (t, J = 5.4 Hz, 2H).

### Example 30. Preparation of Compound (6-(4-((4-(1H-pyrazol-4-yl)phenyl)amino)-6-fluoropyrimidin-2-yl)-1-methyl-4,5,6,7-tetrahydro-1H-pyrrolo[2,3-c]pyridin-2-yl)(3,3-difluoroazetidin-1-yl)methanone (L030)

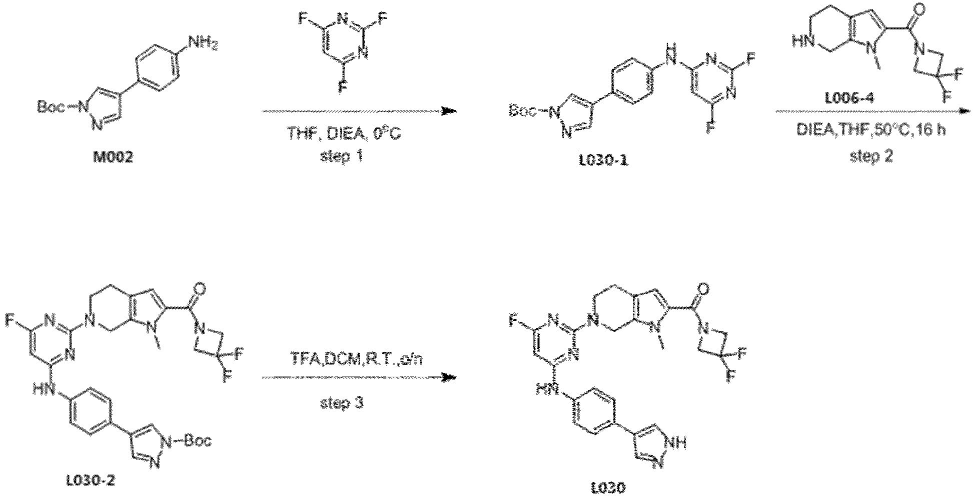

### 30.1 Preparation of compound tert-butyl 4-(4-((2,6-difluoropyrimidin-4-yl)amino)phenyl)-1H-pyrazole-1-carboxylate (L030-1)

*Tert*-butyl 4-(4-aminophenyl)-1*H*-pyrazole-1-carboxylate (500 mg, 1.93 mmol) was dissolved in anhydrous tetrahydrofuran (10 mL), and 2,4,6-trifluoropyrimidine (258 mg, 1.93 mmol) and *N*,*N*-diisopropylethylamine (747 mg, 5.79 mmol) were added successively. The mixture was stirred and reacted overnight at 0 °C. After the reaction was completed, water (20 mL) was added to quench the reaction, and ethyl acetate (15 mL × 3) was added for extraction. The organic phases were combined, washed with saturated brine (30 mL × 3), dried over anhydrous sodium sulfate, filtered and concentrated by rotary evaporation, and the resulting crude product was purified by silica gel column chromatography (4 g, petroleum ether:ethyl acetate = 3:1) to give *tert*-butyl 7-(3,3-difluoroazetidin-1-carbonyl)-3,4-dihydroisoquinoline-2(1*H*)-carboxylate in the form of a white solid (200 mg, 27.70% yield). LC-MS [M+H]⁺: 374.0.

### 30.2 Preparation of compound tert-butyl 4-(4-((2-(2-(3,3-difluoroazetidin-1-carbonyl)-1-methyl-1,4,5,7-tetrahydro-6H-pyrrolo[2,3-c]pyridin-6-yl)-6-fluoropyrimidin-4-yl)amino)phenyl)-1H-pyrazole-1-carboxylate (L030-2)

*Tert*-butyl 4-(4-((2,6-difluoropyrimidin-4-yl)amino)phenyl)-1*H*-pyrazole-1-carboxylate (120 mg, 0.32 mmol) was dissolved in acetonitrile (5 mL), and (3,3-difiuoroazetidin-1-yl)(1-methyl-4,5,6,7-tetrahydro-1*H-*pyrrolo[2,3-c]pyridin-2-yl)methanone hydrochloride (140 mg, 0.48 mmol) and *N*,*N*-diisopropylethylamine (156 mg, 1.21 mmol) were added to the system. The mixture was stirred overnight at 50 °C. After the reaction was completed, water (10 mL) was added to quench the reaction, and ethyl acetate (10 mL × 3) was added for extraction. The organic phases were combined, washed with saturated brine (20 mL × 3), dried over anhydrous sodium sulfate, filtered and concentrated by rotary evaporation, and the resulting crude product was purified by silica gel column chromatography (4 g, dichloromethane:methanol = 20:1) and lyophilized to give *tert*-butyl 4-(4-((2-(2-(3,3-difluoroazetidin-1-carbonyl)-1-methyl-1,4,5,7-tetrahydro-6H pyrrolo[2,3-*c*]pyridin-6-yl)-6-fluoropyrimidin-4-yl)amino)phenyl)-1*H*-pyrazole-1-carboxylate in the form of a white solid (130 mg, 66.71% yield). LC-MS [M+H]⁺: 609.0.

### 30.3 Preparation of compound (6-(4-((4-(1H-pyrazol-4-yl)phenyl)amino)-6-fluoropyrimidin-2-yl)-1-methyl-4,5,6,7-tetrahydro-1H pyrrolo[2,3-c]pyridin-2-yl)(3,3-difluoroazetidin-1-yl)methanone (L030)

*Tert*-butyl 4-(4-((2-(2-(3,3-difluoroazetidin-1-carbonyl)-1-methyl-1,4,5,7-tetrahydro-6H pyrrolo[2,3-*c*]pyridin-6-yl)-6-fluoropyrimidin-4-yl)amino)phenyl)-1*H*-pyrazole-1-carboxylate (130 mg, 0.21 mmol) was dissolved in dichloromethane (3 mL), and trifluoroacetic acid (1 mL) was added to the system. The mixture was stirred overnight at room temperature. After the reaction was completed, the system was concentrated by rotary evaporation, and the resulting crude product was purified by preparative chromatography (column:-Gemini-C18, 150×21.2 mm, 5 µm; mobile phase: ACN-H₂O (0.1% FA); gradient: 20-35) and lyophilized to give (6-(4-((4-(1*H*-pyrazol-4-yl)phenyl)amino)-6-fluoropyrimidin-2-yl)-1-methyl-4,5,6,7-tetrahydro-1H pyrrolo[2,3-c]pyridin-2-yl)(3,3-difluoroazetidin-1-yl)methanone in the form of a white solid (81.9 mg, 76.19% yield). LC-MS [M+H]⁺: 509.0.

¹H NMR (301 MHz, DMSO) δ 9.58 (s, 1H), 8.01 (s, 2H), 7.57 (s, 4H), 6.46 (s, 1H), 5.62 (s, 1H), 4.76 (s, 2H), 4.55 (t, J = 11.6 Hz, 4H), 3.94 (s, 2H), 3.75 (s, 3H), 2.56 (s, 2H).

### Example 31. Preparation of Compound (6-(4-((4-(1H-pyrazol-4-yl)phenyl)amino)-7,8-dihydro-5H-pyrrolo[4,3-d]pyrimidin-2-yl)-1-methyl-4,5,6,7-tetrahydro-1H-pyrrolo[2,3-c]pyridin-2-yl)(3,3-difluoroazetidin-1-yl)methanone (L031)

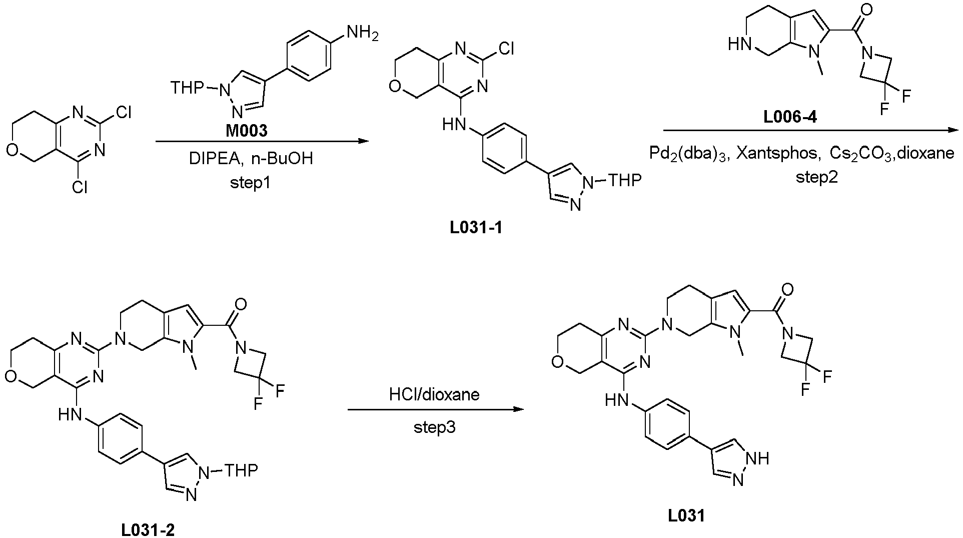

### 31.1 Preparation of compound 2-chloro-N-(4-(1-(tetrahydro-2H-pyran-2-yl)-1H-pyrazol-4-yl)phenyl)-7,8-dihydro-5H-pyran[4,3-d]pyrimidin-4-amine (L031-1):

2,4-dichloro-7,8-dihydro-5*H*-pyrano[4,3-*d*]pyrimidine (150 mg, 0.732 mmol) and 4-(1-(tetrahydro-2*H*-pyran-2-yl)-1*H*-pyrazol-4-yl)aniline (178 mg, 0.732 mmol) were dissolved in n-butanol (10 mL) successively, and DIPEA (189 mg, 1.464 mmol) was added. The reaction system was purged with N₂ three times and then stirred and reacted at 110 °C for 48 h. After the reaction was completed as detected by LCMS, the reaction liquid was cooled to room temperature and concentrated under reduced pressure, and the resulting crude product was separated and purified by column chromatography (PE/EA = 3:1-1:3) to give a tan solid (140 mg). LC-MS: [M+H]⁺ = 412.15.

### 31.2 Preparation of compound (3,3-difluoroazetidin-1-yl)(1-methyl-6-(4-((4-(1-(tetrahydro-2H-pyran-2-yl)-1H-pyrazol-4-yl)phenyl)amino)-7,8-dihydro-5H-pyrano[4,3-d]pyrimidin-2-yl)-4,5,6,7-tetrahydro-1H-pyrrolo[2,3-c]pyridin-2-yl)methanone (L031-2):

L031-1 (140 mg, 0.341 mmol), (3,3-difluoroazetidin-1-yl)(1-methyl-4,5,6,7-tetrahydro-1H pyrrolo[2,3-c]pyridin-2-yl)methanone (104 mg, 0.409 mmol), Pd₂(dba)₃ (31 mg, 0.034 mmol), Xantsphos (39 mg, 0.068 mmol) and Cs₂CO₃ (222 mg, 0.682 mmol) were each weighed out, and 1,4-dioxane was added. The reaction system was purged with N₂ and reacted overnight at 90 °C. After the reaction was completed as detected by LCMS, the reaction liquid was cooled to room temperature and concentrated by rotary evaporation to remove 1,4-dioxane. H₂O (30 mL) was added for dilution, and then EA (50 mL × 3) was added for extraction.

The EA phases were combined, washed with saturated brine, dried over anhydrous sodium sulfate and filtered, and the filtrate was concentrated under reduced pressure to give a crude product. The crude product was separated and purified by silica gel column chromatography (DCM/MeOH = 20:1) to give a yellow solid (44 mg). LC-MS: [M+H]⁺ = 631.25.

### 31.3 Preparation of compound (6-(4-((4-(1H-pyrazol-4-yl)phenyl)amino)-7,8-dihydro-5H-pyrrolo[4,3-d]pyrimidin-2-yl)-1-methyl-4,5,6,7-tetrahydro-1H pyrrolo[2,3-c]pyridin-2-yl)(3,3-difluoroazetidin-1-yl)methanone (L031):

L031-2 (39 mg, 0.063 mmol) and acidic resin (40 mg) were weighed out and placed in a 25 mL round-bottom flask, and methanol (8 mL) and 4 M HCl/dioxane (0.4 mL) were added. The mixture was reacted at room temperature for 1 h. After the reaction was completed as detected by LCMS, triethylamine was added to adjust the pH to 8, and the resulting mixture was concentrated under reduced pressure to give a crude product. The crude product was separated by prep-HPLC and lyophilized to give a white solid (2.0 mg, 95.573% purity). LC-MS: [M+H]⁺ = 547.25.

¹H NMR (400 MHz, DMSO) δ 8.05 (s, 1H), 7.60 (d, J = 9.4 Hz, 2H), 6.46 (s, 1H), 4.74 (s, 1H), 4.56 (d, J = 10.3 Hz, 3H), 3.91 (t, J = 5.5 Hz, 2H), 3.70 (s, 1H), 2.71 (s, 1H), 2.57 (s, 1H).

### Example 32. Preparation of Compound 2,2,2-trifluoroethyl 6-(4-((4-(1H-pyrazol-4-yl)phenyl)amino)pyrimidin-2-yl)-1-methyl-4,5,6,7-tetrahydro-1H-pyrrolo[2,3-c]pyridine-2-carboxylate (L032)

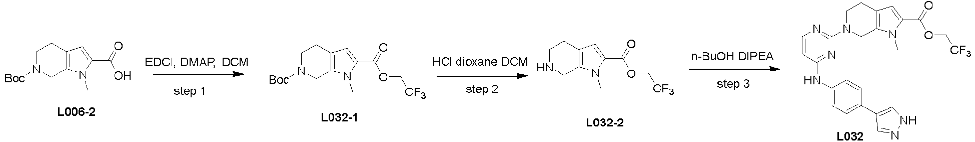

### 32.1 Preparation of compound 6-tert-butyl-2-(2,2,2-trifluoroethyl) 1-methyl-4,5-dihydro-1H pyrrolo[2,3-c]pyridine-2,6(7H)-dicarboxylate (L032-1)

6-(*tert*-butoxycarbonyl)-1-methyl-4,5,6,7-tetrahydro-1*H*-pyrrolo[2,3-*c*]pyridine-2-carboxylic acid (90 mg, 0.32 mmol) was dissolved in anhydrous dichloromethane (5 mL), and 2,2,2-trifluoroethanol (38 mg,0.38 mmol), 1-(3-dimethylaminopropyl)-3-ethylcarbodiimide hydrochloride (73 mg,0.38 mmol) and 4-dimethylaminopyridine (47 mg,0.38 mmol) were added. The mixture was stirred at room temperature for 4 h under nitrogen atmosphere. Water (10 mL) was added for dilution, and dichloromethane (5 mL × 2) was added for extraction. The organic phases were combined, washed with diluted hydrochloric acid (1 N) (5 mL × 2), washed with saturated sodium bicarbonate (5 mL × 2), then washed with saturated brine (10 mL), dried over anhydrous sodium sulfate and concentrated, and the residue was separated by silica gel column chromatography (PE/EA = 20/1) to give a colorless oily liquid (90 mg, 77% yield). LC-MS [M+H]⁺: 362.9.

### 32.2 Preparation of compound 2,2,2-trifluoroethyl 1-methyl-4,5,6,7-tetrahydro-1H-pyrrolo[2,3-c]pyridine-2-carboxylate (L032-2)

6-*tert*-butyl 2-(2,2,2-trifluoroethyl) 1-methyl-4,5-dihydro-1*H*-pyrrolo[2,3-*c*]pyridine-2,6(7*H*)-dicarboxylate (90 mg, 0.25 mmol) was dissolved in anhydrous dichloromethane (3 mL), and a solution of hydrochloric acid in dioxane (1 mL) was added. The mixture was reacted at room temperature for 2 h under nitrogen atmosphere. After the reaction was completed, the mixture was concentrated to give a crude product in the form of a light yellow solid (80 mg). LC-MS [M+H]⁺: 262.8.

### 32.3 Preparation of compound 2,2,2-trifluoroethyl 6-(4-((4-(1H-pyrazol-4-yl)phenyl)amino)pyrimidin-2-yl)-1-methyl-4,5,6,7-tetrahydro-1H-pyrrolo[2,3-c]pyridine-2-carboxylate (L032)

2,2,2-trifluoroethyl 1-methyl-4,5,6,7-tetrahydro-1*H*-pyrrolo[2,3-*c*]pyridine-2-carboxylate hydrochloride (80 mg, 0.25 mmol, crude product) was dissolved in n-butanol (2 mL), and *N*,*N*-diisopropylethylamine (97 mg, 0.75 mmol) and *N*-(4-(1*H*-pyrazol-4-yl)phenyl)-2-chloropyrimidin-4-amine (94 mg, 0.33 mmol) were added. The mixture was warmed to 120 °C and reacted for 4 h under nitrogen atmosphere. After the reaction was completed, water (5 mL) was added for dilution, and ethyl acetate (5 mL × 2) was added for extraction. The organic phases were combined, washed with saturated brine (10 mL), dried over anhydrous sodium sulfate and concentrated, and the residue was subjected to preparative chromatography to give a white solid (47.9 mg, 38% two-step yield). LC-MS [M+H]⁺: 497.8.

¹H NMR (400 MHz, DMSO) δ 10.54 (s, 1H), 8.08 (s, 2H), 7.96 (d, J = 6.9 Hz, 2H), 7.72-7.60 (m, 4H), 6.85 (s, 1H), 6.34 (d, J = 6.8 Hz, 1H), 4.95-4.82 (m, 4H), 4.03-3.93 (m, 2H), 3.80 (s, 3H), 2.72-2.63 (m, 2H).

### Example 34. Preparation of Compound 4-((4-(1H-pyrazol-4-yl)phenyl)amino)-2-(2-(3,3-difluoroazetidin-1-carbonyl)-1-methyl-4,5-dihydro-1H-pyrrolo[2,3-c]pyridin-6(7H)-yl)pyrimidine-5-carbonitrile (L034)

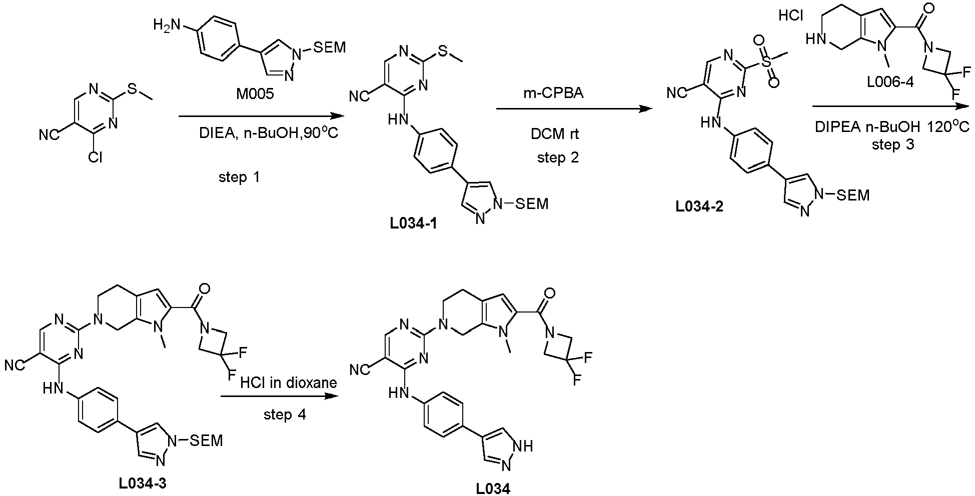

### 34.1 Preparation of compound 2-(methylthio)-4-((4-(1-((2-(trimethylsilyl)ethoxy)methyl)-1H-pyrazol-4-yl)phenyl)amino)pyrimidine-5-carbonitrile (L034-1)

4-chloro-2-(methylthio)pyrimidine-5-carbonitrile (300 mg, 1.62 mmol) was dissolved in n-butanol (4 mL), and 4-(1-((2-(trimethylsilyl)ethoxy)methyl)-1*H*-pyrazol-4-yl)aniline (468 mg, 1.62 mmol) and *N,N-*diisopropylethylamine (418 mg, 3.24 mmol) were added. The mixture was reacted at 90 °C for 4 h under nitrogen atmosphere. After the reaction was completed, water (10 mL) was added for dilution, and ethyl acetate (10 mL × 2) was added for extraction. The organic phases were combined, washed with saturated brine (20 mL), dried over anhydrous sodium sulfate and concentrated, and the residue was separated by silica gel column chromatography (PE/EA = 5/1) to give a white solid (540 mg, 76% yield). LC-MS [M+H]⁺: 438.9.

In this step, for the preparation of 4-(1-((2-(trimethylsilyl)ethoxy)methyl)-1H pyrazol-4-yl)aniline (MOOS), reference was made to the preparation method of L038-1 described in Example 38 below.

### 34.2 Preparation of compound 2-(methylsulfonyl)-4-((4-(1-((2-(trimethylsilyl)ethoxy)methyl)-1H-pyrazol-4-yl)phenyl)amino)pyrimidine-5-carbonitrile (L034-2)

2-(methylthio)-4-((4-(1-((2-(trimethylsilyl)ethoxy)methyl)-1*H*-pyrazol-4-yl)phenyl)amino)pyrimidine-5-carbonitrile (200 mg, 0.46 mmol) was dissolved in anhydrous dichloromethane (5 mL), and *m-*chloroperoxybenzoic acid (280 mg, 1.38 mmol) was added. The mixture was reacted at room temperature for 2 h under nitrogen atmosphere. After the reaction was completed, saturated sodium bicarbonate solution (10 mL) was added to quench the reaction and allow the pH of the aqueous phase to be about 10, and dichloromethane (10 mL × 2) was added for extraction. The organic phases were combined, washed with saturated brine (20 mL), dried over anhydrous sodium sulfate and concentrated, and the residue was separated by silica gel column chromatography (PE/EA = 3/1) to give a white solid (110 mg, 51% yield). LC-MS [M+H]⁺: 471.2.

### 34.3 Preparation of compound 2-(2-(3,3-difluoroazetidin-1-carbonyl)-1-methyl-4,5-dihydro-1H pyrrolo[2,3-c]pyridin-6(7H)-yl)-4-((4-(1-((2-(trimethylsilyl)ethoxy)methyl)-1H-pyrazol-4-yl)phenyl)amino)pyrimidine-5-carbonitrile (L034-3)

2-(methylsulfonyl)-4-((4-(1-((2-(trimethylsilyl)ethoxy)methyl)-1*H*-pyrazol-4-yl)phenyl)amino)pyrimidine-5-carbonitrile (110 mg, 0.23 mmol) was dissolved in n-butanol (3 mL), and (3,3-difluoroazetidin-1-yl)(1-methyl-4,5,6,7-tetrahydro-1H pyrrolo[2,3-c]pyridin-2-yl)methanone hydrochloride (80 mg, 0.28 mmol) and *N,N-*diisopropylethylamine (89 mg, 0.69 mmol) were added. The mixture was reacted at 120 °C for 4 h under nitrogen atmosphere. After the reaction was completed, water (10 mL) was added for dilution, and ethyl acetate (10 mL × 2) was added for extraction. The organic phases were combined, washed with saturated brine (20 mL), dried over anhydrous sodium sulfate and concentrated, and the residue was separated by silica gel column chromatography (PE/EA = 5/1) to give a yellow solid (100 mg, 65% yield). LC-MS [M+H]⁺: 645.8.

34.4 Preparation of compound 4-((4-(1*H*-pyrazol-4-yl)phenyl)amino)-2-(2-(3,3-difluoroazetidin-1-carbonyl)-1-methyl-4,5-dihydro-1*H*-pyrrolo[2,3-*c*]pyridin-6(7*H*-yl)pyrimidine-5-carbonitrile (L034) 2-(2-(3,3-difluoroazetidin-1-carbonyl)-1-methyl-4,5-dihydro-1*H*-pyrrolo[2,3-*c*]pyridin-6(7*H*-yl)-4-((4-(1-((2-(trimethylsilyl)ethoxy)methyl)-1H pyrazol-4-yl)phenyl)amino)pyrimidine-5-carbonitrile (100 mg, 0.15 mmol) was dissolved in dichloromethane (3 mL), and a solution of hydrochloric acid in 1,4-dioxane (1 mL) was added. The mixture was reacted at room temperature for 2 h under nitrogen atmosphere. After the reaction was completed, water (5 mL) was added for dilution, and dichloromethane (5 mL × 2) was added for extraction. The aqueous phase was adjusted to pH of about 10 with sodium hydroxide (2 N) and extracted with dichloromethane (5 mL × 2). The organic phases were combined, washed with saturated brine (20 mL), dried over anhydrous sodium sulfate and concentrated, and the residue was subjected to preparative chromatography to give a white solid (43 mg, 55% yield). LC-MS [M+H]⁺: 515.8.

¹H NMR (300 MHz, DMSO) δ 9.01 (s, 1H), 8.38 (s, 1H), 7.92 (s, 2H), 7.60 - 7.49 (m, 4H), 6.39 (s, 1H), 4.79 (s, 2H), 4.50 (t, J = 12.5 Hz, 4H), 3.99 (t, J = 5.8 Hz, 2H), 3.69 (s, 3H), 2.56 (t, J = 5.6 Hz, 2H).

### Example 35. Preparation of Compound (6-(4-((4-(1H-pyrazol-4-yl)phenyl)amino)-5-(trifluoromethyl)pyrimidin-2-yl)-1-methyl-4,5,6,7-tetrahydro-1H pyrrolo[2,3-c]pyridin-2-yl)(3,3-difluoroazetidin-1-yl)methanone (L035)

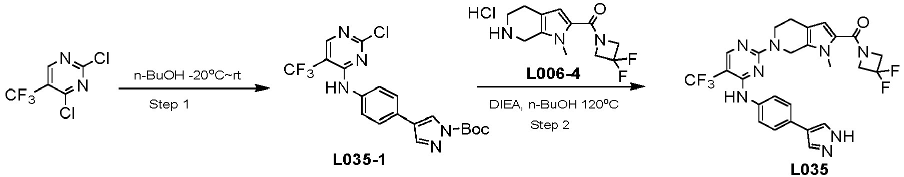

### 35.1 Preparation of compound tert-butyl 4-(4-((2-chloro-5-(trifluoromethyl)pyrimidin-4-yl)amino)phenyl)-1H pyrazole-1-carboxylate (L035-1)

2,4-dichloro-5-(trifluoromethyl)pyrimidine (100 mg, 0.46 mmol) was dissolved in n-butanol (2 mL), and *N,N-*diisopropylethylamine (118 mg, 0.92 mmol) was added. The mixture was cooled to -20 °C under nitrogen atmosphere, and tert-butyl 4-(4-aminophenyl)-1*H*-pyrazole-1-carboxylate (143 mg, 0.55 mmol) was slowly added to the reaction liquid. The resulting mixture was stirred for 1 h, warmed to room temperature and then stirred for 5 h. After the reaction was completed, water (5 mL) was added for dilution, and ethyl acetate (5 mL × 2) was added for extraction. The organic phases were combined, washed with saturated brine (10 mL), dried over anhydrous sodium sulfate and concentrated, and the residue was separated by silica gel column chromatography (PE/EA = 8/1) to give a white solid (120 mg, containing isomers). LC-MS [M+H]⁺: 439.8.

### 35.2 Preparation of compound (6-(4-((4-(1H pyrazol-4-yl)phenyl)amino)-5-(trifluoromethyl)pyrimidin-2-yl)-1-methyl-4,5,6,7-tetrahydro-1H pyrrolo[2,3-c]pyridin-2-yl)(3,3-difluoroazetidin-1-yl)methanone (L035)

*Tert*-butyl 4-(4-((2-chloro-5-(trifluoromethyl)pyrimidin-4-yl)amino)phenyl)-1H pyrazole-1-carboxylate (containing isomers) (120 mg, 0.27 mmol) was dissolved in n-butanol (2 mL), and *N*,*N-*diisopropylethylamine (106 mg, 0.82 mmol) and (3,3-difluoroazetidin-1-yl)(1-methyl-4,5,6,7-tetrahydro-1H pyrrolo[2,3-c]pyridin-2-yl)methanone carbonate (94 mg, 0.33 mmol) were added. The mixture was warmed to 120 °C and reacted for 4 h under nitrogen atmosphere. After the reaction was completed, water (5 mL) was added for dilution, and ethyl acetate (5 mL × 2) was added for extraction. The organic phases were combined, washed with saturated brine (10 mL), dried over anhydrous sodium sulfate and concentrated, and the residue was subjected to preparative chromatography to give a white solid (30.2 mg, 20% two-step yield). LC-MS [M+H]⁺: 558.9. ¹H NMR (400 MHz, DMSO) δ 12.93 (s, 1H), 8.61 (d, J = 29.9 Hz, 1H), 8.32 (s, 1H), 8.20 (s, 1H), 7.93 (s, 1H), 7.61 (d, J = 8.6 Hz, 2H), 7.51 (s, 2H), 6.45 (s, 1H), 4.90-4.65 (m, 2H), 4.60-4.42 (m, 4H), 4.12-3.80 (m, 2H), 3.76 - 3.50 (m, 3H), 2.56-2.52 (m, 2H).

### Example 36. Preparation of Compound (6-(4-((4-(1H-pyrazol-4-yl)phenyl)amino)-1,3,5-triazin-2-yl)-1-methyl-4,5,6,7-tetrahydro-1H-pyrrolo(2,3-c)pyridin-2-yl)(3,3-difluoroazetidin-1-yl)methanone (L036)

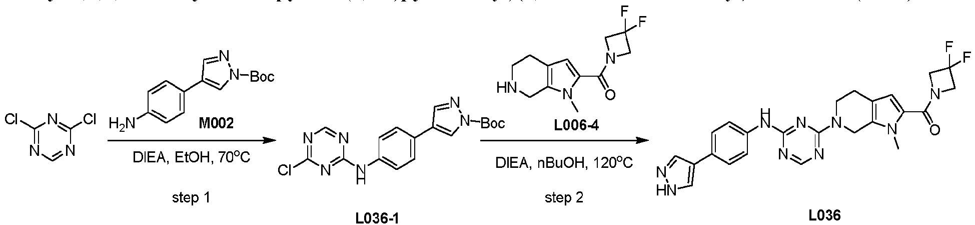

### 36.1 Preparation of compound tert-butyl 4-(4-(((4-chloro-1,3,5-triazin-2-yl)amino)phenyl)-1H-pyrazole-1-carboxylate (L036-1):

2,4-dichloro-1,3,5-triazine (120 mg, 0.80 mmol) and *tert*-butyl 4-(4-aminophenyl)-1*H*-pyrazole-1-carboxylate (219 mg, 0.84 mmol) were added to ethanol (3 mL), and diisopropylethylamine (517 mg, 4.0 mmol) was added. The mixture was stirred at 70 °C for 5 h. After the reaction was completed as detected by liquid chromatography, suction filtration was carried out to give *tert*-butyl 4-(4-(((4-chloro-1,3,5-triazin-2-yl)amino)phenyl)-1*H*-pyrazole-1-carboxylate in the form of a white solid (100 mg, 31.43% yield). MS(M+H)⁺ = 372.7.

### 36.2 Preparation of compound (6-(4-((4-(1H-pyrazol-4-yl)phenyl)amino)-1,3,5-triazin-2-yl)-1-methyl-4,5,6,7-tetrahydro-1H pyrrolo(2,3-c)pyridin-2-yl)(3,3-difluoroazetidin-1-yl)methanone (L036):

*Tert*-butyl 4-(4-(((4-chloro-1,3,5-triazin-2-yl)amino)phenyl)-1*H*-pyrazole-1-carboxylate (50 mg, 0.13 mmol) and 3,3-difluoro-1-((1-methyl-4*H*,5*H*,6*H*,7*H*-pyrrolo(2,3-*c*)pyridin-2-yl)carbonyl)azetidine (34 mg, 0.13 mmol) were added to n-butanol (2 mL), and diisopropylethylamine (86 mg, 0.7 mmol) was added. The mixture was stirred at 120 °C for 5 h. After the reaction was completed as detected by liquid chromatography, the mixture was subjected to preparative chromatography to give (6-(4-((4-(1*H*-pyrazol-4-yl)phenyl)amino)-1,3,5-triazin-2-yl)-1-methyl-4,5,6,7-tetrahydro-1H pyrrolo(2,3-c)pyridin-2-yl)(3,3-difluoroazetidin-1-yl)methanone in the form of a white solid (24.2 mg, 36.55% yield). MS(M+H)⁺ = 492.2.

¹H NMR (400 MHz, DMSO) δ 12.89 (s, 1H), 9.74 (s, 1H), 8.30 (s, 1H), 8.13 (s, 1H), 7.88 (s, 1H), 7.76 - 7.67 (m, J = 8.6 Hz, 2H), 7.61 - 7.51 (m, 2H), 6.49 (s, 1H), 4.86 (s, 2H), 4.57 (s, 4H), 4.03 (t, J = 5.5 Hz, 2H), 3.82 - 3.71 (m, J = 14.8 Hz, 3H), 2.63 - 2.56 (m, 2H).

### Example 37. Preparation of Compound (6-(5-((4-(1H-pyrazol-4-yl)phenyl)amino)-1,2,4-triazin-3-yl)-1-methyl-4,5,6,7-tetrahydro-1Hpyrrolo(2,3-c)pyridin-2-yl)(3,3-difluoroazetidin-1-yl)methanone (L037)

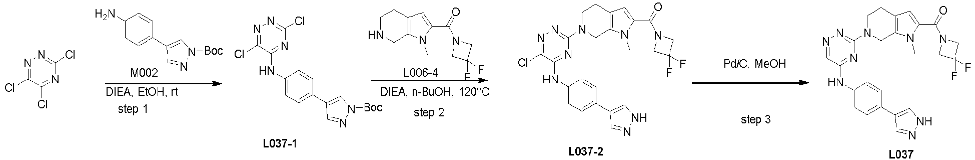

### 37.1 Preparation of compound tert-butyl 4-(4-(((3,6-dichloro-1,2,4-triazin-5-yl)amino)phenyl)-1H-pyrazole-1-carboxylate (L037-1):

3,5,6-trichloro-1,2,4-triazine (205 mg, 1.4 mmol) and tert-butyl 4-(4-aminophenyl)-1*H*-pyrazole-1-carboxylate (353 mg, 1.4 mmol) were added to ethanol (5 mL), and diisopropylethylamine (350 mg, 2.7 mmol) was added. The mixture was stirred at room temperature for 1 h. After the reaction was completed as detected by liquid chromatography, the mixture was concentrated to give tert-butyl 4-(4-(((3,6-dichloro-1,2,4-triazin-5-yl)amino)phenyl)-1*H*-pyrazole-1-carboxylate (600 mg, crude product). MS(M+H)⁺ = 406.8.

37.2 Preparation of compound (6-(5-((4-(1*H*-pyrazol-4-yl)phenyl)amino)-6-chloro-1,2,4-triazin-3-yl)-1-methyl-4,5,6,7-tetrahydro-1H pyrrolo(2,3-c)pyridin-2-yl)(3,3-difluoroazetidin-1-yl)methanone (L037-2): *Tert*-butyl 4-(4-(((3,6-dichloro-1,2,4-triazin-5-yl)amino)phenyl)-1*H*-pyrazole-1-carboxylate (400 mg, 0.98 mmol) and 3,3-difluoro-1-((1-methyl-4H,SH,6H,7H pyrrolo(2,3-c)pyridin-2-yl)carbonyl)azetidine (286 mg, 0.98 mmol) were added to n-butanol (10 mL), and diisopropylethylamine (633 mg, 4.9 mmol) was added. The mixture was stirred at 120 °C for 2 h. After the reaction was completed as detected by liquid chromatography, a part of the mixture was subjected to preparative chromatography to give (6-(5-((4-(1*H*-pyrazol-4-yl)phenyl)amino)-6-chloro-1,2,4-triazin-3-yl)-1-methyl-4,5,6,7-tetrahydro-1*H*-pyrrolo(2,3-c)pyridin-2-yl)(3,3-difluoroazetidin-1-yl)methanone in the form of a white solid (127.7 mg). MS(M+H)⁺ = 526.0.

### 37.3 Preparation of compound (6-(5-((4-(1H-pyrazol-4-yl)phenyl)amino)-1,2,4-triazin-3-yl)-1-methyl-4,5,6,7-tetrahydro-1H pyrrolo(2,3-c)pyridin-2-yl)(3,3-difluoroazetidin-1-yl)methanone (L037):

(6-(5-((4-(1*H-*pyrazol-4-yl)phenyl)amino)-6-chloro-1,2,4-triazin-3-yl)-1-methyl-4,5,6,7-tetrahydro-1*H-*pyrrolo(2,3-c)pyridin-2-yl)(3,3-difluoroazetidin-1-yl)methanone (the product of the above step) was dissolved in methanol (10 mL), and palladium on carbon (50 mg) was added. The mixture was stirred homogeneously, and then triethylamine (1 mL) was added. A hydrogen balloon was provided, and the reaction system was purged with hydrogen and stirred at room temperature for 10 h. After the reaction was completed as detected, suction filtration was carried out, and the filtrate was concentrated. The residue was subjected to preparative chromatography to give (6-(5-((4-(1*H*-pyrazol-4-yl)phenyl)amino)-1,2,4-triazin-3-yl)-1-methyl-4,5,6,7-tetrahydro-1H pyrrolo(2,3-c)pyridin-2-yl)(3,3-difluoroazetidin-1-yl)methanone in the form of a white solid (36.6 mg). MS(M+H)⁺ = 491.8.

¹HNMR (400 MHz, ) δ 12.88 (s, 1H), 9.96 (s, 1H), 8.22 - 7.87 (m, 3H), 7.71 (d, J = 8.6 Hz, 2H), 7.64 (d, J = 8.7 Hz, 2H), 6.48 (s, 1H), 4.86 (s, 2H), 4.57 (s, 4H), 4.01 (s, 2H), 3.78 (s, 3H), 2.60 (t, J = 5.4 Hz, 2H).

### Example 38. Preparation of Compound (6-(5-chloro-4-((4-(3-fluoro-1H-pyrazol-4-yl)phenyl)amino)pyrimidin-2-yl)-1-methyl-4,5,6,7-tetrahydro-1H pyrrolo[2,3-c]pyridin-2-yl)(3,3-difluoroazetidin-1-yl)methanone (L038)

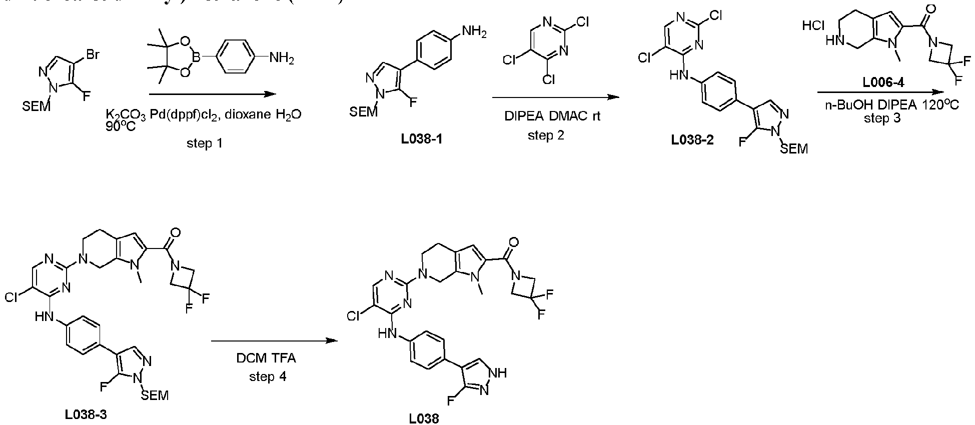

### 38.1 Preparation of compound 4-(5-fluoro-1-((2-(trimethylsilyl)ethoxy)methyl)-1H-pyrazol-4-yl)aniline (L038-1):

4-bromo-5-fluoro-1-((2-(trimethylsilyl)ethoxy)methyl)-1*H*-pyrazole (500 mg, 1.70 mmol) was dissolved in dioxane (5 mL) and water (1 mL), and 4-(4,4,5,5-tetramethyl-1,3,2-dioxaborolan-2-yl)aniline (446 mg, 2.04 mmol), potassium carbonate (73 mg, 0.38 mmol) and[1,1'-bis(diphenylphosphino)ferrocene]dichloropalladium(II) (124 mg, 0.17 mmol) were added. The mixture was warmed to 90 °C and stirred for 4 h under nitrogen atmosphere, and then filtered. Water (10 mL) was added for dilution, and ethyl acetate (10 mL × 2) was added for extraction. The organic phases were combined, washed with saturated brine (20 mL), dried over anhydrous sodium sulfate and concentrated, and the residue was separated by silica gel column chromatography (PE/EA = 5/1) to give a white solid (300 mg, 57% yield).

LC-MS [M+H]⁺: 307.8.

### 38.2 Preparation of compound 2,5-dichloro-N-(4-(5-fluoro-1-((2-(trimethylsilyl)ethoxy)methyl)-1H-pyrazol-4-yl)phenyl)pyrimidin-4-amine (L038-2):

4-(5-fluoro-1-((2-(trimethylsilyl)ethoxy)methyl)-1H pyrazol-4-yl)aniline (70 mg, 0.23 mmol) was dissolved in *N*,*N*-dimethylformamide (3 mL), and 2,4,5-trichloropyrimidine (63 mg, 0.34 mmol) and *N,N-*diisopropylethylamine (89 mg, 0.69 mmol) were added. The mixture was reacted at room temperature for 5 h under nitrogen atmosphere. Water (10 mL) was added for dilution, and ethyl acetate (10 mL × 2) was added for extraction. The organic phases were combined, washed with saturated brine (20 mL), dried over anhydrous sodium sulfate and concentrated, and the residue was separated by silica gel column chromatography (PE/EA = 4/1) to give a yellow solid (90 mg, 86% yield). LC-MS [M+H]⁺: 453.8.

### 38.3 Preparation of compound (6-(5-chloro-4-((4-(5-fluoro-1-((2-(trimethylsilyl)ethoxy)methyl)-1H-pyrazol-4-yl)phenyl)amino)pyrimidin-2-yl)-1-methyl-4,5,6,7-tetrahydro-1H-pyrrolo[2,3-c]pyridin-2-yl)(3,3-difluoroazetidin-1-yl)methanone (L038-3):

2,5-dichloro-*N*-(4-(5-fluoro-1-((2-(trimethylsilyl)ethoxy)methyl)-1*H*-pyrazol-4-yl)phenyl)pyrimidin-4-amine (90 mg, 0.19 mmol) was dissolved in n-butanol (2 mL), and *N*,*N*-diisopropylethylamine (73 mg, 0.57 mmol) and (3,3-difluoroazetidin-1-yl)(1-methyl-4,5,6,7-tetrahydro-1*H*-pyrrolo[2,3-*c*]pyridin-2-yl)methanone hydrochloride (66 mg, 0.23 mmol) were added. The mixture was warmed to 120 °C and reacted for 4 h under nitrogen atmosphere. After the reaction was completed, water (5 mL) was added for dilution, and ethyl acetate (5 mL × 2) was added for extraction. The organic phases were combined, washed with saturated brine (10 mL), dried over anhydrous sodium sulfate and concentrated, and the residue was separated by silica gel column chromatography (PE/EA = 2/1) to give a white solid (110 mg, 86% yield). LC-MS [M+H]⁺: 672.8.

### 38.4 Preparation of compound (6-(5-chloro-4-((4-(3-fluoro-1H-pyrazol-4-yl)phenyl)amino)pyrimidin-2-yl)-1-methyl-4,5,6,7-tetrahydro-1H-pyrrolo[2,3-c]pyridin-2-yl)(3,3-difluoroazetidin-1-yl)methanone (L038):

(6-(5-chloro-4-((4-(5-fluoro-1-((2-(trimethylsilyl)ethoxy)methyl)-1*H*-pyrazol-4-yl)phenyl)amino)pyrimidin-2-yl)-1-methyl-4,5,6,7-tetrahydro-1H pyrrolo[2,3-c]pyridin-2-yl)(3,3-difluoroazetidin-1-yl)methanone (110 mg, 0.16 mmol) was dissolved in anhydrous dichloromethane (3 mL), and trifluoroacetic acid (1 mL) was added to the reaction liquid. The mixture was reacted at room temperature for 2 h under nitrogen atmosphere. After the reaction was completed, water (5 mL) was added for dilution, and dichloromethane (5 mL × 2) was added for extraction. The aqueous phase was adjusted to pH of about 10 with aqueous sodium hydroxide solution (2 N) and extracted with dichloromethane (5 mL × 2). The organic phases were combined, washed with saturated brine (10 mL), dried over anhydrous sodium sulfate and concentrated, and the residue was subjected to preparative chromatography to give a white solid (45.3 mg, 52% yield). LC-MS [M+H]⁺: 542.8.

¹H NMR (400 MHz, DMSO) δ 12.65 (s, 1H), 8.86 (s, 1H), 8.15 (d, J = 2.0 Hz, 1H), 8.10 (s, 1H), 7.71 (d, J = 8.6 Hz, 2H), 7.55 (d, J = 8.5 Hz, 2H), 6.45 (s, 1H), 4.72 (s, 2H), 4.68-4.48 (s, 4H), 3.98-3.90 (m, 2H), 3.71 (s, 3H), 2.55-2.53 (m, 2H).

### Example 39. Preparation of Compound (3-(difluoromethyl)azetidin-1-yl)(6-(5-fluoro-4-((4-(3-fluoro-1H-pyrazol-4-yl)phenyl)amino)pyrimidin-2-yl)-1-methyl-4,5,6,7-tetrahydro-1H pyrrolo[2,3-c]pyridin-2-yl)methanone (L039)

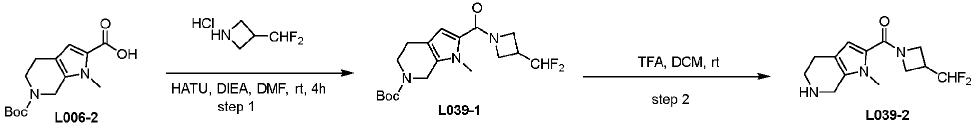

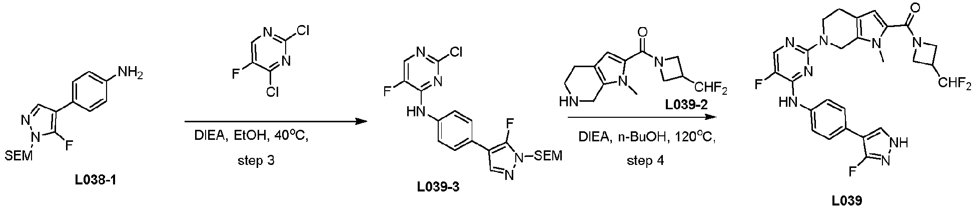

### 39.1 Preparation of compound tert-butyl 2-(3-(difluoromethyl)azetidin-1-carbonyl)-1-methyl-4,5-dihydro-1H-pyrrolo[2,3-c]pyridine-6(7H)-carboxylate (L039-1):

6-(*tert*-butoxycarbonyl)-1-methyl-4,5,6,7-tetrahydro-1*H*-pyrrolo[2,3-*c*]pyridine-2-carboxylic acid (300 mg, 1.07 mmol) was dissolved in anhydrous *N*,*N*-dimethylformamide (5 mL), and 3-(difluoromethyl)azetidine hydrochloride (183 mg, 1.28 mmol), 2-(7-azabenzotriazol)-*N*,*N*,*N'*,*N'*-tetramethyluronium hexafluorophosphate (487 mg, 1.28 mmol) and *N*,*N-*diisopropylethylamine (414 mg, 3.21 mmol) were added. The mixture was stirred at room temperature for 4 h under nitrogen atmosphere. Water (10 mL) was added for dilution, and ethyl acetate (10 mL × 2) was added for extraction. The organic phases were combined, dried over anhydrous sodium sulfate and concentrated, and the residue was separated by silica gel column chromatography (PE/EA = 3/1) to give a colorless oily liquid (280 mg, 71% yield). LC-MS [M+H]⁺: 369.9.

### 39.2 Preparation of compound (3-(difluoromethyl)azetidin-1-yl)(1-methyl-4,5,6,7-tetrahydro-1H-pyrrolo[2,3-c]pyridin-2-yl)methanone (L039-2):

*Tert-butyl* 2-(3-(difluoromethyl)azetidin-1-carbonyl)-1-methyl-4,5-dihydro-1*H*-pyrrolo[2,3-*c*]pyridine-6(7*H*)-carboxylate (280 mg, 0.75 mmol) was dissolved in anhydrous dichloromethane (6 mL), and trifluoroacetic acid (2 mL) was added. The mixture was reacted at room temperature for 2 h under nitrogen atmosphere. After the reaction was completed, water (10 mL) was added for dilution, and dichloromethane (10 mL × 2) was added for extraction. The aqueous phase was adjusted to pH of about 10 with aqueous sodium hydroxide solution (2 N) and extracted with dichloromethane (10 mL × 2). The organic phases were combined and concentrated to give a yellow solid (160 mg, crude product). LC-MS [M+H]⁺: 269.8.

### 39.3 Preparation of compound 2-chloro-5-fluoro-N (4-(5-fluoro-1-((2-(trimethylsilyl)ethoxy)methyl)-1H pyrazol-4-yl)phenyl)pyrimidin-4-amine (L039-3):

4-(5-fluoro-1-((2-(trimethylsilyl)ethoxy)methyl)-1H pyrazol-4-yl)aniline (80 mg, 0.26 mmol) was dissolved in absolute ethanol (3 mL), and *N,N*-diisopropylethylamine (100 mg, 0.78 mmol) and 2,4-dichloro-5-fluoropyrimidine (86 mg, 0.52 mmol) were added. The mixture was warmed to 40 °C and reacted for 2 h under nitrogen atmosphere. After the reaction was completed, water (10 mL) was added for dilution, and ethyl acetate (10 mL × 2) was added for extraction. The organic phases were combined, washed with saturated brine (20 mL), dried over anhydrous sodium sulfate and concentrated, and the residue was separated by silica gel column chromatography (PE/EA = 3/1) to give a white solid (100 mg, 88% yield). LC-MS [M+H]⁺: 438.2.

### 39.4 Preparation of compound (3-(difluoromethyl)azetidin-1-yl)(6-(5-fluoro-4-((4-(3-fluoro-1H pyrazol-4-yl)phenyl)amino)pyrimidin-2-yl)-1-methyl-4,5,6,7-tetrahydro-1H pyrrolo[2,3-c]pyridin-2-yl)methanone (L039):

2-chloro-5-fluoro-*N*-(4-(5-fluoro-1-((2-(trimethylsilyl)ethoxy)methyl)-1*H*-pyrazol-4-yl)phenyl)pyrimidin-4-amine (100 mg, 0.22 mmol) was dissolved in n-butanol (3 mL), and *N,N*-diisopropylethylamine (85 mg, 0.66 mmol) and (3-(difluoromethyl)azetidin-1-yl)(1-methyl-4,5,6,7-tetrahydro-1H pyrrolo[2,3-c]pyridin-2-yl)methanone (71 mg, 0.26 mmol) were added to the reaction liquid. The mixture was warmed to 120 °C and reacted for 12 h under nitrogen atmosphere. After the reaction was completed, water (10 mL) was added for dilution, and ethyl acetate (10 mL × 2) was added for extraction. The organic phases were combined, washed with saturated brine (20 mL), dried over anhydrous sodium sulfate and concentrated, and the residue was subjected to preparative chromatography to give a white solid (10.9 mg, 10% yield). LC-MS [M+H]⁺: 541.2.

NMR (400 MHz, ) DMSO δ 12.51 (s, 1H), 9.36 (s, 1H), 8.10 (t, J = 2.0 Hz, 1H), 8.03 (d, J = 3.7 Hz, 1H), 7.75 (d, J = 1.9 Hz, 2H), 7.50 (d, J = 8.6 Hz, 2H), 6.44 (d, J = 4.4 Hz, 0.25H), 6.33 (s, 1H), 6.29 (d, J = 4.3 Hz, 0.5H), 6.15 (d, J = 4.4 Hz, 0.25H), 4.68 (s, 2H), 4.35 - 4.00 (m, 4H), 3.87 (t, J = 5.6 Hz, 2H), 3.69 (s, 3H), 3.15 - 3.04 (m, 1H), 2.53 - 2.46 (m, 2H).

### Example 40. Preparation of Compound (6-(5-fluoro-4-((2-fluoro-4-(1H-pyrazol-4-yl)phenyl)amino)pyrimidin-2-yl)-1-methyl-4,5,6,7-tetrahydro-1H-pyrrolo[2,3-c]pyridin-2-yl)(3-fluoroazetidin-1-yl)methanone (L040)

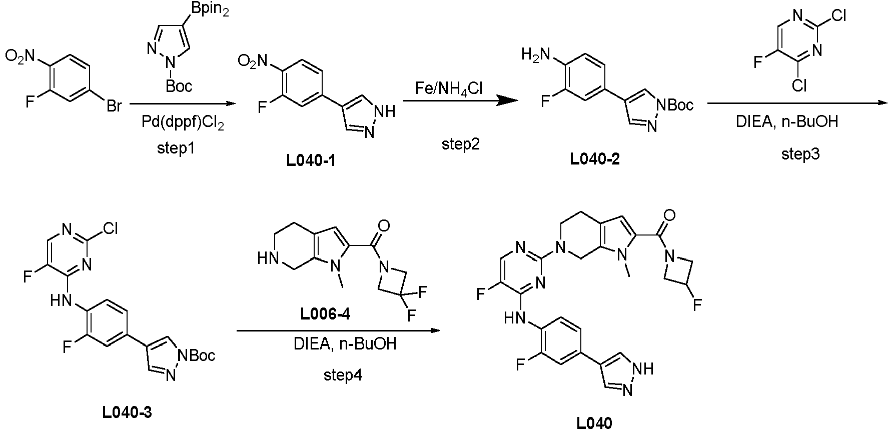

### 40.1 Preparation of compound 4-(3-fluoro-4-nitrophenyl)-1H-pyrazole (L040-1):

4-bromo-2-fluoro-1-nitrobenzene (4.0 g, 18.18 mmol), tert-butyl (4-(4,4,5-trimethyl-1,3,2-dioxaborolan-2-yl)pyrazol-1-yl)carboxylate (8.02 g, 27.27 mmol), Pd(dppf)Cl₂ (1.99 g, 2.732 mmol) and K₂CO₃ (7.54 g, 54.55 mol) were each weighed out and added to 1,4-dioxane (90 mL) and water (9 mL). The reaction system was purged with nitrogen, heated to 100 °C and reacted for 3 h. After the reaction was completed as detected by TLC, the reaction liquid was cooled, filtered and concentrated by rotary evaporation to remove the solvent, and the residue was purified by silica gel column chromatography (PE/EA = 20/1-3/1) to give a yellow solid powder (2.3 g).

### 40.2 Preparation of compound tert-butyl 4-(4-amino-3-fluorophenyl)-1H pyrazole-1-carboxylate (L040-2):

L040-1 (500 mg, 2.41 mmol) and Pd/C (150 mg) were each weighed out and added to methanol (5 mL). The reaction system was purged with hydrogen, heated to 40 °C and reacted for 2 h. After the reaction was completed as detected by LCMS, the mixture was filtered and concentrated by rotary evaporation to remove the solvent, thus giving a yellow oily liquid (320 mg). LC-MS: [M+H]⁺ = 278.30.

### 40.3 Preparation of compound tert-butyl 4-(4-((2-chloro-5-fluoropyrimidin-4-yl)amino)-3-fluorophenyl)-1H pyrazole-1-carboxylate (L040-3):

2,4-dichloro-5-fluoropyrimidine (390 mg, 2.37 mmol) and L040-2 (540 mg, 1.95 mmol) were weighed out and placed in a 50 mL eggplant-shaped bottle, and then n-butanol was added for dissolution, followed by addition of DIEA (1.01 g, 7.8 mmol). The mixture was heated to 120 °C and reacted for 3 h. After the reaction was completed as detected by LCMS, the reaction liquid was concentrated under reduced pressure, and the residue was purified by silica gel column chromatography (PE/EA = 20/1-5/1) and further purified by thin layer chromatography (pure DCM) to give a yellow solid (70 mg). LC-MS: [M+H]⁺ = 308.04.

### 40.4 Preparation of compound (6-(5-fluoro-4-((2-fluoro-4-(1H-pyrazol-4-yl)phenyl)amino)pyrimidin-2-yl)-1-methyl-4,5,6,7-tetrahydro-1H-pyrrolo[2,3-c]pyridin-2-yl)(3-fluoroazetidin-1-yl)methanone (L040):

L040-3 (70 mg, 0.23 mmol) and (3,3-difluoroazetidin-1-yl)(1-methyl-4,5,6,7-tetrahydro-1*H*-pyrrolo[2,3-c]pyridin-2-yl)methanone (70 mg, 0.27 mmol) were weighed out and placed in a 35 mL sealed tube, and *n-*butanol (2 mL) was added for dissolution, followed by addition of DIEA (119 mg, 0.92 mmol). The mixture was reacted overnight at 120 °C. After the reaction was completed as detected by LCMS, the mixture was concentrated under reduced pressure to give a crude product. The crude product was separated by prep-HPLC using H₂O/ACN system and lyophilized to give a white solid (7.2 mg, 95.736% purity). LC-MS: [M+H]⁺ = 527.10.

¹H-NMR (400 MHz, dmso) δ 8.97 (d, J = 0.6 Hz, 1H), 8.60 (d, J = 4.2 Hz, 1H), 8.31 (d, J = 0.6 Hz, 1H), 7.52 (dd, J = 12.8, 1.8 Hz, 1H), 7.34 (dd, J = 8.2, 1.9 Hz, 1H), 6.79 (dd, J = 9.5, 8.3 Hz, 1H), 6.48 (s, 1H), 5.25 (s, 2H), 4.83 (s, 2H), 4.54 (t, J = 11.8 Hz, 4H), 4.06 (t, J = 5.3 Hz, 2H), 3.78 (s, 3H), 2.60 (t, J = 5.3 Hz, 2H), 2.05 (s, 1H).

### Example 41. Preparation of Compound (6-(4-((4-(1H-pyrazol-4-yl)phenyl)amino)-6-(trifluoromethyl)pyrimidin-2-yl)-1-methyl-4,5,6,7-tetrahydro-1H pyrrolo[2,3-c]pyridin-2-yl)(3,3-difluoroazetidin-1-yl)methanone (L041)

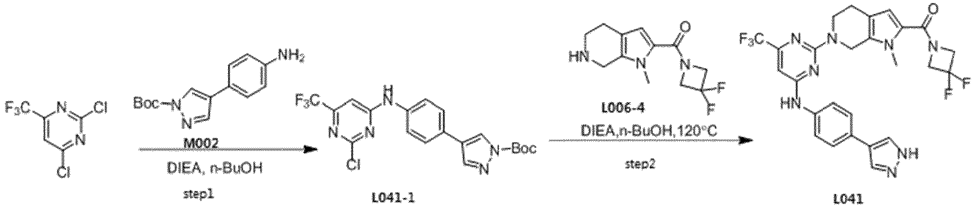

### 41.1 Preparation of compound tert-butyl 4-(4-((2-chloro-6-(trifluoromethyl)pyrimidin-4-yl)amino)phenyl)-1H pyrazole-1-carboxylate (L041-1):

2,4-dichloro-6-trifluoromethylpyrimidine (100 mg, 0.46 mmol) and tert-butyl 4-(4-aminophenyl)-1*H-*pyrazole-1-carboxylate (119.52 mg, 0.46 mmol) were each weighed out and placed in a 5 mL sample bottle, and n-butanol (1 mL) was added for dissolution, followed by addition of DIEA (297.8 mg, 1.15 mmol). The mixture was stirred at 70 °C for 2 h. After the reaction was completed as detected by LCMS, the reaction liquid was concentrated and extracted twice with EA/H₂O. The organic phase was separated out, dried and concentrated to give a light yellow solid (233 mg, crude product). LC-MS: [M+H]⁺ = 440.10.

### 41.2 Preparation of compound (6-(4-((4-(1H-pyrazol-4-yl)phenyl)amino)-6-(trifluoromethyl)pyrimidin-2-yl)-1-methyl-4,5,6,7-tetrahydro-1H pyrrolo[2,3-c]pyridin-2-yl)(3,3-difluoroazetidin-1-yl)methanone (L041):

L041-1 (200 mg, 0.454 mmol) and (3,3-difluoroazetidin-1-yl)(1-methyl-4,5,6,7-tetrahydro-1H pyrrolo[2,3-c]pyridin-2-yl)methanone (116 mg, 0.454 mmol) were each weighed out and placed in a 25 mL eggplant-shaped bottle, and n-butanol (5 mL) was added for dissolution, followed by addition of DIEA (294 mg, 2.27 mmol). The mixture was stirred at 120 °C for 2 h. After the reaction was completed as detected by LCMS, the reaction liquid was concentrated and extracted twice with EA/H₂O. The organic phase was separated out, dried and concentrated to give a crude product, and the crude product was subjected to preparative chromatography and lyophilized to give a white solid (56.1 mg, 96.192% purity). LC-MS: [M+H]⁺ = 559.15.

¹H NMR (600 MHz, DMSO-*d₆*) δ 12.86 (s, 1H), 9.82 (s, 1H), 8.33 - 7.81 (m, 1H), 7.64 (dd, *J=* 28.2, 8.0 Hz, 2H), 6.48 (s, 1H), 6.43 (s, 1H), 4.82 (s, 1H), 4.56 (s, 2H), 4.01 (t, *J=* 5.0 Hz, 1H), 3.77 (s, 2H), 2.59 (s, 1H).

### Example 43. Preparation of Compound (6-(5-((4-(1H-pyrazol-4-yl)phenyl)amino)-6-methoxy-1,2,4-triazin-3-yl)-1-methyl-4,5,6,7-tetrahydro-1H pyrrolo[2,3-c]pyridin-2-yl)(3,3-difluoroazetidin-1-yl)methanone (L043)

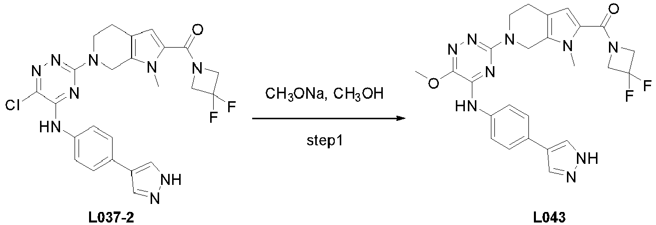

(6-(5-((4-(1*H*-pyrazol-4-yl)phenyl)amino)-6-chloro-1,2,4-triazin-3-yl)-1-methyl-4,5,6,7-tetrahydro-1*H-*pyrrolo(2,3-*c*)pyridin-2-yl)(3,3-difluoroazetidin-1-yl)methanone (300 mg, 0.570 mmol) was weighed out and placed in a 35 mL sealed tube, and methanol (10 mL) was added for dissolution, followed by addition of sodium methoxide (308 mg, 5.703 mmol). The mixture was reacted at 100 °C for 11 h. After the reaction was completed as detected by LCMS, the mixture was concentrated under reduced pressure to give a crude product. The crude product was separated by prep-HPLC using H₂O/ACN system and lyophilized to give a white solid (15.0 mg, 97.816% purity). LC-MS: [M+H]⁺ = 522.20.

¹H NMR (400 MHz, DMSO-d) δ 9.46 (s, 1H), 8.14 (s, 1H), 8.05 (s, 2H), 7.84 (d, J = 8.6 Hz, 2H), 7.63 (dd, J = 8.6,1.7 Hz, 2H), 6.46 (s, 1H), 4.72 (s, 2H), 4.56 (t, J = 11.8 Hz, 4H), 4.01 (s, 3H), 3.89 (t, J = 5.5 Hz, 2H), 3.76 (s, 3H), 2.58 (s, 2H).

### Example 45. Preparation of Compound (6-(4-((4-(1H-pyrazol-4-yl)phenyl)amino)-1,3,5-triazin-2-yl)-1-(difluoromethyl)-4,5,6,7-tetrahydro-1H pyrrolo[2,3-c]pyridin-2-yl)(3,3-difluoroazetidin-1-yl)methanone (L045)

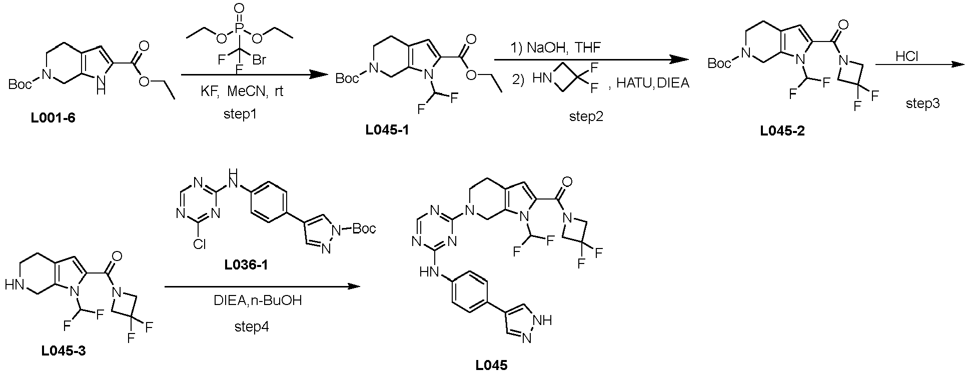

### 45.1 Preparation of compound 6-(tert-butyl) 2-ethyl 1-(difluoromethyl)-1,4,5,7-tetrahydro-6H-pyrrolo[2,3-c]pyridine-2,6-dicarboxylic acid (L045-1):

6-(*tert*-butyl) 2-ethyl-1,4,5,7-tetrahydro-6*H*-pyrrolo[2,3-*c*]pyridine-2,6-dicarboxylic acid (2.0 g, 6.8 mmol) and diethyl bromofluoromethylphosphonate (2.7 g, 10.2 mmol) were weighed out and placed in a 100 mL eggplant-shaped bottle, and acetonitrile (20 mL) was added for dissolution, followed by addition of KF (1.2 g, 20.4 mmol). The mixture was reacted at room temperature for 16 h. After the reaction was completed as detected by TLC, the reaction was stopped, and the mixture was filtered under reduced pressure. The filtrate was concentrated to give a crude product, and the crude product was separated by silica gel column chromatography (PE:EA = 10:1-4:1) to give an off-white solid (800 mg). LC-MS: [2M+H]⁺ = 689.30.

### 45.2 Preparation of compound tert-butyl 2-(3,3-difluoroazetidine-1-carbonyl)-1-(difluoromethyl)-1,4,5,7-tetrahydro-6H-pyrrolo[2,3-c|pyridine-6-carboxylate (L045-2):

L045-1 (720 mg, 2.0 mmol) was weighed out and placed in a 50 mL eggplant-shaped bottle, and THF (20 mL) and water (4 mL) were added for dissolution, followed by addition of NaOH solid (0.4 g, 10 mmol). The mixture was reacted at room temperature for 4 h. After the reaction was completed as detected by LCMS, the pH was adjusted to 5-6 with acetic acid under an ice bath. The solid was filtered out, and the filtrate was concentrated by rotary evaporation to give a product. The product was dissolved by addition of DCM (20 mL), followed by addition of DIEA (1 mL, 6.0 mmol), HATU (1.5 g, 4.0 mmol) and 3,3-difluoroazetidine (389 mg, 3.0 mmol). The mixture was reacted at room temperature for 3 h. After the starting material was completely reacted as detected by LCMS, the reaction was stopped, and the mixture was filtered under reduced pressure. The filtrate was concentrated to give a crude product, and the crude product was separated by silica gel column chromatography (PE:EA = 4:1) to give a light yellow solid (900 mg).

LC-MS: [M+H-56]⁺ = 336.05.

### 45.3 Preparation of compound (3,3-difluoroazetidin-1-yl)(1-(difluoromethyl)-4,5,6,7-tetrahydro-1H-pyrrolo[2,3-c]pyridin-2-yl)methanone (L045-3):

L045-2 (900 mg, 2.3 mmol) was weighed out and placed in a 50 mL eggplant-shaped bottle, and methanol (20 mL) was added for dissolution, followed by addition of a solution of HCl in 1,4-dioxane(20 mL, 4 M). The mixture was reacted at room temperature for 5 h. After the reaction was completed as detected by LCMS, the mixture was concentrated by rotary evaporation to remove the solvent, and HCl was removed by using DCM, thus giving the hydrochloride of L045-3 in the form of a yellow solid (700 mg). LC-MS: [M+H]⁺ = 292.05.

### 45.4 Preparation of compound (6-(4-((4-(1H-pyrazol-4-yl)phenyl)amino)-1,3,5-triazin-2-yl)-1-(difluoromethyl)-4,5,6,7-tetrahydro-1H pyrrolo[2,3-c]pyridin-2-yl)(3,3-difluoroazetidin-1-yl)methanone (L045):

Tert-butyl 4-(4-(((4-chloro-1,3,5-triazin-2-yl)amino)phenyl)-1*H* pyrazole-1-carboxylate (100 mg, 0.268 mmol) and L045-03 (78 mg, 0.268 mmol) were each weighed out and placed in a 25 mL eggplant-shaped bottle, and n-butanol (2 mL) was added for dissolution, followed by addition of DIEA (173 mg, 1.34 mmol). The mixture was stirred at 120 °C for 2 h. After the reaction was completed as detected by LCMS, the reaction liquid was concentrated and extracted twice with EA/H₂O. The organic phase was separated out, dried and concentrated to give a crude product, and the crude product was subjected to preparative chromatography and lyophilized to give a white solid (70.4 mg, 98.524% purity). LC-MS: [M+H]⁺ = 528.15.

¹H NMR (400 MHz, DMSO-d6) δ 12.84 (s, 1H), 9.74 (s, 1H), 8.40 - 7.74 (m, 2H), 7.68 (s, 1H), 7.52 (dd, J = 31.2, 7.6 Hz, 1H), 6.72 (s, 1H), 5.06 (s, 1H), 4.62 (s, 2H), 4.06 (t, J = 5.6 Hz, 1H), 2.60 (s, 1H).

### Example 46. Preparation of Compound (6-(5-((4-(1H-pyrazol-4-yl)phenyl)amino)-1,3,4-oxadiazol-2-yl)-1-methyl-4,5,6,7-tetrahydro-1Hpyrrolo[2,3-c]pyridin-2-yl)(3,3-difluoroazetidin-1-yl)methanone (L046)

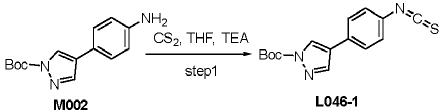

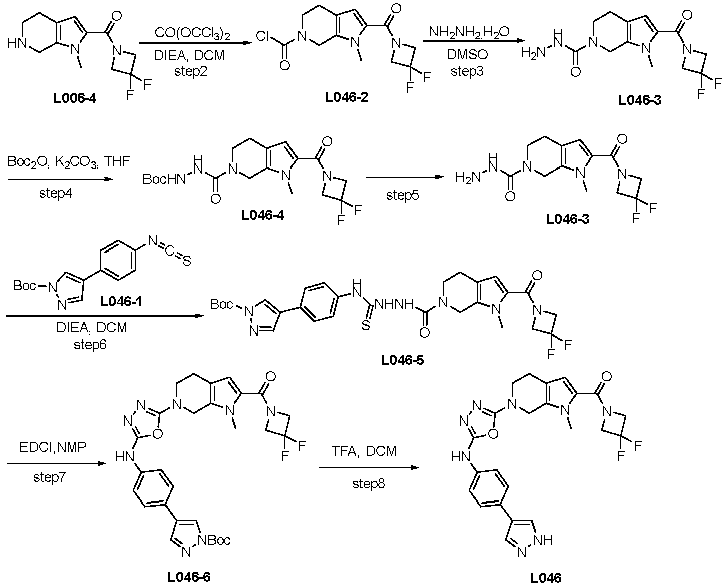

### 46.1 Preparation of compound tert-butyl 4-(4-isothiocyanatophenyl)-1H-pyrazole-1-carboxylate (L046-1)

*Tert*-butyl 4-(4-aminophenyl)-1*H-*pyrazole-1-carboxylate (M002) (520 mg, 2.005 mmol) was weighed out, and tetrahydrofuran (10 mL) was added for dissolution, followed by addition of CS₂ (1.522 g, 20.050 mmol) and NaH (200 mg, 5.013 mmol).The mixture was reacted overnight at 50 °C. After the starting material was completely reacted as detected by LCMS, BoczO (437 mg, 2.005 mmol) and DMAP (25 mg, 0.205 mmol) were added to the reaction system. The mixture was reacted at room temperature for 5 h. After the reaction was completed as detected by LCMS, an appropriate amount of water was added for dilution, and EA (50 mL × 3) was added for extraction. The organic phases were combined, dried over anhydrous sodium sulfate and concentrated under reduced pressure to give a crude product, and the crude product was separated by silica gel column chromatography (PE:EA = 8:1) to give *tert*-butyl 4-(4-isothiocyanatophenyl)-1*H*-pyrazole-1-carboxylate in the form of a light yellow solid (650 mg). LC-MS: [M+H]⁺ = 302.05.

### 46.2 Preparation of compound 2-(3,3-difluoroazetidin-1-carhonyl)-1-methyl-1,4,5,7-tetrahydro-6H-pyrrolo[2,3-c]pyridine-6-carbonyl chloride (L046-2)

(3,3-difluoroazetidin-1-yl)(1-methyl-4,5,6,7-tetrahydro-1H pyrrolo[2,3-c]pyridin-2-yl)methanone (500 mg, 1.961 mmol) and DIEA (2.529 g, 19.610 mmol) were weighed out and placed in a 50 mL eggplant-shaped bottle, and DCM (10 mL) was added for dissolution. The mixture was stirred at room temperature for 10 min. A solution of solid triphosgene (1.741 g, 5.883 mmol) in DCM was added dropwise under an ice bath, and after the dropwise addition was completed, the mixture was reacted at room temperature for 5 h. After the reaction was completed as detected by LCMS, water was added for dilution, and DCM (50 mL × 3) was added for extraction. The organic phases were combined, dried over anhydrous sodium sulfate and concentrated under reduced pressure to give a tan oil (1 g, crude product). LC-MS: [M+H]⁺ = 318.00.

### 46.3 Preparation of DMSO solution of compound 2-(3,3-difluoroazetidin-1-carbonyl)-1-methyl-1,4,5,7-tetrahydro-6H-pyrrolo [2,3-c]pyridine-6-carbohydrazide (L046-3)

L046-2 (1.0 g, 3.147 mmol) was weighed out and placed in a 50 mL eggplant-shaped bottle, and DMSO (10 mL) was added for dissolution, followed by slow dropwise addition of hydrazine hydrate (1.6 g, 31.474 mmol) under an ice bath. After the dropwise addition was completed, the mixture was reacted overnight at room temperature. After the reaction was completed as detected by LCMS, the DMSO solution of L046-3 (10 mL) was given, which was light yellow. LC-MS: [M+H]⁺ = 314.10.

### 46.4 Preparation of compound tert-butyl 2-(2-(3,3-difluoroazetidin-1-carbonyl)-1-methyl-4,5,6,7-tetrahydro-1H pyrrolo[2,3-c]pyridin-6-carbonyl)hydrazine-1-carboxylate (L046-4)

Water (10 mL), THF (10 mL), K₂CO₃ (2 g) and BoczO (5 mL) were added to the DMSO solution (10 mL) obtained in the previous step, and the mixture was reacted overnight at room temperature. After the reaction was completed as detected by LCMS, an appropriate amount of water was added for dilution, and EA (50 mL × 3) was added for extraction. The organic phases were combined, dried over anhydrous sodium sulfate and concentrated under reduced pressure to give a crude product, and the crude product was separated by silica gel column chromatography (PE:EA = 1:2) to give an off-white solid (380 mg). LC-MS: [M+H]⁺ = 414.20.

### 46.5 Preparation of compound 2-(3,3-difluoroazetidin-1-carbonyl)-1-methyl-1,4,5,7-tetrahydro-6H pyrrolo[2,3-c]pyridine-6-carbohydrazide (L046-3)

L046-4 (380 mg, 0.921 mmol) was weighed out and placed in a 50 mL eggplant-shaped bottle, and DCM (4 mL) was added for dissolution, followed by addition of trifluoroacetic acid (2 mL). The mixture was reacted at room temperature for 1 h. After the reaction was completed as detected by LCMS, the mixture was concentrated under reduced pressure to give a light yellow oil (380 mg, crude product). LC-MS: [M+H]⁺ = 314.15.

### 46.6 Preparation of compound tert-butyl 4-(4-(2-(2-(3,3-difluoroazetidin-1-carbonyl)-1-methyl-4,5,6,7-tetrahydro-1H-pyrrolo[2,3-c]pyridin-6-carbonyl)hydrazine-1-thioamino)phenyl)-1H-pyrazole-1-carboxylate (L046-5)

L046-3 (300 mg, 0.958 mmol, crude product) was weighed out and placed in a 50 mL eggplant-shaped bottle, and DIEA (248 mg, 1.916 mmol) was added, followed by addition of dichloromethane (10 mL) for dissolution. The mixture was stirred at room temperature for 10 min, and then a solution of L046-1 (288 mg, 0.958 mmol) in dichloromethane was added dropwise. After the dropwise addition was completed, the mixture was reacted overnight at room temperature. After the reaction was completed as detected by LCMS, the reaction liquid was concentrated under reduced pressure to give a crude product, and the crude product was separated by silica gel column chromatography (DCM:MeOH = 1:1-1:3) to give a light yellow solid (360 mg). LC-MS: [M+H]⁺ = 615.15.

### 46.7 Preparation of compound tert-butyl 4-(4-((5-(2-(3,3-difluoroazetidin-1-carbonyl)-1-methyl-1,4,5,7-tetrahydro-6H-pyrrolo[2,3-c]pyridin-6-yl)-1,3,4-oxadiazol-2-yl)amino)phenyl)-1H-pyrazole-1-carboxylate (L046-6)

L046-5 (360 mg, 0.586 mmol) was weighed out and placed in a 50 mL eggplant-shaped bottle, and EDCI (1.122 g, 5.856 mmol) was added, followed by addition of NMP (20 mL) for dissolution. The mixture was stirred at 60 °C for 5 h. After the reaction was completed as detected by LCMS, an appropriate amount of water was added for dilution, and EA (50 mL × 3) was added for extraction. The organic phases were combined, dried over anhydrous sodium sulfate and concentrated under reduced pressure to give a crude product, and the crude product was separated by silica gel column chromatography (DCM:MeOH = 20:1-10:1) to give a light yellow solid (280 mg).

### 46.8 Preparation of compound (6-(5-((4-(1H-pyrazol-4-yl)phenyl)amino)-1,3,4-oxadiazol-2-yl)-1-methyl-4,5,6,7-tetrahydro-1H-pyrrolo[2,3-c]pyridin-2-yl)(3,3-difluoroazetidin-1-yl)methanone (L046)

L046-6 (260 mg, 0.448 mmol) was weighed out and placed in a 50 mL eggplant-shaped bottle, and DCM (16 mL) was added for dissolution, followed by addition of trifluoroacetic acid (4 mL). The mixture was reacted at room temperature for 2 h. After the reaction was completed as detected by LCMS, the mixture was concentrated under reduced pressure to remove the solvent. Water was added to dilute the residue, the pH was adjusted to 9 with saturated NaHCO₃, and EA (50 mL × 3) was added for extraction. The organic phase was collected to give a crude product, and the crude product was separated by prep-HPLC and lyophilized to give a white solid (28.0 mg, 98.641% purity). LC-MS: [M+H]⁺ = 481.15.

¹H-NMR (400 MHz, DMSO-*d*) δ 10.07 (s, 1H), 7.94 (s, 1H), 7.52 (d, *J* = 8.7 Hz, 1H), 7.45 (d, *J* = 8.7 Hz, 1H), 6.47 (s, 1H), 4.55 (s, 1H), 4.47 (s, 1H), 3.70 (s, 1H), 3.59 (t, *J=* 5.6 Hz, 1H), 2.62 (t, *J=* 5.5 Hz, 1H).

### Example 47. Preparation of Compound (6-(4-((4-(1H-pyrazol-4-yl)phenyl)amino)-5-fluoropyrimidin-2-yl)-1-(difluoromethyl)-4,5,6,7-tetrahydro-1H-pyrrolo[2,3-c]pyridin-2-yl)(3,3-difluoroazetidin-1-yl)methanone (L047)

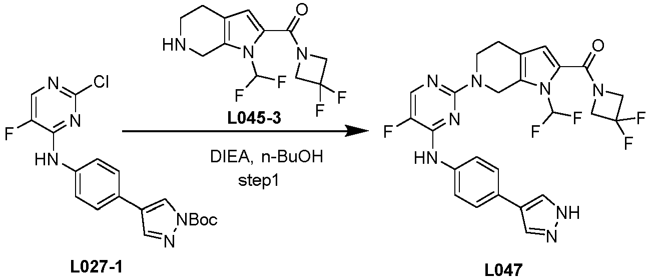

*Tert*-butyl 4-(4-((2-chloro-5-fluoropyrimidin-4-yl)amino)phenyl)-1*H*-pyrazole-1-carboxylate (190 mg, 0.49 mmol) was weighed out and placed in a 50 mL eggplant-shaped bottle, and n-BuOH (10 mL) was added for dissolution, followed by addition of DIEA (0.40 mL, 2.45 mmol) and hydrochloride of L045-3 (192 mg, 0.59 mmol). The mixture was reacted at 120 °C for 16 h. After the reaction was completed as detected by TLC, the reaction was stopped. The mixture was concentrated under reduced pressure to remove the solvent. Water (50 mL × 3) was added for washing, and then EA (50 mL × 3) was added for extraction. The organic phase was collected to give a crude product, and the crude product was separated by prep-HPLC and lyophilized to give a white solid (38.0 mg, 98.251% purity). LC-MS: [M+H]⁺ = 545.15.

¹H NMR (400 MHz, dmso) δ 12.83 (s, 1H), 9.33 (s, 1H), 8.36 (s, 0H), 8.21 (s, 1H), 8.04 (t, *J* = 13.2 Hz, 3H), 7.75 (d, *J* = 8.7 Hz, 2H), 7.54 (d, *J=* 8.6 Hz, 2H), 6.69 (s, 1H), 4.97 (s, 2H), 4.62 (s, 4H), 3.94 (t, *J=* 5.6 Hz, 2H), 2.58 (t, *J* = 5.1 Hz, 2H).

Example 48. Preparation of Compound (6-(4-((4-(1*H*-pyrazol-4-yl)phenyl)amino)-6-ethyl-1,3,5-triazin-2-yl)-1-methyl-4,5,6,7-tetrahydro-1H pyrrolo[2,3-c]pyridin-2-yl)(3,3-difluoroazetidin-1-yl)methanone (L048)

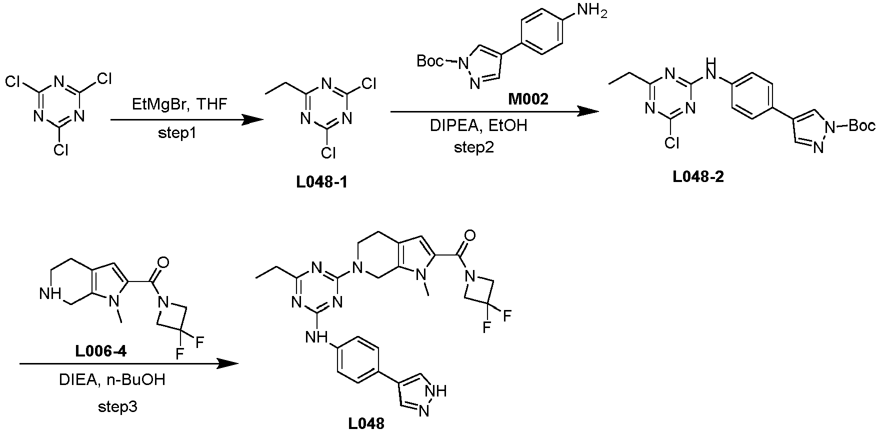

### 48.1. Preparation of compound 2,4-dichloro-6-ethyl-1,3,5-triazine (L048-1):

2,4,6-trichloro-1,3,5-triazine (1.84 g, 10.0 mmol) was dissolved in DCM (20 mL), and the mixture was then cooled to -10 °C under a dry ice/acetonitrile bath, followed by dropwise addition of ethyl magnesium bromide solution (11.0 mmol). After the addition was completed, the mixture was reacted at a low temperature for 0.5 h and then naturally warmed to room temperature. Then ammonium chloride was added to quench the reaction, and an EA/water system was added for extraction. The organic phase was dried over anhydrous sodium sulfate and concentrated by rotary evaporation to give a yellow solid (1.4 g), which was used directly in the next step. LC-MS: [M+H]⁺ = 178.00.

### 48.2 Preparation of compound tert-butyl 4-(4-((4-chloro-6-ethyl-1,3,5-triazin-2-yl)amino)phenyl)-1H pyrazole-1-carboxylate (L048-2):

2,4-dichloro-6-ethyl-1,3,5-triazine (534 mg, 3.0 mmol) and tert-butyl 4-(4-aminophenyl)-1*H*-pyrazole-1-carboxylate (932 mg, 3.6 mmol) were each weighed out and placed in a 50 mL eggplant-shaped bottle, and ethanol (30 mL) was added for dissolution, followed by addition of DIEA (1.5 mL, 9.0 mmol). The mixture was stirred overnight at 60 °C. After the reaction was completed as detected by TLC, the reaction was stopped. The reaction liquid was concentrated under reduced pressure and extracted with an EA/water system, and the resulting crude product was subjected to silica gel column chromatography (PE:EA = 4:1) to give a yellow solid (1.4 g). LC-MS: [M+H]⁺ = 401.10.

### 48.3 Preparation of compound (6-(4-((4-(1H-pyrazol-4-yl)phenyl)amino)-6-ethyl-1,3,5-triazin-2-yl)-1-methyl-4,5,6,7-tetrahydro-1H-pyrrolo[2,3-c]pyridin-2-yl)(3,3-difluoroazetidin-1-yl)methanone (L048):

L048-2 (400.87 mg, 1 mmol) and (3,3-difluoroazetidin-1-yl)(1-methyl-4,5,6,7-tetrahydro-1H pyrrolo[2,3-c]pyridin-2-yl)methanone (255.27 mg, 1 mmol) were each weighed out and placed in a 25 mL single-neck flask, and n-butanol (5 mL) was added for dissolution, followed by addition of DIEA (646.2 mg, 5 mmol). The mixture was stirred at 120 °C for 3 h. After the reaction was completed as detected by LCMS, the reaction liquid was concentrated and extracted twice with EA/H₂O. The organic phase was separated out, dried and concentrated to give a crude product, and the crude product was subjected to preparative chromatography and lyophilized to give a white solid (135 mg, 97.309% purity). LC-MS: [M+H]⁺ = 520.20.

¹H-NMR (400 MHz, DMSO-*d*) δ 12.87 (s, 1H), 9.62 (s, 1H), 8.11 (s, 1H), 7.88 (s, 1H), 7.74 (d, *J=* 8.6 Hz, 1H), 7.54 (d, *J=* 8.3 Hz, 1H), 6.48 (s, 1H), 4.86 (d, *J=* 11.9 Hz, 1H), 4.56 (t, *J=* 11.3 Hz, 2H), 4.04 (s, 1H), 3.77 (d, *J* = 9.9 Hz, 1H), 2.64 - 2.52 (m, 2H), 1.23 (t, *J* = 7.6 Hz, 2H).

### Example 50. Preparation of Compound 1-(6-(4-((4-(1H-pyrazol-4-yl)phenyl)amino)-6-methyl-1,3,5-triazin-2-yl)-1-methyl-4,5,6,7-tetrahydro-1H pyrrolo[2,3-c]pyridin-2-carbonyl)azetidine-3-carbonitrile (L050)

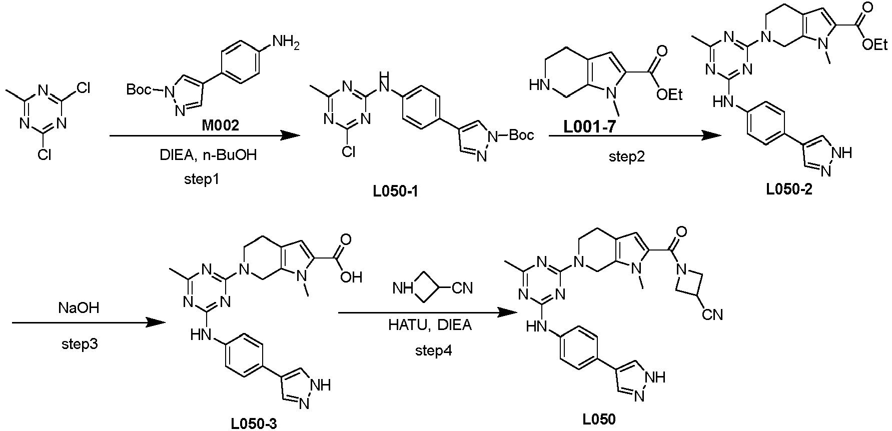

### 50.1. Preparation of compound tert-butyl 4-(4-((4-chloro-6-methyl-1,3,5-triazin-2-yl)amino)phenyl)-1H-pyrazole-1-carboxylate (L050-1)

2,4-dichloro-6-methyl-1,3,5-triazine (150 mg, 0.915 mmol) and *tert*-butyl 4-(4-aminophenyl)-1*H*-pyrazole-1-carboxylate (237 mg, 0.915 mmol) were each weighed out and placed in a 25 mL single-neck flask, and *n-*butanol (5 mL) was added for dissolution, followed by addition of DIEA (591 mg, 4.573 mmol). The mixture was stirred at 70 °C for 3 h. After the reaction was completed as detected by LCMS, the reaction liquid was concentrated and extracted twice with EA/H₂O. The organic phase was separated out, dried and concentrated to give a light yellow solid (350 mg, crude product). LC-MS: [M+H]⁺ = 387.10.

### 50.2 Preparation of compound ethyl 6-(4-((4-(1H-pyrazol-4-yl)phenyl)amino)-6-methyl-1,3,5-triazin-2-yl)-1-methyl-4,5,6,7-tetrahydro-1H-pyrrolo[2,3-c]pyridine-2-carboxylate (L050-2)

L050-1 (300 mg, 0.776 mmol) and ethyl 1-methyl-4,5,6,7-tetrahydro-1*H*-pyrrolo[2,3-*c*]pyridine-2-carboxylate (162 mg, 0.776 mmol) were each weighed out and placed in a 25 mL single-neck flask, and *n-*butanol (5 mL) was added for dissolution, followed by addition of DIEA (502 mg, 3.88 mmol). The mixture was stirred at 130 °C for 6 h. After the reaction was completed as detected by LCMS, the reaction liquid was concentrated and extracted twice with EA/H₂O. The organic phase was separated out, dried and concentrated to give a light yellow solid (1.46 g, crude product). LC-MS: [M+H]⁺ = 459.20.

### 50.3 Preparation of compound 6-(4-((4-(1H-pyrazol-4-yl)phenyl)amino)-6-methyl-1,3,5-triazin-2-yl)-1-methyl-4,5,6,7-tetrahydro-1H pyrrolo[2,3-c]pyridine-2-carboxylic acid (L050-3)

L050-2 (1.36 g, 2.966 mmol), NaOH (356 mg, 8.898 mmol) and MeOH/H₂O (12 mL/3 mL) were each weighed out and placed in a 50 mL eggplant-shaped bottle, and the mixture was stirred at 55 °C for 12 h. After the reaction was completed as detected by LCMS, the reaction liquid was concentrated and extracted twice with EA/H₂O. The organic phase was separated out, dried and concentrated to give a light yellow solid (600 g, crude product). LC-MS: [M+H]⁺ = 387.15.

### 50.4 Preparation of compound 1-(6-(4-((4-(1H-pyrazol-4-yl)phenyl)amino)-6-methyl-1,3,5-triazin-2-yl)-1-methyl-4,5,6,7-tetrahydro-1H-pyrrolo[2,3-c]pyridin-2-carbonyl)azetidine-3-carbonitrile (L050)

L050-3 (200 mg, 0.465 mmol), 3-acetonitrile cyclobutylamine hydrochloride (83 mg, 0.697 mmol) and HATU (354 mg, 0.93 mmol) were each weighed out and placed in a 25 mL eggplant-shaped bottle, and DCM (3 mL) was added for dissolution, followed by addition of DIEA (181 mg, 1.395 mmol). The mixture was stirred at room temperature for 12 h. After the reaction was completed as detected by LCMS, the reaction liquid was extracted twice with DCM/H₂O. The organic phase was separated out, dried and concentrated to give a crude product, and the crude product was subjected to preparative chromatography and lyophilized to give a white solid (15.8 mg, 98.055% purity). LC-MS: [M+H]⁺ = 495.15.

¹H NMR (400 MHz, DMSO-d) δ 12.81 (s, 1H), 9.63 (s, 1H), 8.01 (s, 1H), 7.72 (d, J = 8.7 Hz, 1H), 7.54 (d, J = 8.2 Hz, 1H), 6.39 (s, 1H), 4.84 (s, 1H), 4.35 (d, J = 40.3 Hz, 2H), 4.02 (t, J = 5.3 Hz, 1H), 3.85 - 3.78 (m, 1H), 3.75 (d, J = 10.7 Hz, 1H), 2.57 (s, 1H), 2.27 (s, 1H).

### Example 51. Preparation of Compound 2-(4-((4-(1H-pyrazol-4-yl)phenyl)amino)-6-(2-(3,3-difluoroazetidin-1-carbonyl)-1-methyl-1,4,5,7-tetrahydro-6H-pyrrolo[2,3-c]pyridin-6-yl)-1,3,5-triazin-2-yl)acetonitrile (L051)

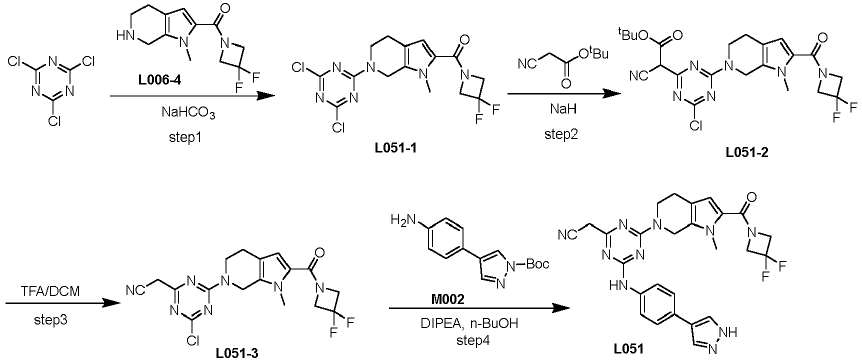

### 51.1 Preparation of compound (6-(4,6-dichloro-1,3,5-triazin-2-yl)-1-methyl-4,5,6,7-tetrahydro-1H-pyrrolo[2,3-c]pyridin-2-yl)(3,3-difluoroazetidin-1-yl)methanone (L051-1):

2,4,6-trichloro-1,3,5-triazine (184 mg, 1.0 mmol) was added in acetone (4 mL), and sodium bicarbonate (248 mg, 3.0 mmol) was added, followed by addition of (3,3-difluoroazetidin-1-yl)(1-methyl-4,5,6,7-tetrahydro-1*H*-pyrrolo[2,3-*c*]pyridin-2-yl)methanone hydrochloride (292 mg, 1.0 mmol) in batches. After the addition was completed, the mixture was reacted at room temperature for 3 h. After the product was present, which was the main peak, as detected by LCMS, the reaction liquid was extracted with an EA/water system, and the organic phase was dried and concentrated by rotary evaporation to give a yellow solid (400 mg), which was used directly in the next step. LC-MS: [M+H]⁺ = 403.05

### 51.2 Preparation of compound tert-butyl 2-(4-chloro-6-(2-(3,3-difluoroazetidin-1-carbonyl)-1-methyl-1,4,5,7-tetrahydro-6H-pyrrolo[2,3-c)]pyridin-6-yl)-1,3,5-triazin-2-yl)-2-cyanoacetate (L051-2):

L051-1 (320 mg, 0.8 mmol) and tetrahydrofuran (3 mL) were added to a 25 mL reaction flask, and the mixture was stirred at room temperature. *Tert*-butyl cyanoacetate (128 mg, 0.88 mmol) and tetrahydrofuran (3 mL) were added to another 25 mL reaction flask, and NaH (40 mg) was added in batches under an ice bath. After the addition was completed, the mixture was reacted for 30 min at room temperature. The mixed solution was added dropwise to the solution of L051-1 in tetrahydrofuran. After the addition was completed, the mixture was reacted at room temperature for 4.0 h. After the reaction was completed as detected by LCMS, ammonium chloride was added to quench the reaction, and an EA/water system was added for extraction. The organic phase was dried and concentrated by rotary evaporation to give a yellow solid (400 mg, crude product), which was used directly in the next step. LC-MS: [M+H]⁺ = 508.15.

### 51.3 Preparation of compound 2-(4-chloro-6-(2-(3,3-difluoroazetidin-1-carbonyl)-1-methyl-1,4,5,7-tetrahydro-6H-pyrrolo[2,3-c]pyridin-6-yl)-1,3,5-triazin-2-yl)acetonitrile(L051-3):

L051-2 (400 mg, 0.79 mmol) was weighed out, and dichloromethane (4 mL) was added for dissolution, followed by addition of trifluoroacetic acid (1 mL). After the addition was completed, the mixture was reacted at room temperature for 2 h. After the reaction was completed as detected by LCMS, the following treatment was carried out: dichloromethane (20 mL) was added to dilute the reaction liquid, sodium bicarbonate was added for neutralization, and then dichloromethane/water system was added for extraction. The organic phase was dried and concentrated, and the residue was purified by column chromatography to give a white solid (200 mg). LC-MS: [M+H]⁺ = 408.10.

### 51.4 Preparation of compound 2-(4-((4-(1H-pyrazol-4-yl)phenyl)amino)-6-(2-(3,3-difluoroazetidin-1-carbonyl)-1-methyl-1,4,5,7-tetrahydro-6H-pyrrolo[2,3-c]pyridin-6-yl)-1,3,5-triazin-2-yl)acetonitrile (L051):

L051-3 (180 mg, 0.44 mmol), tert-butyl 4-(4-aminophenyl)-1*H*-pyrazole-1-carboxylate (148 mg, 0.57 mmol), KF (38 mg, 0.66 mmol) and DIEA (170 mg, 1.32 mmol) were each weighed out and placed in a reaction flask, and n-butanol (3 mL) was added for dissolution. The mixture was reacted overnight at 90 °C. After the reaction was completed as detected by LCMS, the mixture was concentrated under reduced pressure and extracted with an EA/water system, and the organic phase was concentrated by rotary evaporation to give a crude product. The crude product was subjected to preparative chromatography and lyophilized to give a white solid (68.0 mg, 98.924% purity). LC-MS: [M+H]⁺ = 531.25.

¹H NMR (400 MHz, DMSO-d6) δ 12.85 (s, 1H), 9.85 (s, 1H), 7.98 (d, J = 88.1 Hz, 2H), 7.71 (d, J = 8.4 Hz, 2H), 7.53 (s, 2H), 6.46 (d, J = 4.1 Hz, 1H), 4.85 (d, J = 10.2 Hz, 2H), 4.53 (s, 4H), 4.02 (s, 4H), 3.73 (d, J = 19.2 Hz, 3H), 2.55 (s, 2H).

### Example 52. Preparation of Compound (6-(4-((4-(1H-pyrazol-4-yl)phenyl)amino)-6-(oxetan-3-yl)-1,3,5-triazin-2-yl)-1-methyl-4,5,6,7-tetrahydro-1H-pyrrolo[2,3-c]pyridin-2-yl)(3,3-difluoroazetidin-1-yl)methanone (L052)

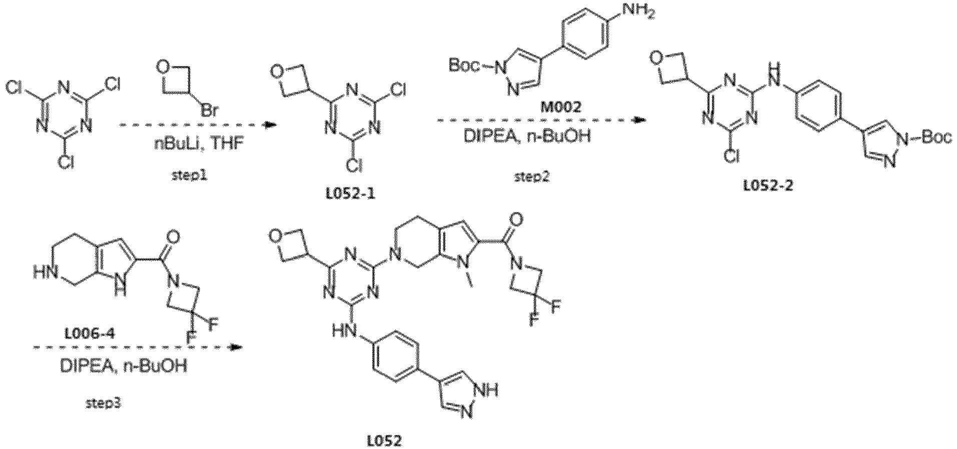

### 52.1 Preparation of compound 2,4-dichloro-6-(oxetan-3-yl)-1,3,5-triazine (L052-1):

3-bromooxetane (411 mg, 3.0 mmol) was added in tetrahydrofuran (4 mL), and the mixture was cooled to - 78 °C under a dry ice/ethyl acetate bath, followed by dropwise addition of n-butyl lithium solution (3.0 mmol). After the addition was completed, the mixture was reacted at a low temperature for 0.5 h.

2,4,6-trichloro-1,3,5-triazine (607 mg, 3.3 mmol) was weighed out and placed in another reaction flask, and tetrahydrofuran (6.0 mL) was added. The mixture was cooled to -78 °C under a dry ice/ethyl acetate bath, and the 3-bromooxetane solution described above was added dropwise. After the addition was completed, the mixture was reacted at a low temperature for 0.5 h, then naturally warmed to room temperature and stirred for 1 h. After about 50% product was present as detected by LCMS, the reaction liquid was quenched by ammonium chloride and extracted with an EA/water system, and the organic phase was dried and concentrated by rotary evaporation to give a yellow solid (500 mg), which was used directly in the next step.

LC-MS: [M+H]⁺ = 206.00.

### 52.2 Preparation of compound tert-butyl 4-(4-((4-chloro-6-(oxetan-3-yl)-1,3,5-triazin-2-yl)amino)phenyl)-1H pyrazole-1-carboxylate (L052-2):

2,4-dichloro-6-(oxetan-3-yl)-1,3,5-triazine (206 mg, 1.0 mmol) and M002 (260 mg, 1.0 mmol) were each weighed out and placed in a 50 mL eggplant-shaped bottle, and ethanol was added for dissolution, followed by addition of DIEA (390 mg, 3.0 mmol). The mixture was stirred overnight at 50 °C. After the reaction was completed as detected by LCMS, the reaction liquid was concentrated under reduced pressure and extracted with an EA/water system to give a yellow solid (400 mg, crude product), which was used directly in the next step. LC-MS: [M+H]⁺ = 429.15.

### 52.3 Preparation of compound (6-(4-((4-(1H-pyrazol-4-yl)phenyl)amino)-6-(oxetan-3-yl)-1,3,5-triazin-2-yl)-1-methyl-4,5,6,7-tetrahydro-1H pyrrolo[2,3-c]pyridin-2-yl)(3,3-difluoroazetidin-1-yl)methanone (L052):

*Tert*-butyl 4-(4-((4-chloro-6-(oxetan-3-yl)-1,3,5-triazin-2-yl)amino)phenyl)-1*H*-pyrazole-1-carboxylate (400 mg, 0.93 mmol), L006-4 hydrochloride (298 mg, 1.02 mmol) and DIEA (360 mg, 2.79 mmol) were weighed out and placed in a reaction flask, and n-butanol (5 mL) was added for dissolution. The mixture was reacted overnight at 100 °C. After the reaction was completed as detected by LCMS, the mixture was concentrated under reduced pressure and extracted with an EA/water system, and the organic phase was concentrated by rotary evaporation to give a crude product. The crude product was subjected to preparative chromatography and lyophilized to give a white solid (68.0 mg, 96.727% purity). LC-MS: [M+H]⁺ = 548.20.

### Example 53. Preparation of Compound (6-(4-((4-(1H-pyrazol-4-yl)phenyl)amino)-6,7-dihydro-5H-cyclopenta[d]pyrimidin-2-yl)-1-methyl-4,5,6,7-tetrahydro-1H-pyrrolo[2,3-c]pyridin-2-yl)(3,3-difluoroazetidin-1-yl)methanone (L053)

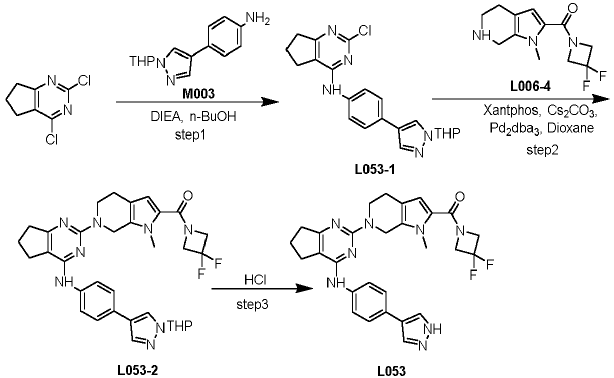

### 53.1 Preparation of compound 2-chloro-N (4-(1-(tetrahydro-2H pyran-2-yl)-1H pyrazol-4-yl)phenyl)-6,7-dihydro-5H-cyclopenta[d]pyrimidin-4-amine (L053-1):

2,4-dichloro-6,7-dihydro-5*H*-cyclopenta[*d*]pyrimidine (189.04 mg, 1 mmol) and 4-(1-(tetrahydro-2H pyran-2-yl)-1*H*-pyrazol-4-yl)aniline (243.31 mg, 1 mmol) were each weighed out and placed in a 25 mL eggplant-shaped bottle, and n-butanol (5 mL) was added for dissolution, followed by addition of DIEA (646.2 mg, 5 mmol). The mixture was stirred at 100 °C for 12 h. After the reaction was completed as detected by LCMS, the reaction liquid was concentrated and extracted twice with EA/H₂O. The organic phase was separated out, dried and concentrated, and the residue was separated by silica gel column chromatography (PE/EA = 4/1) to give a light yellow solid (176 mg). LC-MS: [M+H]⁺ = 396.15.

### 53.2 Preparation of compound (3,3-difluoroazetidin-1-yl)(1-methyl-6-(4-((4-(1-(tetrahydro-2H pyran-2-yl)-1H-pyrazol-4-yl)phenyl)amino)-6,7-dihydro-5H-cyclopenta[d]pyrimidin-2-yl)-4,5,6,7-tetrahydro-1H-pyrrolo[2,3-c]pyridin-2-yl)methanone (L053-2):

L053-1 (150 mg, 0.379 mmol), (3,3-difluoroazetidin-1-yl)(1-methyl-4,5,6,7-tetrahydro-1H pyrrolo[2,3-c]pyridin-2-yl)methanone (110 mg, 0.379 mmol), Pd₂dba₃ (69.4 mg, 0.0758 mmol), xantphos (43.8 mg, 0.0758 mmol) and Cs₂CO₃ (246 mg, 0.758 mmol) were each weighed out and placed in a 25 mL single-neck flask, and dioxane (5 mL) was added for dissolution. The reaction system was purged with nitrogen 3 times and stirred at 100 °C for 12 h. After the reaction was completed as detected by LCMS, the reaction liquid was concentrated and extracted twice with EA/H₂O. The organic phase was separated out, dried and concentrated, and the residue was separated by silica gel column chromatography (PE/EA = 3/1) to give a light yellow solid (100 mg). LC-MS: [M+H]⁺ = 615.25.

### 53.3 Preparation of compound (6-(4-((4-(1H-pyrazol-4-yl)phenyl)amino)-6,7-dihydro-5H cyclopenta[d]pyrimidin-2-yl)-1-methyl-4,5,6,7-tetrahydro-1H-pyrrolo[2,3-c]pyridin-2-yl)(3,3-difluoroazetidin-1-yl)methanone (L053):

L053-2 (100 mg, 0.163 mmol), acidic resin (20 mg) and a HCl/dioxane solution (0.4 mL) were each weighed out and placed in a 25 mL eggplant-shaped bottle, and methanol (3 mL) was added for dissolution. The mixture was stirred at room temperature for 2 h. After the reaction was completed as detected by LCMS, the reaction liquid was filtered and concentrated to give a crude product, and the crude product was subjected to preparative chromatography and lyophilized to give a white solid (5 mg, 98.148% purity). LC-MS: [M+H]⁺= 531.20.

¹H NMR (400 MHz, DMSO-*d*) δ 12.85 (s, 1H), 8.46 (s, 1H), 8.11 (s, 1H), 7.90 (s, 1H), 7.72 (d, *J* = 8.7 Hz, 2H), 7.55 (d, *J* = 8.6 Hz, 2H), 6.45 (s, 1H), 4.76 (s, 2H), 4.55 (t, *J* = 11.8 Hz, 4H), 3.94 (t, *J* = 5.6 Hz, 2H), 3.75 (s, 3H), 2.75 - 2.67 (m, 4H), 2.54 (d, *J* = 5.5 Hz, 2H), 2.01 - 1.96 (m, 2H).

### Example 56. Preparation of Compound 1-(6-(4-((4-(1H-pyrazol-4-yl)phenyl)amino)-5-fluoropyrimidin-2-yl)-1-methyl-4,5,6,7-tetrahydro-1H-pyrrolo[2,3-c]pyridin-2-carbonyl)azetidine-3-carbonitrile (L056)

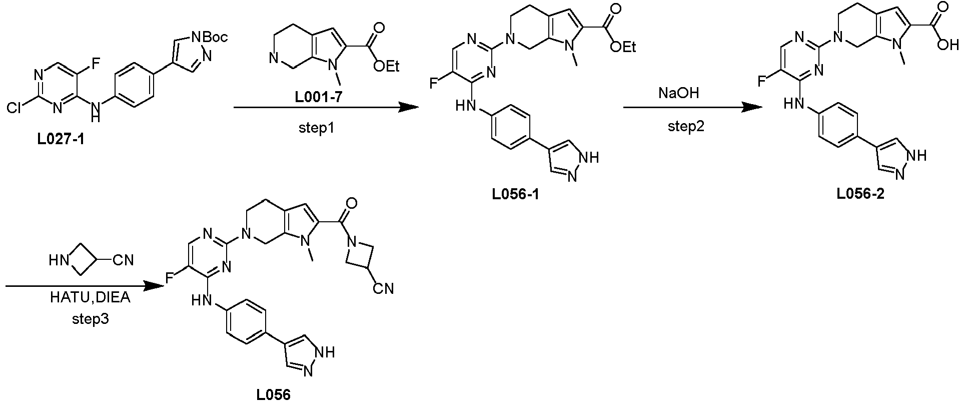

### 56.1 Preparation of compound ethyl 6-(4-((4-(1H-pyrazol-4-yl)phenyl)amino)-5-fluoropyrimidin-2-yl)-1-methyl-4,5,6,7-tetrahydro-1H pyrrolo[2,3-c]pyridine-2-carboxylate (L056-1):

*Tert*-butyl 4-(4-((2-chloro-5-fluoropyrimidin-4-yl)amino)phenyl)-1*H*-pyrazole-1-carboxylate (450 mg, 1.154 mmol) and ethyl 1-methyl-1,4,5,7-tetrahydro-1*H*-pyrrolo[2,3-c]pyridine-2-carboxylate (240 mg, 1.154 mmol) were each weighed out and placed in a 50 mL single-neck flask, and n-butanol (6 mL) was added for dissolution, followed by addition of DIEA (746 mg, 5.770 mmol). The mixture was stirred at 140 °C for 4 h. After the reaction was completed as detected by LCMS, the reaction liquid was concentrated and extracted twice with EA/H₂O. The organic phase was separated out, dried and concentrated, and the residue was separated by silica gel column chromatography (PE/EA = 5/1) to give a light yellow solid (406 mg). LC-MS: [M+H]⁺ = 462.15.

### 56.2 Preparation of compound 6-(4-((4-(1H-pyrazol-4-yl)phenyl)amino)-5-fluoropyrimidin-2-yl)-1-methyl-4,5,6,7-tetrahydro-1H pyrrolo[2,3-c]pyridine-2-carboxylic acid (L056-2):

L056-1 (406 mg, 0.880 mmol), NaOH (352 mg, 8.80 mmol) and MeOH/H₂O (5 mL/5 mL) were each weighed out and placed in a 50 mL single-neck flask, and the mixture was stirred at 155 °C for 12 h. After the reaction was completed as detected by LCMS, the reaction liquid was concentrated and extracted twice with EA/H₂O. The organic phase was separated out, dried and concentrated to give a light yellow solid (140 mg, crude product). LC-MS: [M+H]⁺ = 434.15.

### 56.3 Preparation of compound 1-(6-(4-((4-(1H-pyrazol-4-yl)phenyl)amino)-5-fluoropyrimidin-2-yl)-1-methyl-45,6,7-tetrahydro-1H-pyrrolo[2,3-c]pyridin-2-carbonyl)azetidine-3-carbonitrile (L056):

L056-2 (140 mg, 0.323 mmol), 3-acetonitrile cyclobutylamine hydrochloride (58 mg, 0.484 mmol) and HATU (246 mg, 0.646 mmol) were each weighed out placed in a 25 mL eggplant-shaped bottle, and DCM (3 mL) was added for dissolution, followed by addition of DIEA (126 mg, 0.969 mmol). The mixture was stirred at room temperature for 12 h. After the reaction was completed as detected by LCMS, the reaction liquid was extracted twice with DCM/H₂O. The organic phase was separated out, dried and concentrated to give a crude product, and the crude product was subjected to preparative chromatography and lyophilized to give a white solid (22.4 mg, 99.220% purity). LC-MS: [M+H]⁺ = 498.25.

¹H NMR (400 MHz, DMSO-*d*) δ 12.83 (s, 1H), 9.31 (s, 1H), 8.05 (d, *J=* 3.7 Hz, 1H), 7.79 - 7.72 (m, 1H), 7.61 - 7.55 (m, 1H), 6.37 (s, 1H), 4.72 (s, 1H), 4.34 (d, *J=* 41.2 Hz, 2H), 3.91 (t, *J=* 5.6 Hz, 1H), 3.80 (tt, *J* = 9.2, 6.1 Hz, 1H), 3.73 (s, 1H), 2.55 (t, *J=* 5.3 Hz, 1H).

### Example 57. Preparation of Compound (6-(4-((4-(1H-pyrazol-4-yl)phenyl)amino)pyrrolo[2,1-f][1,2,4]triazin-2-yl)-1-methyl-4,5,6,7-tetrahydro-1H pyrrolo[2,3-c]pyridin-2-yl)(3,3-difluoroazetidin-1-yl)methanone (L057)

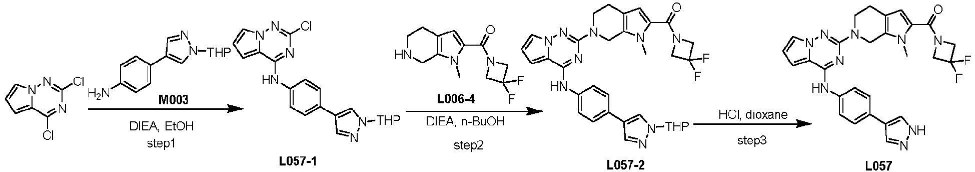

### 57.1 Preparation of compound 2-chloro-N (4-(1-(tetrahydro-2H pyran-2-yl)-1H pyrazol-4-yl)phenyl)pyrrolo[2,1-f][1,2,4]triazin-4-amine (L057-1):

2,4-dichloropyrrolo[2,1-*f*][1,2,4]triazine (400 mg, 2.128 mmol) and 4-(1-(tetrahydro-2*H*-pyran-2-yl)-1*H* pyrazol-4-yl)aniline (518 mg, 2.128 mmol) were each weighed out and placed in a 50 mL eggplant-shaped bottle, and n-butanol (10 mL) was added for dissolution, followed by addition of DIEA (1375 mg, 10.64 mmol). The mixture was stirred at 40 °C for 12 h. After the reaction was completed as detected by LCMS, the reaction liquid was concentrated and extracted twice with EA/H₂O. The organic phase was separated out, dried and concentrated, and the residue was separated by silica gel column chromatography (PE/EA = 4/1) to give a light yellow solid (579 mg). LC-MS: [M+H]⁺ = 395.15.

### 57.2 Preparation of compound (3,3-difluoroazetidin-1-yl)(1-methyl-6-(4-((4-(1-(tetrahydro-2H pyran-2-yl)-1H-pyrazol-4-yl)phenyl)amino)pyrrolo[2,1-f][1,2,4]triazin-2-yl)-4,5,6,7-tetrahydro-1H-pyrrolo[2,3-c]pyridin-2-yl)methanone (L057-2):

L057-1 (360 mg, 0.912 mmol), (3,3-difluoroazetidin-1-yl)(1-methyl-4,5,6,7-tetrahydro-1H pyrrolo[2,3-c]pyridin-2-yl)methanone (265 mg, 0.912 mmol), Pd₂dba₃ (168 mg, 0.1824 mmol), dit-Bu-xphos (78 mg, 0.1824 mmol) and t-BuONa (176 mg, 1.824 mmol) were each weighed out and placed in a 50 mL single-neck flask, and dioxane (10 mL) was added for dissolution. The reaction system was purged with nitrogen 3 times and stirred at 100 °C for 18 h. After the reaction was completed as detected by LCMS, the reaction liquid was concentrated and extracted twice with EA/H₂O. The organic phase was separated out, dried and concentrated, and the residue was separated by silica gel column chromatography (PE/EA = 3/1) to give a light yellow solid (83.6 mg). LC-MS: [M+H]⁺ = 614.25.

### 57.3 Preparation of compound (6-(4-((4-(1H-pyrazol-4-yl)phenyl)amino)pyrrolo[2,1-f][1,2,4]triazin-2-yl)-1-methyl-4,5,6,7-tetrahydro-1Hpyrrolo[2,3-c]pyridin-2-yl)(3,3-difluoroazetidin-1-yl)methanone (L057):

L057-2 (80 mg, 0.130 mmol), acidic resin (100 mg) and a HCl/dioxane solution (0.1 mL) were each weighed out and placed in a 25 mL eggplant-shaped bottle, and methanol (3 mL) was added for dissolution. The mixture was stirred at room temperature for 2 h. After the reaction was completed as detected by LCMS, the reaction liquid was filtered and concentrated to give a crude product, and the crude product was subjected to preparative chromatography and lyophilized to give a white solid (3 mg, 98.796% purity). LC-MS: [M+H]⁺= 530.15.

¹H-NMR (400 MHz, DMSO-*d*) δ 12.91 (s, 1H), 9.70 (s, 1H), 8.19 (s, 1H), 7.93 (s, 1H), 7.82 (d, *J* = 8.7 Hz, 1H), 7.65 (d, *J* = 8.7 Hz, 1H), 7.49 (dd, *J* = 2.3,1.7 Hz, 1H), 7.02 (dd, *J* = 4.4,1.5 Hz, 1H), 6.51 (dd, *J* = 4.4, 2.5 Hz, 1H), 6.46 (s, 1H), 4.67 (s, 1H), 4.63 - 4.45 (m, 2H), 3.88 (t, *J=* 5.5 Hz, 1H), 3.76 (s, 1H), 2.59 (t, *J=* 5.4 Hz, 1H).

### Example 58. Preparation of Compound (6-(5-((4-(1H-pyrazol-4-yl)phenyl)amino)-1,3,4-thiadiazol-2-yl)-1-methyl-4,5,6,7-tetrahydro-1H-pyrrolo[2,3-c]pyridin-2-yl)(3,3-difluoroazetidin-1-yl)methanone (L058)

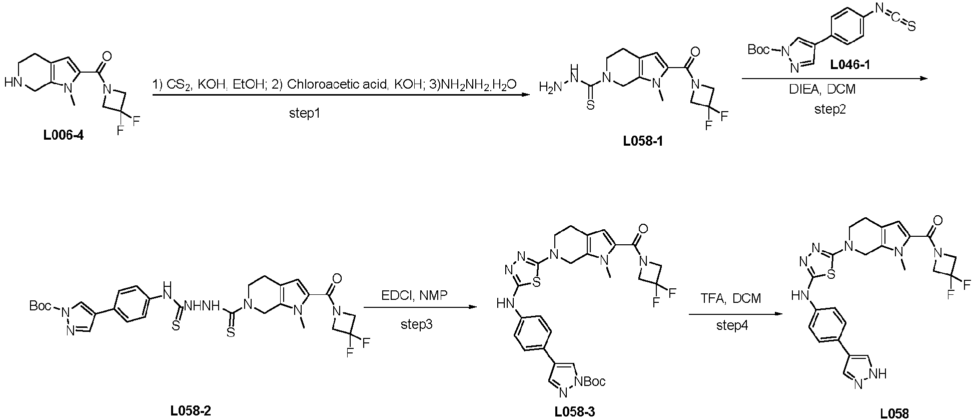

### 58.1 Preparation of compound 2-(3,3-difluoroazetidin-1-carbonyl)-1-methyl-1,4,5,7-tetrahydro-6H pyrrolo[2,3-c]pyridine-6-carbothiohydrazide (L058-1)

(3,3-difluoroazetidin-1-yl)(1-methyl-4,5,6,7-tetrahydro-1*H* pyrrolo[2,3-c]pyridin-2-yl)methanone (400 mg, 1.568 mmol) and KOH (176 mg, 3.137 mmol) were each weighed out and placed in a 100 mL eggplant-shaped bottle, and ethanol was added for dissolution, followed dropwise addition of a solution of CS₂ (238 mg, 3.137 mmol) in ethanol under an ice bath. After the dropwise addition was completed, the mixture was reacted for 5 h at a temperature of below 10 °C. A mixed solution of chloroacetic acid (298 mg, 3.137 mmol) and KOH (176 mg, 3.137 mmol) in ethanol was added, and after the addition was completed, the mixture was reacted overnight at room temperature. After the starting material was completely reacted as detected by LCMS, hydrazine hydrate (784 mg, 15.683 mmol) was added to the reaction liquid, and the mixture was reacted at 65 °C for 4 h. After the reaction was completed as detected by LCMS, the reaction liquid was concentrated under reduced pressure, water was added for dilution, and then ethyl acetate (50 mL × 3) was added for extraction. The organic phase was collected to give a crude product, and the crude product was separated by silica gel column chromatography (DCM/MeOH = 50:1-20:1) to give an off-white solid (350 mg). LC-MS: [M+H]⁺ = 330.05.

### 58.2 Preparation of compound tert-butyl 4-(4-(2-(2-(3,3-difluoroazetidin-1-carbonyl)-1-methyl-4,5,6,7-tetrahydro-1H-pyrrolo[2,3-c]pyridine-6-carbothioacyl)hydrazine-1-carbothioamido)phenyl)-1H-pyrazole-1-carboxylate (L058-2)

L058-1 (330 mg, 1.001 mmol) was weighed out and placed in a 100 mL eggplant-shaped bottle, and DIEA (258 mg, 2.002 mmol) was added, followed by addition of dichloromethane (10 mL) for dissolution. The mixture was stirred at room temperature for 10 min, and then a solution of L046-1 (241 mg, 0.801 mmol) in dichloromethane was added dropwise. After the dropwise addition was completed, the mixture was reacted overnight at room temperature. After the reaction was completed as detected by LCMS, the reaction liquid was concentrated under reduced pressure to give a crude product, and the crude product was separated by silica gel column chromatography (DCM:MeOH = 20:1) to give a yellow solid (150 mg). LC-MS: [M+H]⁺ = 631.15.

### 58.3 Preparation of compound tert-butyl 4-(4-((5-(2-(3,3-difluoroazetidin-1-carbonyl)-1-methyl-1,4,5,7-tetrahydro-6H-pyrrolo[2,3-c]pyridin-6-yl)-1,3,4-thiadiazol-2-yl)amino)phenyl)-1H-pyrazole-1-carboxylate (L058-3)

L058-2 (100 mg, 0.156 mmol) was weighed out and placed in a 50 mL eggplant-shaped bottle, and EDCI (303 mg, 1.561 mmol) was added, followed by addition of NMP (10 mL) for dissolution. The mixture was stirred at 50 °C for 5 h. After the reaction was completed as detected by LCMS, an appropriate amount of water was added for dilution, and EA (30 mL × 3) was added for extraction. The organic phases were combined, dried over anhydrous sodium sulfate and concentrated under reduced pressure to give a crude product, and the crude product was separated by silica gel column chromatography (DCM:MeOH = 50:1-20:1) to give a yellow oil (80 mg). LC-MS: [M+H]⁺ = 597.20.

### 58.4 Preparation of compound (6-(5-((4-(1H-pyrazol-4-yl)phenyl)amino)-1,3,4-thiadiazol-2-yl)-1-methyl-4,5,6,7-tetrahydro-1H-pyrrolo[2,3-c]pyridin-2-yl)(3,3-difluoroazetidin-1-yl)methanone (L058)

L058-3 (80 mg, 0.134 mmol) was weighed out and placed in a 25 mL eggplant-shaped bottle, and DCM (6 mL) was added for dissolution, followed by addition of trifluoroacetic acid (2 mL). The mixture was reacted at room temperature for 1 h. After the reaction was completed as detected by LCMS, the mixture was concentrated under reduced pressure to remove the solvent. Water was added to dilute the residue, the pH was adjusted to 9 with saturated NaHCO₃, and EA (20 mL × 3) was added for extraction. The organic phase was collected to give a crude product, and the crude product was separated by prep-HPLC and lyophilized to give a white solid (11.0 mg, 98.435% purity). LC-MS: [M+H]⁺ = 497.10.

¹H NMR (400 MHz, DMSO-d) δ 12.83 (s, 1H), 9.81 (s, 1H), 8.08 (s, 1H), 7.84 (s, 1H), 7.50 (td, *J* = 8.9, 2.2 Hz, 2H), 6.48 (s, 1H), 4.54 (d, *J* = 13.2 Hz, 2H), 3.73 (s, 1H), 3.61 (t, *J* = 5.7 Hz, 1H), 2.65 (dt, *J* = 11.1, 3.7 Hz, 1H).

### Example 59. Preparation of Compound (6-(4-((4-(1H-pyrazol-4-yl)phenyl)amino)-5-fluoropyrimidin-2-yl)-3-chloro-1-methyl-4,5,6,7-tetrahydro-1H-pyrrolo[2,3-c]pyridin-2-yl)(3,3-difluoropyrrolidin-1-yl)methanone (L059)

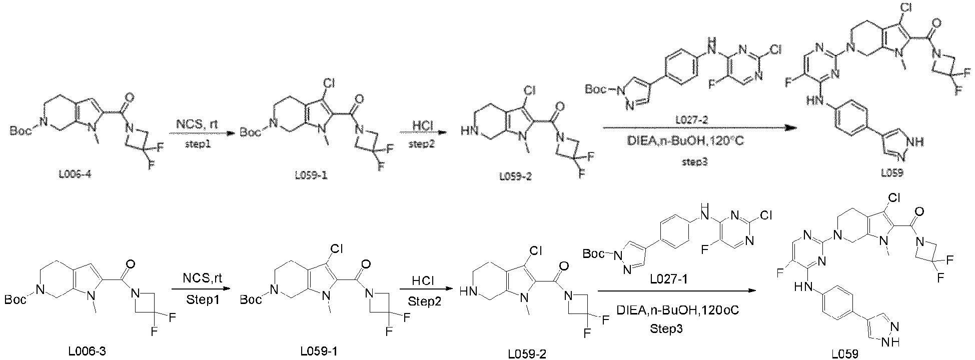

### 59.1 Preparation of compound tert-butyl 3-chloro-2-(3,3-difluoroazetidin-1-carbonyl)-1-methyl-1,4,5,7-tetrahydro-6H-pyrrolo[2,3-c|pyridine-6-carboxylate (L059-1):

*Tert*-butyl 2-(3,3-difluoroazetidin-1-carbonyl)-1-methyl-4,5-dihydro-1*H*-pyrrolo[2,3-*c*]pyridine-6(7*H*)-carboxylate (810 mg, 2.279 mmol) and NCS (304 mg, 2.279 mmol) were each weighed out and placed in a 50 mL single-neck flask, and MeCN (10 mL) was added. The mixture was stirred at room temperature for 2 h. After the reaction was completed and the product accounted for 20%, as detected by LCMS, the reaction liquid was concentrated and extracted twice with EA/H₂O. The organic phase was separated out, dried and concentrated, and the residue was separated by silica gel column chromatography (PE/EA = 4/1) to give a light yellow solid (200 mg). LC-MS: [M+H]⁺ = 390.10.

### 59.2 Preparation of compound (3-chloro-1-methyl-4,5,6,7-tetrahydro-1H-pyrrolo[2,3-c]pyridin-2-yl)(3,3-difluoroazetidin-1-yl)methanone (L059-2):

L059-1 (200 mg, 0.513 mmol), a HCl/dioxane solution (5 mL) and DCM (2 mL) were each weighed out and placed in a 50 mL single-neck flask, and the mixture was stirred at room temperature for 2 h. After the reaction was completed and the product accounted for 20%, as detected by LCMS, the reaction liquid was concentrated and extracted twice with EA/H₂O. The organic phase was separated out, dried and concentrated to give a light yellow solid (177.6 mg, crude product). LC-MS: [M+H]⁺ = 290.05.

### 59.3 Preparation of compound (6-(4-((4-(1H-pyrazol-4-yl)phenyl)amino)-5-fluoropyrimidin-2-yl)-3-chloro-1-methyl-4,5,6,7-tetrahydro-1H-pyrrolo[2,3-c]pyridin-2-yl)(3,3-difluoropyrrolidin-1-yl)methanone (L059):

L059-2 (145 mg, 0.445 mmol) and *tert*-butyl 4-(4-((2-chloro-5-fluoropyrimidin-4-yl)amino)phenyl)-1*H-*pyrazole-1-carboxylate (174 mg, 0.445 mmol) were each weighed out and placed in a 25 mL eggplant-shaped bottle, and n-butanol (3 mL) was added for dissolution, followed by addition of DIEA (288 mg, 2.225 mmol). The mixture was stirred at 120 °C for 2 h. After the reaction was completed as detected by LCMS, the reaction liquid was concentrated and extracted twice with EA/H₂O. The organic phase was separated out, dried and concentrated to give a crude product, and the crude product was subjected to preparative chromatography and lyophilized to give a white solid (33 mg, 97.663% purity). LC-MS: [M+H]⁺ = 543.15. ¹H NMR (400 MHz, DMSO-*d*) δ 12.89 (s, 1H), 9.34 (s, 1H), 8.15 (s, 1H), 8.06 (d, *J* = 3.7 Hz, 1H), 7.90 (s, 1H), 7.77 - 7.72 (m, 1H), 7.62 - 7.57 (m, 1H), 4.74 (s, 1H), 4.52 (t, *J* = 11.9 Hz, 2H), 3.93 (t, *J* = 5.6 Hz, 1H), 3.59 (s, 2H), 2.52 (d, *J* = 1.9 Hz, 1H).

### Example 60. Preparation of Compound (6-(4-((4-(1H-pyrazol-4-yl)phenyl)amino)pyrimidin-2-yl)-1-methyl-4,5,6,7-tetrahydro-1H-pyrrolo[2,3-c]pyridin-2-yl)(3-(difluoromethyl)azetidin-1-yl)methanone (L060)

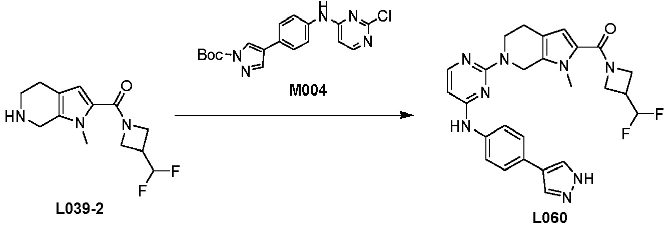

(3-(difluoromethyl)azetidin-1-yl)(1-methyl-4,5,6,7-tetrahydro-1H pyrrolo[2,3-c]pyridin-2-yl)methanone (55 mg, 0.20 mmol) was dissolved in n-butanol (2 mL), and *N*,*N-*diisopropylethylamine (77 mg, 0.60 mmol) and *tert*-butyl 4-(4-((2-chloropyrimidin-4-yl)amino)phenyl)-1*H*-pyrazole-1-carboxylate (89 mg, 0.24 mmol) were added. The mixture was warmed to 120 °C and reacted for 4 h under nitrogen atmosphere. After the reaction was completed, water (5 mL) was added for dilution, and ethyl acetate (5 mL × 2) was added for extraction. The organic phases were combined, washed with saturated brine (10 mL), dried over anhydrous sodium sulfate and concentrated, and the residue was subjected to preparative chromatography to give a white solid (44.5 mg, 36% two-step yield). LC-MS [M+H]⁺: 504.9.

¹H NMR (400 MHz, DMSO) δ 12.84 (s, 1H), 9.31 (s, 1H), 8.01 (s, 2H), 7.96 (d, J = 5.7 Hz, 1H), 7.65 (d, J = 8.7 Hz, 2H), 7.56 (d, J = 8.7 Hz, 2H), 6.48 (d, J = 4.3 Hz, 0.25H), 6.38 (s, 1H), 6.34 (d, J = 4.3 Hz, 0.5H), 6.20 (d, J = 4.4 Hz, 0.25H), 6.07 (d, J = 5.7 Hz, 1H), 4.79 (s, 2H), 4.65-4.05(m, 4H),3.98 (t, J = 5.5 Hz, 2H), 3.76 (s, 3H), 3.19 - 3.09 (m, 1H), 2.55 (t, J = 6.6 Hz, 2H).

For the preparation of Compound L029, reference was made to the method described in Example 28 above. For the preparation of Compound L033, reference was made to the method described in Example 39 above. For the preparation of Compound L042, reference was made to the method described in Example 41 above. For the preparation of Compound L044, reference was made to the method described in Example 36 above. For the preparation of Compound L049, reference was made to the method described in Example 48 above. For the preparation of Compound L054, reference was made to the method described in Example 53 above. For the preparation of Compound L055, reference was made to the method described in Example 11 above.

Characterization data for compounds synthesized with reference to the corresponding synthetic methods of the above Examples are shown in the table below:

| **Compound No.** | **LC-MS: [M+H]⁺** |
|---|---|
| **L029** | **488.0** |
| **L033** | **522.5** |
| **L042** | **516.1** |
| **L044** | **506.2** |
| **L049** | **534.2** |
| **L054** | **523.2** |
| **L055** | **472.8** |

### Biological activity assay

In some embodiments, the compounds disclosed herein are found to result in kinase inhibition, particularly inhibition of ROCK1 and/or ROCK2 kinase, *in vitro* and/or *in vivo,* as tested by methods conventional in the art. Meanwhile, it is also found that the compounds disclosed herein have relatively low cytotoxicity and good pharmacokinetic property, and experiments also show that the compounds disclosed herein have relatively proper metabolic stability and are promising in druggability.

### 1. In Vitro Evaluation of ROCK2 Kinase Activity

ROCK2 activity screening. ROCK2 activity was detected using a 96-well (Cisbio) time-resolved fluorometric assay. The assay for ROCK2 was performed in the following assay buffer: 5 mM MgCl₂ (Sigma), 1 mM DTT (Sigma) and 1 × kinase buffer. The kinase buffer was used for dilution. 7.5 µL of ROCK2 kinase (Invitrogen, PV3759) was added to a 96-well microplate to reach a final concentration of 0.4 ng/µL, and then 0.25 µL of test compound (DMSO content: 1% (volume fraction)) was added. The mixture was incubated at room temperature for 0.5 h. To start the reaction, the kinase buffer was used to mix ATP (Aladdin) and the substrate STK-substrate 2-biotin. 7.5 µL of the mixed solution was added to the microplate to reach final concentrations of 6.739 µM and 1 µM. The resulting mixture was incubated at room temperature for 2 h. 5 mL of detection buffer was mixed with STK antibody-Cryptate, and then an appropriate volume of the mixture was mixed with an equal volume of streptavidin-XL665. 10 µL of the resulting mixture was added to the microplate to stop the reaction. After further incubation for about 1 h, the microplate was read on a Molecular Devices SpectraMax i3x multifunctional microplate reader. The kinase buffer, the STK-substrate 2-biotin, the detection buffer, the STK antibody-Cryptate, and the streptavidin-XL665 were all from HTRF KinEASE-STK kit (Cisbio, 1000 tests, 61GSTXLA).

### 2. In Vitro Evaluation of ROCK1 Kinase Selectivity

ROCK1 activity was detected using a 96-well (Cisbio) time-resolved fluorometric assay. The assay for ROCK1 was performed in the following assay buffer: 5 mM MgCl₂ (Sigma), 1 mM DTT (Sigma) and 1× kinase buffer. The kinase buffer was used for dilution. 7.5 µL of ROCK1 kinase (Invitrogen) was added to a 96-well microplate to reach a final concentration of 0.4 ng/µL, and then 0.25 µL of test compound (DMSO content: 1%) was added. The mixture was incubated at room temperature for 0.5 h. To start the reaction, the kinase buffer was also used to mix ATP (Aladdin) and the substrate STK-substrate 2-biotin. 7.5 µL of the mixed solution was added to the microplate to reach final concentrations of 3.53 µM and 1 µM. The resulting mixture was incubated at room temperature for 2 h. 5 mL of detection buffer was mixed with STK antibody-Cryptate, and then an appropriate volume of the mixture was mixed with an equal volume of streptavidin-XL665. 10 µL of the resulting mixture was added to stop the reaction. After further incubation for about 1 h, the microplate was read on a Molecular Devices SpectraMax i3x multifunctional microplate reader. The kinase buffer, the STK-substrate 2-biotin, the detection buffer, the STK antibody-Cryptate, and the streptavidin-XL665 were all from HTRF KinEASE-STK kit (Cisbio, 1000 tests, 61GSTXLA).

### Example 61

The compounds of the Examples of the present disclosure were subjected to the experiments on evaluation of *in vitro* kinase activity described above to determine the inhibitory activity of each compound against ROCK1/2. It was found that the compounds disclosed herein all have good activity, wherein for both kinases, some compounds have an IC₅₀ < 10 µM, some compounds have an IC₅₀ ≤ 50 nM (activity level A), some compounds have an IC₅₀ > 50 nM and ≤ 500 nM (activity level B), some compounds have an IC₅₀ > 500 nM and ≤ 4.5 µM (activity level C), and some compounds have an IC₅₀ > 4.5 µM (activity level D).

IC₅₀ values of representative compounds are shown in Table 1 below. Note: (A: ≤ 50 nM; B: > 50 nM and ≤ 500 nM; C: > 500 nM and ≤ 4.5 µM; D: > 4.5 µM; ND: undetectable)

**Table 1. Inhibition of ROCK1 and ROCK2 by compounds disclosed herein**

| **Compound** | **IC₅₀ for ROCK1** | **IC₅₀ for ROCK2** |
|---|---|---|
| **L001** | **ND** | **D** |
| **L002** | **ND** | **D** |
| **L003** | **ND** | **D** |
| **L004** | **C** | **B** |
| **L005** | **D** | **A** |
| **L006** | **C** | **B** |
| **L007** | **ND** | **C** |
| **L008** | **D** | **A** |
| **L009** | **ND** | **C** |
| **L010** | **D** | **A** |
| **L011** | **ND** | **C** |
| **L012** | **B** | **B** |
| **L013** | **ND** | **D** |
| **L014** | **ND** | **D** |
| **L015** | **D** | **B** |
| **L016** | **D** | **A** |
| **L017** | **D** | **A** |
| **L018** | **D** | **B** |
| **L019** | **D** | **A** |
| **L020** | **D** | **B** |
| **L021** | **D** | **B** |
| **L022** | **ND** | **C** |
| **L023** | **C** | **B** |
| **L024** | **C** | **A** |
| **L025** | **ND** | **D** |
| **L026** | **D** | **A** |
| **L027** | **D** | **A** |
| **L028** | **D** | **C** |
| **L029** | **D** | **C** |
| **L030** | **D** | **B** |
| **L031** | **D** | **A** |
| **L032** | **D** | **B** |
| **L033** | **D** | **A** |
| **L034** | **D** | **A** |
| **L035** | **D** | **B** |
| **L036** | **D** | **B** |
| **L037** | **D** | **A** |
| **L038** | **D** | **B** |
| **L039** | **D** | **B** |
| **L040** | **D** | **B** |
| **L041** | **D** | **B** |
| **L042** | **D** | **B** |
| **L043** | **ND** | **B** |
| **L044** | **ND** | **B** |
| **L045** | **D** | **D** |
| **L046** | **D** | **D** |
| **L047** | **D** | **D** |
| **L048** | **D** | **C** |
| **L049** | **D** | **B** |
| **L050** | **D** | **B** |
| **L051** | **D** | **B** |
| **L052** | **D** | **B** |
| **L053** | **D** | **B** |
| **L054** | **D** | **A** |
| **L055** | **D** | **C** |
| **L056** | **D** | **A** |
| **L057** | **D** | **B** |
| **L060** | **D** | **A** |

### Example 62. In Vitro Cytotoxicity Assay for Compounds Disclosed Herein

*In vitro* cytotoxicity assay for the compounds disclosed herein was performed in HepG2 cells using the CCK-8 method. HepG2 cells (Beina Bio) in the logarithmic growth phase were collected, the concentration of cell suspension was adjusted, and then the cells were plated on a 96-well cell culture plate at 50,000 cells/well. The cells were then incubated overnight in a cell incubator (5%, 37 °C), and after 80-90% cell confluence was achieved, test compound at each concentration gradient or vehicle (DMSO) was added after medium change. The resulting mixture was incubated in the cell incubator (5%, 37 °C) for 48 h. After the treatment, the medium in the plate was discarded. The plate was washed twice with PBS, added with CCK-8 working solution (Beyotime) at 100 µL per well, and then incubated at 37 °C for 1.5 h in the dark. Absorbance at OD_{450 nm} was measured for each well on a microplate reader, and CC₅₀ value of each compound was analyzed and calculated.

The compounds of the Examples of the present disclosure were subjected to the experiment described above, and it was found that the compounds disclosed herein all have good safety, wherein the CC₅₀ values of all the compounds are > 10 µM, some preferred compounds have a CC₅₀ > 30 µM, and more preferred compounds have a CC₅₀ > 50 µM. CC₅₀ values of representative compounds are shown in Table 2 below.

**Table 2. CC₅₀ values obtained for compounds**

| **Compound** | **HepG2 CC₅₀ (µM)** |
|---|---|
| **L005** | **>100** |
| **L017** | **33.38** |
| **L026** | **>100** |
| **L027** | **>100** |
| **L030** | **>100** |
| **L031** | **33.38** |
| **L032** | **28.43** |
| **L033** | **>100** |
| **L036** | **>100** |
| **L037** | **>100** |
| **L042** | **27.95** |
| **L043** | **>100** |
| **L044** | **58.12** |
| **L045** | **>100** |
| **L047** | **>100** |
| **L048** | **100.3** |
| **L050** | **>100** |
| **L051** | **>100** |
| **L056** | **>100** |
| **L057** | **>100** |
| **L060** | **>100** |

### Example 63. In Vitro Metabolic Stability Test for Compounds Disclosed Herein

The *in vitro* metabolic stability of the compounds disclosed herein was determined through incubation of liver microsomes of various species. A proper amount of test compound was added into a liver microsome reaction system (1 mg/mL liver microsome protein, 25 U/mL glucose-6 phosphate dehydrogenase, 1 mM NADP, 6 mM D-glucose 6-phosphate and 5 mM MgCh), and then the mixture was incubated in a water bath kettle at 37 °C to start reaction. At each time point, 100 µL of the reaction liquid was added into a centrifuge tube containing 400 µL of internal standard working solution (containing a 200 ng/mL solution of dexamethasone, diclofenac, tolbutamide and labetalol in acetonitrile) precooled at 0 °C so as to stop the reaction, and the mixture was then centrifuged at 10,000 g for 10 min at 4 °C. The supernatant was collected for LC-MS assay so as to obtain the values of *in vitro* metabolic half life of the test compounds in liver microsomes of various species. T_{1/2} values obtained using the above method are shown in Table 3.

**Table 3. Metabolic half-life of each compound in liver microsome protein**

| **Compound** | **T_{1/2} in human liver microsome min** | **T_{1/2} in rat liver microsome min** | **T_{1/2} in guinea pig liver microsome min** |
|---|---|---|---|
| **L005** | **175.32** | **103.38** | **64.83** |
| **L017** | **186.38** | **72.53** | **45.03** |
| **KD-025** | **91.31** | **17.22** | **96.37** |
| **L026** | **237.27** | **61.23** | **28.36** |
| **L027** | **57.35** | **126.78** | **617.17** |
| **L030** | **207.36** | **158.15** | **58.16** |
| **L032** | **80.97** | **42.51** | **33.77** |
| **L033** | **93.24** | **68.28** | **50.20** |
| **L036** | **177.97** | **94.36** | **75.16** |
| **L037** | **156.72** | **33.24** | **60.00** |
| **L042** | **438.19** | **344.77** | **348.71** |
| **L043** | **111.96** | **80.41** | **85.20** |
| **L044** | **438.91** | **209.67** | **169.51** |
| **L050** | **258.93** | **61.70** | **64.16** |
| **L051** | **289.46** | **65.04** | **50.38** |
| **L056** | **60.38** | **52.75** | **42.07** |
| **L057** | **190.15** | **45.53** | **131.03** |
| **L060** | **95.32** | **69.82** | **58.16** |

The compounds of the Embodiments of the present disclosure were subjected to the experiment described above, and it was found that the compounds disclosed herein have relatively good metabolic stability in human, rat and mice. Preferred is T_{1/2} > 30 min in human liver microsome, and more preferred is T_{1/2} > 90 min in human liver microsome. The inventors also conducted comparative experiment with KD-025, a ROCK inhibitor in clinic phase, and found that preferred compounds of the present disclosure have better *in vitro* stability than KD-025.

### Example 64. In Vivo Pharmacokinetic Study of Compounds Disclosed Herein in Mice

SPF-grade mice (SPF (Beijing) Biotechnology Co., Ltd.) were fasted overnight (without water deprivation) after adaptive feeding, and then received 3 mg/kg of the compound disclosed herein by intragastrical **administration** and bolus injection at tail vein. After administration, plasma of mice was collected at specific time points, and the concentration of the compound in the plasma was detected by LC-MS/MS (AB SCIEX Qtrap4500). The PK parameters of the compounds were statistically analyzed and calculated (WinNonlin V5.2, Pharsight) to show the pharmacokinetic properties of the compounds disclosed herein in mice.

The compounds of the Examples of the present disclosure were subjected to the experiment described above, and the results showed that the compounds disclosed herein all have good pharmacokinetic properties in mice, and can realize high exposure *in vivo* and high oral bioavailability at a low dose, wherein the oral bioavailability of some compounds is > 30%, and the oral bioavailability of some compounds is > 50%. Table 4 below shows experimental data for representative compounds.

**Table 4. PK experiment of compounds disclosed herein in mice**

| **Compound** | **Dose of administration mg/kg** | **Route of administration** | **Cmax (ng/mL)** | **AUCₗₐₛₜ (ng/mL*hr)** | **Oral bioavailability (%)** |
|---|---|---|---|---|---|
| **L005** | **1** | **Po (fasted)** | **37.2** | **71.8** | **59.7** |
| | **1** | **Iv** | **155** | **120** | **/** |
| **L017** | **1** | **Po (fasted)** | **41.3** | **106.0** | **12.2** |
| | **1** | **Iv** | **2570** | **872** | **/** |
| **L027** | **1** | **Po (fasted)** | **116** | **341** | **82.7** |
| | **1** | **Iv** | **517** | **412** | **/** |
| **L044** | **1** | **Po (fasted)** | **109** | **431** | **54.8** |
| | **1** | **Iv** | **594** | **787** | **/** |
| **L036** | **1** | **Po (fasted)** | **98.7** | **209** | **59.9** |
| | **1** | **Iv** | **473** | **349** | **/** |
| **L030** | **1** | **Po (fasted)** | **60** | **225** | **30.6** |
| | **1** | **Iv** | **1121** | **726** | **/** |
| **L060** | **1** | **Po (fasted)** | **64.5** | **144** | **54.0** |
| | **1** | **Iv** | **463** | **266** | **/** |

In the specification, description involving the term "one embodiment", "some embodiments", "examples", "a specific example", "some examples" or the like means that a particular feature, structure, material or characteristic described in reference to the embodiment or example is included in at least one embodiment or example of the present disclosure. In this specification, the schematic descriptions of the terms described above do not necessarily refer to the same embodiment or example. Moreover, the specific features, materials, structures and other characteristics described may be combined in any one or more embodiments or examples in an appropriate manner. Moreover, various embodiments or examples and features of various embodiments or examples described in this specification can be combined by one skilled in the art to the extent that they do not contradict each other.

Although examples of the present disclosure are illustrated and described above, it will be appreciated that the above examples are exemplary and not to be construed as limiting the present disclosure, and that changes, modifications, substitutions and alterations can be made to the above examples by those of ordinary skill in the art within the scope of the present disclosure.

## Claims

1. A compound represented by formula (I) below or a racemate, a stereoisomer, a tautomer, an isotopically labeled compound, a nitrogen oxide, a solvate, a polymorph, a metabolite, an ester, a pharmaceutically acceptable salt or a prodrug thereof: 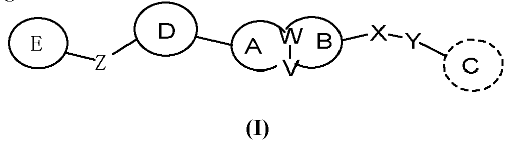 wherein,
ring A is the following group unsubstituted or optionally substituted with one, two or more Rc: 3-20 membered heterocyclyl or 5-20 membered heteroaryl;
ring B is the following group unsubstituted or optionally substituted with one, two or more Rd: C₃₋₂₀ cycloalkyl, 3-20 membered heterocyclyl, C₆₋₂₀ aryl or 5-20 membered heteroaryl;
the ring A and the ring B constitute a fused ring with W and V as connection sites;
Rc and Rd are identical or different and are each independently selected from halogen, amino, hydroxyl, formyl, and the following groups unsubstituted or optionally substituted with one, two or more Rs: C₁₋₂₀ alkyl and C₁₋₂₀ alkoxy;
W and V are each independently C or N, and W and V are not both N at the same time;
X is -C(=O)-;
Y is OH, a chemical bond, C₁₋₂₀ alkoxy, C₁₋₂₀ alkyl, -NH-, or -N(C₁₋₂₀ alkyl)-, -NH(C₁₋₂₀ alkyl) or -NH(C₁₋₂₀ alkoxy), wherein the C₁₋₂₀ alkoxy, the C₁₋₂₀ alkyl and the C₁₋₂₀ alkoxy and C₁₋₂₀ alkyl in the "-N(C₁₋₂₀ alkyl)-, NH(C₁₋₂₀ alkyl) or -NH(C₁₋₂₀ alkoxy)" can be unsubstituted or optionally substituted with one, two or more Rs; 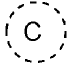
can be present or absent;
provided that Y is OH, C₁₋₂₀ alkoxy, C₁₋₂₀ alkyl, -NH(C₁₋₂₀ alkyl) or -NH(C₁₋₂₀ alkoxy), 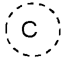 is absent;
when Y is a chemical bond, 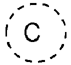 is selected from the following groups unsubstituted or optionally **substituted** with one, two or more Ra: 3-20 membered heterocyclyl, 5-20 membered heteroaryl, C₃₋₂₀ cycloalkyl and C₆₋₂₀ aryl;
when Y is-NH- or -N(C₁₋₂₀ alkyl)-, 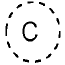 is selected from the following groups unsubstituted or optionally substituted with one, two or more Rb: C₁₋₂₀ alkyl, 3-20 membered heterocyclyl, 5-20 membered heteroaryl, C₃₋₂₀ cycloalkyl and C₆₋₂₀ aryl;
Ra and Rb are identical or different and are each independently selected from halogen, CN, C₁₋₂₀ alkyl unsubstituted or optionally substituted with one, two or more Rs, OH, 5-20 membered heteroaryl and 5-20 membered haloheteroaryl;
ring D is the following group unsubstituted or optionally substituted with one, two or more Re: C₃₋₂₀ cycloalkyl, 3-20 membered heterocyclyl, C₆₋₂₀ aryl or 5-20 membered heteroaryl, and the ring A is connected to the ring D through an N atom;
Re is selected from halogen, CN, OH and the following groups unsubstituted or optionally substituted with one, two or more Rs: C₁₋₂₀ alkyl, C₃₋₂₀ cycloalkyl, C₁₋₂₀ alkoxy, C₆₋₂₀ aryl, 3-20 membered heterocyclyl and 5-20 membered heteroaryl;
or when at least two Re substitutions are present on the ring D, adjacent two Re, together with an atom on the ring D to which they are connected via a bond, form C₃₋₂₀ cycloalkyl, 3-20 membered heterocyclyl or 3-20 membered heteroaryl;
Rs is selected from halogen, CN, OH, C₁₋₂₀ alkyl, C₁₋₂₀ alkoxy, C₆₋₂₀ aryl and 5-20 membered heteroaryl;
Z is -NH-;
ring E is the following group unsubstituted or optionally substituted with one, two or more Rf: C₆₋₂₀ aryl or 5-20 membered heteroaryl;
Rf is selected from halogen, CN, OH, and the following groups unsubstituted or optionally substituted with one, two or more Rg: C₁-C₂₀ alkyl, C₆₋₂₀ aryl and 5-20 membered heteroaryl;
Rg is selected from halogen, CN, OH, C₁-C₂₀ alkyl optionally substituted with one, two or more halogens, and -0-5-20 membered heteroaryl, wherein the -0-5-20 membered heteroaryl is optionally substituted with - C(O)-NH₂.

2. The compound represented by formula (I) or the racemate, the stereoisomer, the tautomer, the isotopically labeled compound, the nitrogen oxide, the solvate, the polymorph, the metabolite, the ester, the pharmaceutically acceptable salt or the prodrug thereof according to claim 1, wherein:
W is selected from N and C;
V is selected from C;
X is -C(=O)-;
Y is OH, a chemical bond, C₁₋₆ alkoxy, C₁₋₆ alkyl, -NH-, or -N(C₁₋₆ alkyl)-, -NH(C₁₋₆ alkyl) or -NH(C₁₋₆ alkoxy), wherein the C₁₋₆ alkoxy, the C₁₋₆ alkyl, and the C₁₋₆ alkoxy and C₁₋₆ alkyl in the "-N(C₁₋₆ alkyl)-, -NH(C₁₋₆ alkyl) or -NH(C₁₋₆ alkoxy)" can be unsubstituted or optionally substituted with one, two or more Rs; Rs is selected from halogen, CN, OH, C₁₋₆ alkyl, C₁₋₆ alkoxy, C₆₋₁₂ aryl and 5-12 membered heteroaryl;
when Y is a chemical bond, 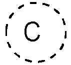 is selected from the following groups unsubstituted or optionally substituted with one, two or more Ra: 3-6 membered heterocyclyl and 5-12 membered heteroaryl;
Ra is selected from F, Cl, CN, OH, and C₁₋₆ alkyl optionally substituted with one, two or more halogens;
when Y is -NH- or -N(C₁₋₆ alkyl)-, 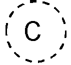 is selected from the following groups unsubstituted or optionally substituted with one, two or more Rb: C₃₋₆ cycloalkyl, C₁₋₆ alkyl, 3-6 membered heterocyclyl and 5-12 membered heteroaryl;
Rb is selected from F, Cl, CN, OH, and 5-12 membered heteroaryl substituted with F or Cl;
when Y is a chemical bond, X is connected to a heteroatom of the 3-6 membered heterocyclyl represented by 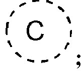
ring A is the following group unsubstituted or optionally substituted with one, two or more Rc: 3-6 membered heterocyclyl or 5-12 membered heteroaryl;
ring B is the following group unsubstituted or optionally substituted with one, two or more Rd: 3-6 membered heterocyclyl, C₆₋₁₂ aryl or 5-12 membered heteroaryl;
the ring A and the ring B constitute a fused ring with W and V as connection sites;
Rc and Rd are identical or different and are each independently selected from F, Cl, amino, hydroxyl, formyl or C₁₋₆ alkyl, wherein the C₁₋₆ alkyl is unsubstituted or optionally substituted with one, two or more halogens;
the ring D is the following group unsubstituted or optionally substituted with one, two or more Re: C₆₋₁₂ aryl or 5-12 membered heteroaryl; and the ring A is connected to the ring D through an N atom;
Re is selected from halogen, CN, OH, and the following groups unsubstituted or optionally substituted with one, two or more Rs: C₁₋₆ alkyl, C₃₋₆ cycloalkyl, C₁₋₆ alkoxy, C₆₋₁₂ aryl, 3-6 membered heterocyclyl and 5-12 membered heteroaryl;
or when at least two Re substitutions are present on the ring D, adjacent two Re, together with an atom on the ring D to which they are connected via a bond, form C₃₋₆ cycloalkyl, 3-6 membered heterocyclyl or 5-12 membered heteroaryl;
Rs is selected from halogen, CN, OH, C₁₋₆ alkyl, C₁₋₆ alkoxy, C₆₋₁₂ aryl and 5-12 membered heteroaryl;
Z is -NH-;
the ring E is C₆₋₁₂ aryl or 5-12 membered heteroaryl unsubstituted or optionally substituted with one, two or more Rf;
Rf is selected from F, Cl, and the following groups unsubstituted or optionally substituted with one, two or more Rg: C₆₋₁₂ aryl and 5-12 membered heteroaryl;
Rg is selected from F, Cl and -0-5-12 membered heteroaryl, wherein the -0-5-12 membered heteroaryl is optionally substituted with -C(O)-NH₂.

3. The compound represented by formula (I) or the racemate, the stereoisomer, the tautomer, the isotopically labeled compound, the nitrogen oxide, the solvate, the polymorph, the metabolite, the ester, the pharmaceutically acceptable salt or the prodrug thereof according to claim 1 or 2, wherein:
W is selected from N and C;
V is selected from C;
X is -C(=O)-;
Y is OH, a chemical bond, -NH- or -N(methyl)-, provided that when Y is OH, 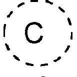 is absent;
when Y is a chemical bond, 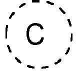 is selected from the following groups: azetidinyl unsubstituted or substituted with one, two or more F; azetidinyl unsubstituted or substituted with one, two or more CN;
azetidinyl unsubstituted or substituted with one, two or more CF₂; azetidinyl unsubstituted or substituted with one, two or more CF₃; and azolidinyl unsubstituted or substituted with F and/or OH;
when Y is -NH- or -N(methyl)-, 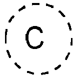 is selected from the following groups: cyclobutyl unsubstituted or substituted with one, two or more F; isopropyl; pyridazinyl; azetidinyl unsubstituted or substituted with one, two or more CN; azolidinyl substituted with F and/or OH; ethyl unsubstituted or substituted with one, two or more F; -N-chloropyridin-piperidinyl; and pyridinyl unsubstituted or substituted with one, two or more F;
when Y is a chemical bond, X is connected to a heteroatom of the heterocyclyl represented by

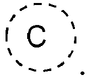

4. The compound represented by formula (I) or the racemate, the stereoisomer, the tautomer, the isotopically labeled compound, the nitrogen oxide, the solvate, the polymorph, the metabolite, the ester, the pharmaceutically acceptable salt or the prodrug thereof according to any one of claims 1-3, wherein Y is methyl, ethyl, propyl, butyl, -CH₂CF₃, -NH-CH₂CF₃, -NHCH₂(CH₂)₂, -OCH₂CF₃ or -OCH₂CH₃; preferably, the ring A and the ring B constitute a fused ring as follows: 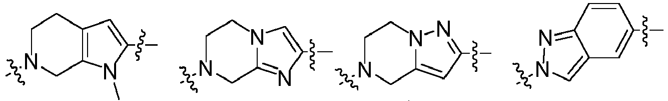 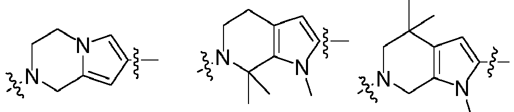 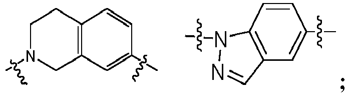
the ring D is the following group unsubstituted or optionally substituted with one, two or more Re: 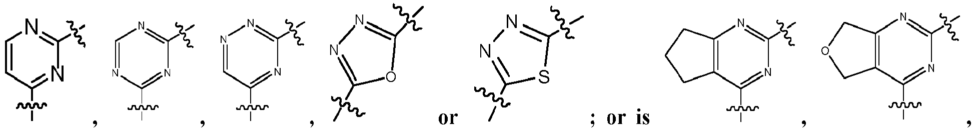 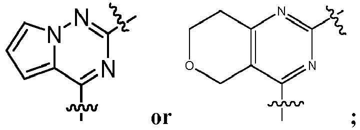
Z is -NH-;
the ring E is

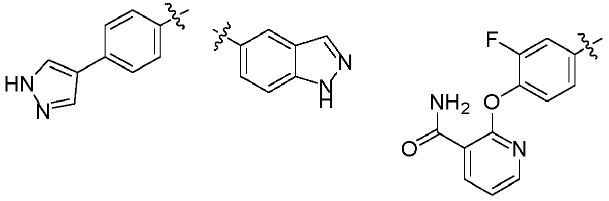

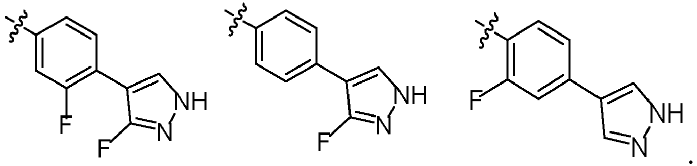

5. The compound represented by formula (I) or the racemate, the stereoisomer, the tautomer, the isotopically labeled compound, the nitrogen oxide, the solvate, the polymorph, the metabolite, the ester, the pharmaceutically acceptable salt or the prodrug thereof according to any one of claims 1-4, wherein the formula I is further selected from the following formula II: 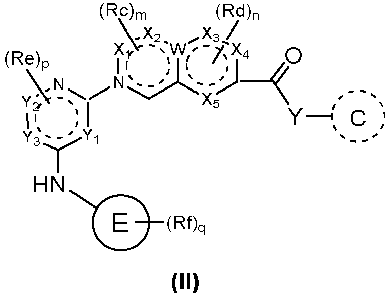
wherein, W is selected from N and C;
a double line comprising a solid line and a dotted line represents a single bond or a double bond;
X₁, X₃ and X₅ are each independently selected from N and C, and X₁, X₃ and X₅ are not N at the same time; X₂ and X₄ are selected from C and a chemical bond;
X₃ is selected from N and C;
Y₁ is selected from N, O and S;
Y₂ is selected from C, N and a chemical bond;
Y₃ is selected from N and C;
Y₂ and Y₃ are not both N at the same time;
m is 0, 1, 2, 3 or 4;
n is 0, 1, 2 or 3;
p is 0, 1, 2, 3 or 4;
q is 0, 1, 2, 3 or 4;
the other groups are as defined in any one of claims 1-4.

6. The compound represented by formula (I) or the racemate, the stereoisomer, the tautomer, the isotopically labeled compound, the nitrogen oxide, the solvate, the polymorph, the metabolite, the ester, the pharmaceutically acceptable salt or the prodrug thereof according to any one of claims 1-4, wherein the formula I is further selected from the following formula III: 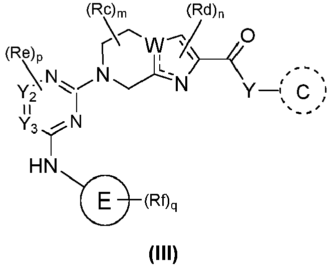
wherein, W is selected from N and C;
a double line comprising a solid line and a dotted line represents a single bond or a double bond;
Y₂ and Y₃ are each independently C or N, and Y₂ and Y₃ are not both N at the same time;
m is 0, 1, 2, 3 or 4;
n is 0, 1, 2 or 3;
p is 0, 1, 2, 3 or 4;
q is 0, 1, 2, 3 or 4;
the other groups are as defined in any one of claims 1-4.

7. The compound represented by formula (I) or the racemate, the stereoisomer, the tautomer, the isotopically labeled compound, the nitrogen oxide, the solvate, the polymorph, the metabolite, the ester, the pharmaceutically acceptable salt or the prodrug thereof according to any one of claims 1-4, wherein the formula I is further selected from the following formula IV: 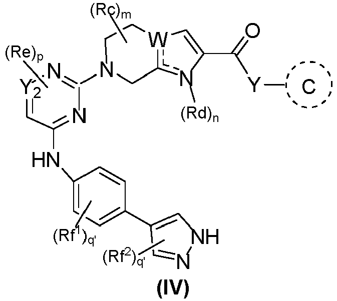
wherein, W is selected from N and C;
Y₂ is selected from N and C;
a double line comprising a solid line and a dotted line represents a single bond or a double bond;
Y is a chemical bond, -NH-, or -N(C₁₋₆ alkyl)- or -NH(C₁₋₆ alkyl);
the C₁₋₆ alkoxy in the "-N(C₁₋₆ alkyl)- or -NH(C₁₋₆ alkyl)" is unsubstituted or optionally substituted with one, two or more halogens;
when Y is a chemical bond, 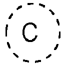 is selected from the following groups: 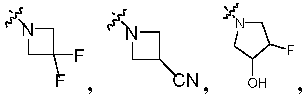 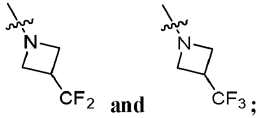
when Y is -NH- or -N(C₁₋₆ alkyl)-, 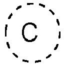 is selected from the following group: 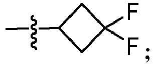
when Y is -N(C₁₋₆ alkyl), 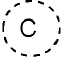 is absent;
Re and Rd are identical or different and are each independently selected from C₁₋₆ alkyl;
Re is selected from halogen, CN, OH, and the following groups unsubstituted or optionally substituted with one, two or more Rs: C₁₋₆ alkyl, C₃₋₆ cycloalkyl, C₁₋₆ alkoxy, C₆₋₁₂ aryl, 3-6 membered heterocyclyl and 5-12 membered heteroaryl;
or when at least two Re are present, adjacent two Re, together with a ring atom to which they are connected via a bond, form C₃₋₆ cycloalkyl, 3-6 membered heterocyclyl or 5-12 membered heteroaryl;
Rs is selected from halogen, CN, OH, C₁₋₆ alkyl, C₁₋₆ alkoxy, C₆₋₁₂ aryl and 5-12 membered heteroaryl;
Rf¹ and Rf² are identical or different and are each independently selected from Rf as defined above, preferably each independently selected from halogen, CN and OH;
m is 0, 1 or 2;
n is 0, 1 or 2;
p is 0, 1 or 2;
q', when present, is each independently 0, 1 or 2.

8. The compound represented by formula (I) or the racemate, the stereoisomer, the tautomer, the isotopically labeled compound, the nitrogen oxide, the solvate, the polymorph, the metabolite, the ester, the pharmaceutically acceptable salt or the prodrug thereof according to any one of claims 1-4, wherein the formula I is further selected from the following formula V: 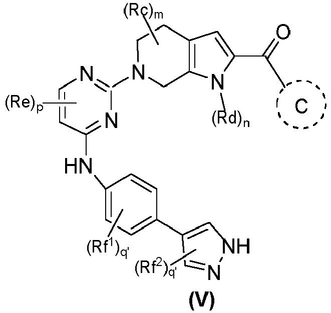 wherein, 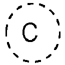 is selected from the following groups: 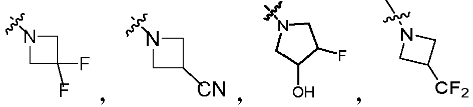 and 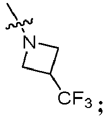
Rc and Rd are identical or different and are each independently selected from C₁₋₆ alkyl;
Re is selected from halogen, CN, OH, and the following groups unsubstituted or optionally substituted with one, two or more Rs: C₁₋₆ alkyl, C₃₋₆ cycloalkyl, C₁₋₆ alkoxy, C₆₋₁₂ aryl, 3-6 membered heterocyclyl and 5-12 membered heteroaryl;
or when at least two Re are present, adjacent two Re, together with a ring atom to which they are connected via a bond, form C₃₋₆ cycloalkyl, 3-6 membered heterocyclyl or 5-12 membered heteroaryl;
Rs is selected from halogen, CN, OH, C₁₋₆ alkyl, C₁₋₆ alkoxy, C₆₋₁₂ aryl and 5-12 membered heteroaryl;
Rf¹ and Rf² are identical or different and are each independently selected from Rf as defined above, preferably each independently selected from halogen, CN and OH;
m is 0, 1 or 2;
n is 0, 1 or 2;
p is 0, 1 or 2;
q', when present, is each independently 0, 1 or 2.

9. The compound represented by formula (I) or the racemate, the stereoisomer, the tautomer, the isotopically labeled compound, the nitrogen oxide, the solvate, the polymorph, the metabolite, the ester, the pharmaceutically acceptable salt or the prodrug thereof according to any one of claims 1-8, wherein the compound is selected from the following structures: 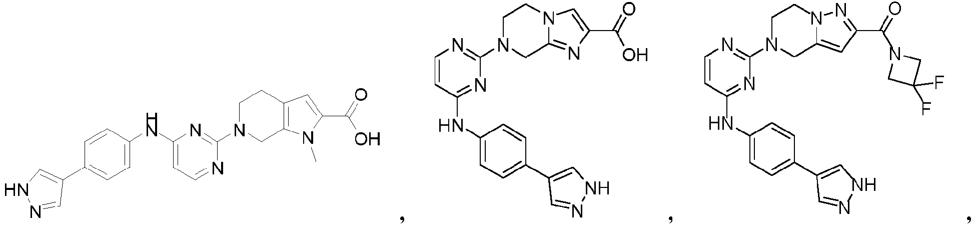 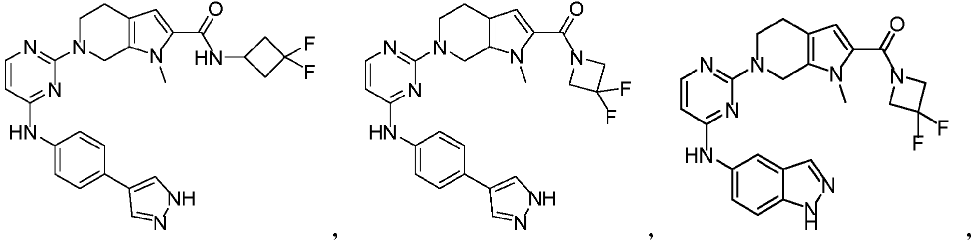 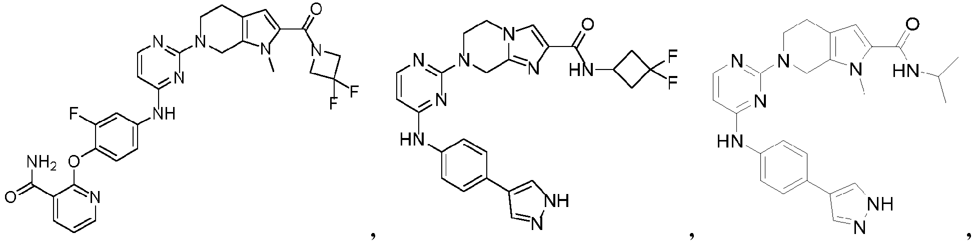 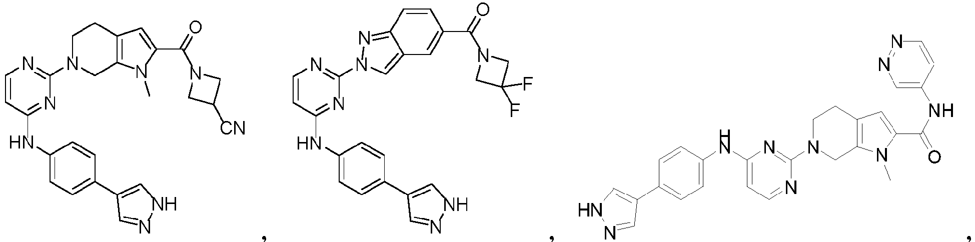 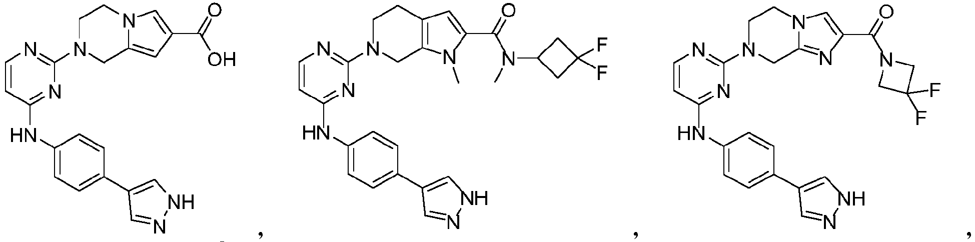 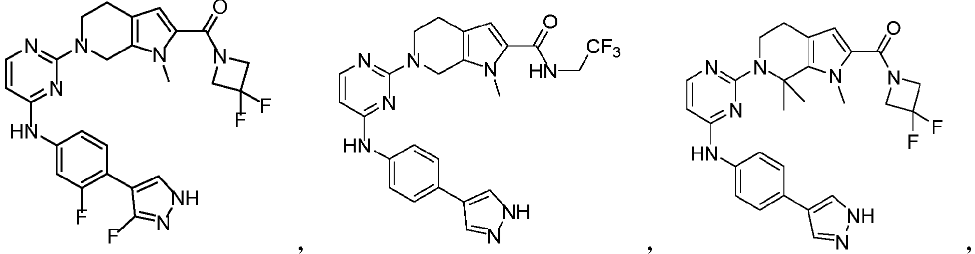 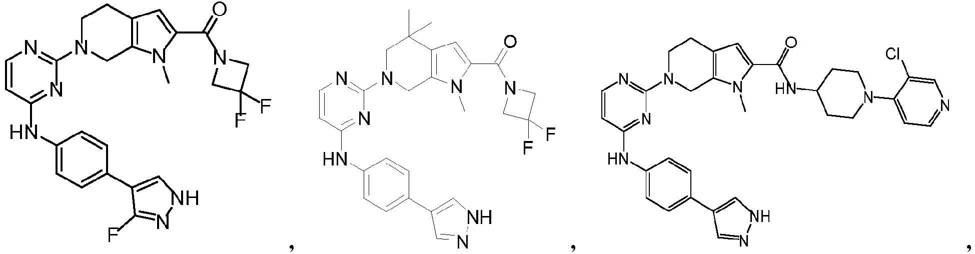 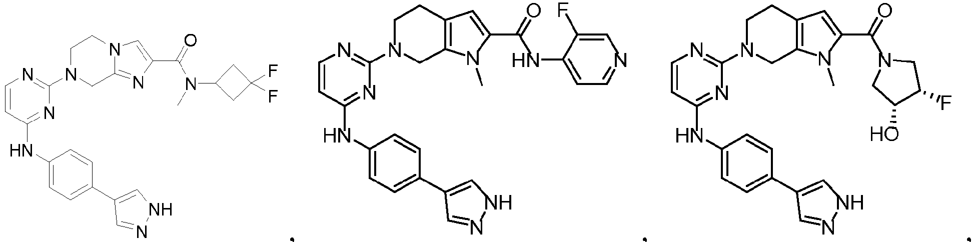 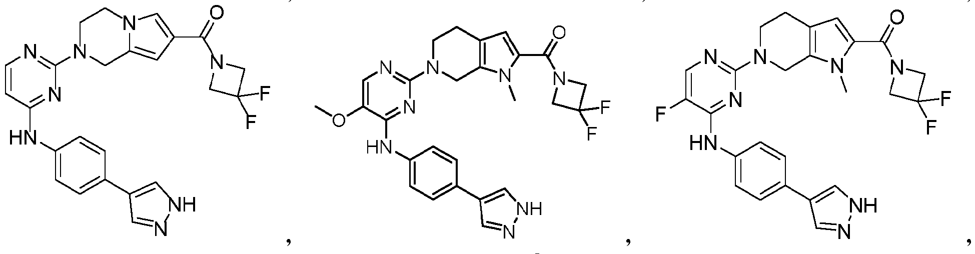 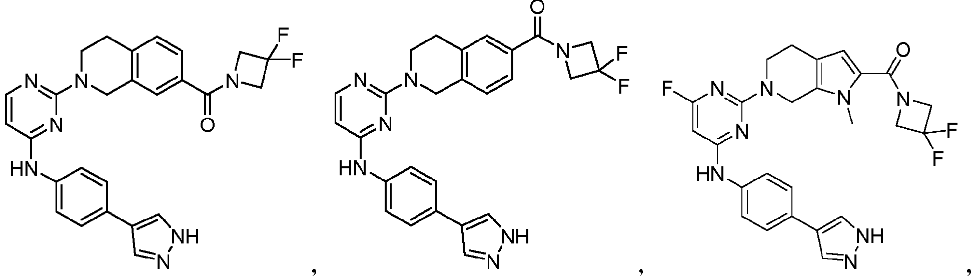 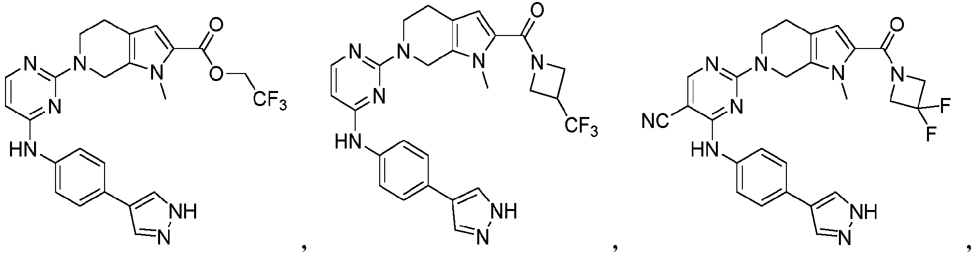 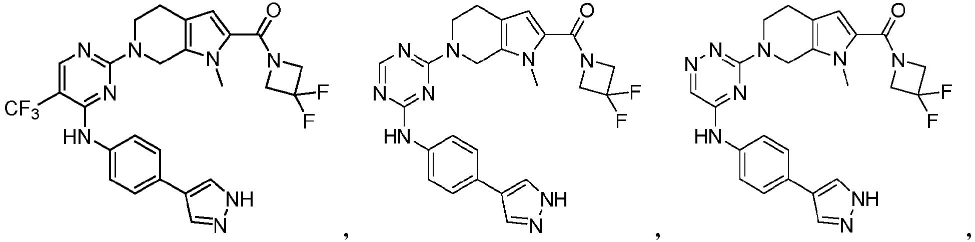 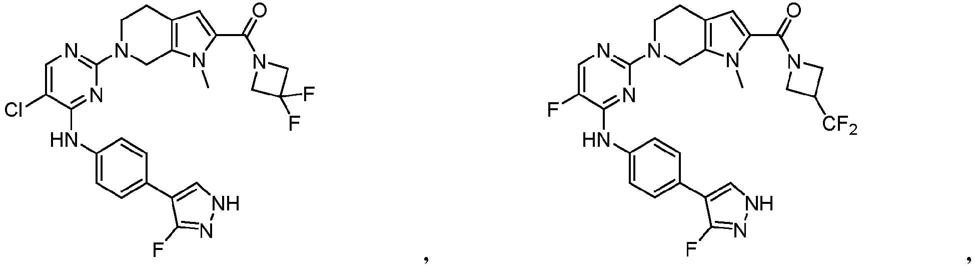 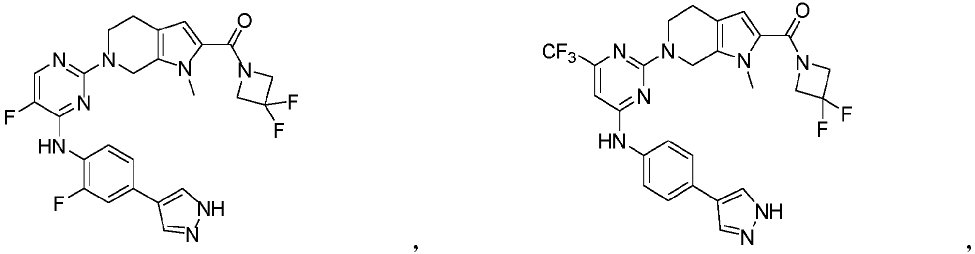 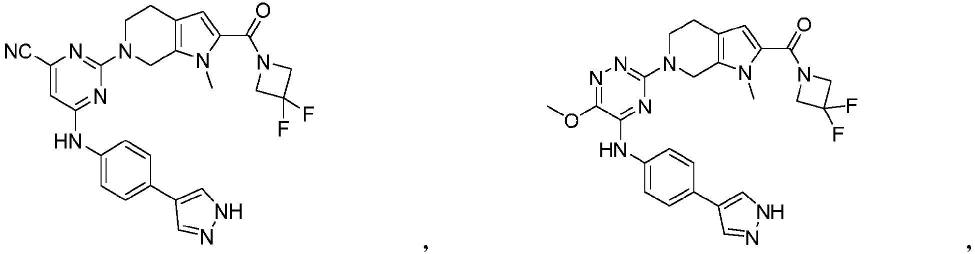 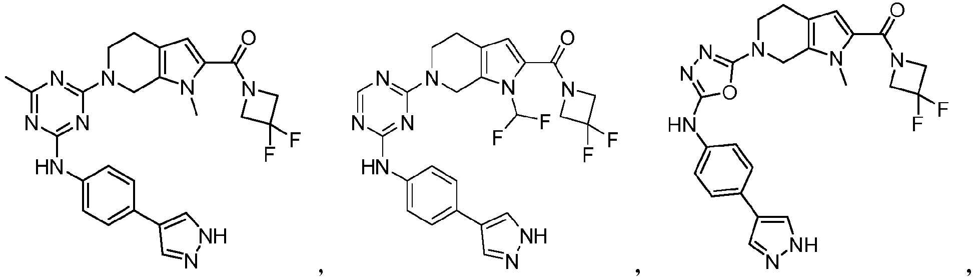 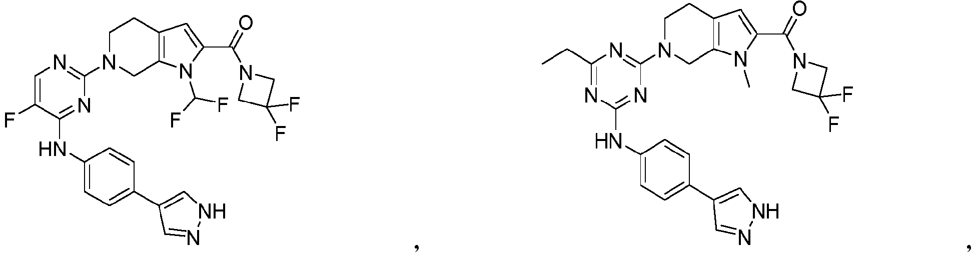 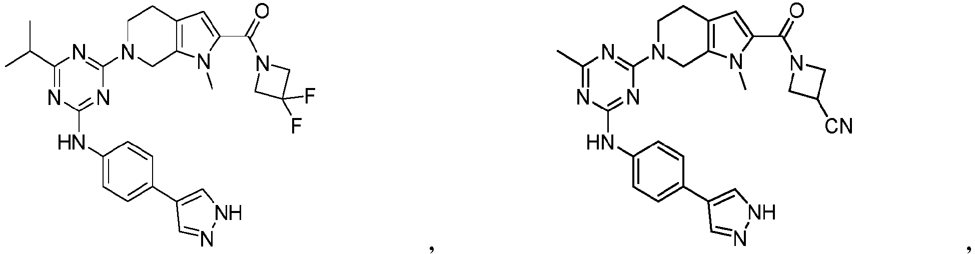 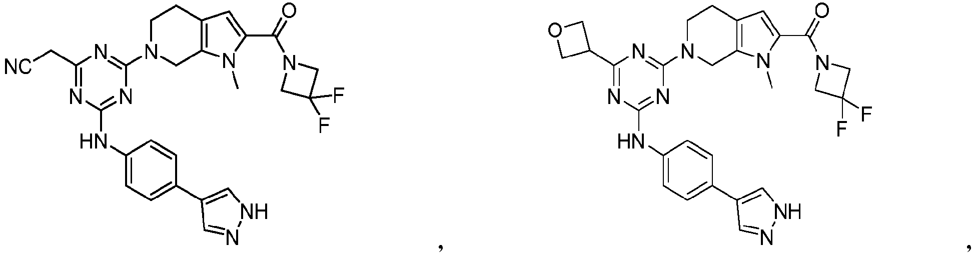 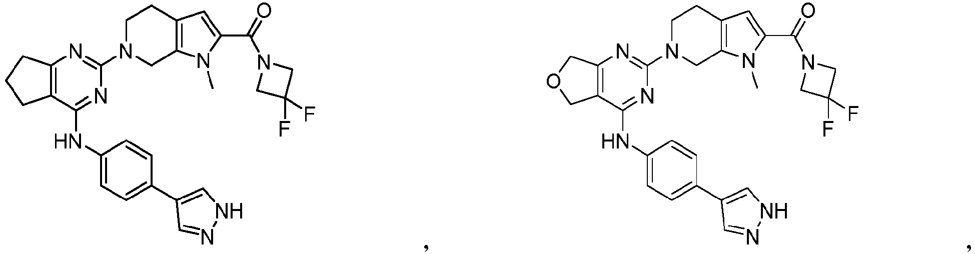 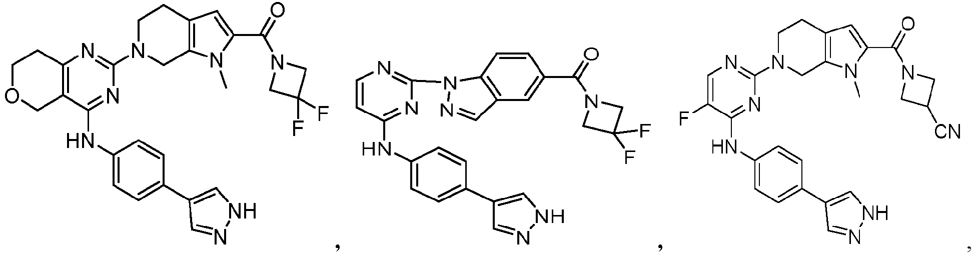 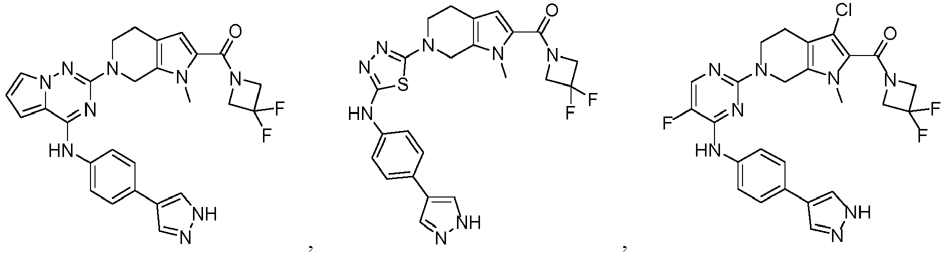 and

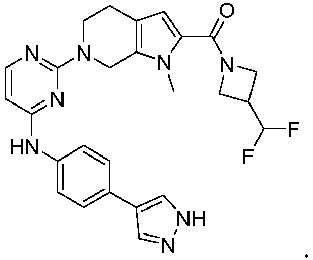

10. A method for preparing the compound represented by formula (I) according to any one of claims 1-9, comprising: 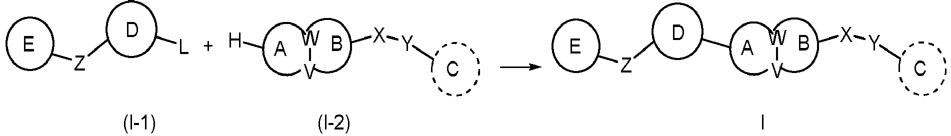
reacting a compound represented by formula (I-1) with a compound represented by formula (I-2) to give the compound represented by formula (I);
wherein, ring A, ring B, ring D, 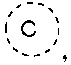 ring E, W, V, X, Y and Z are as defined in any one of claims 1-4 above;
L is selected from leaving groups, such as halogen.

11. An intermediate for preparing the compound represented by formula (I) according to any one of claims 1-9, selected from the following structures: 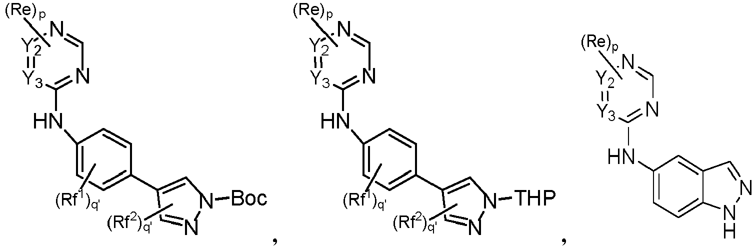 and 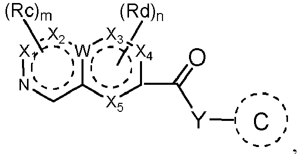 wherein each group is as defined in any one of claims 1-9;
or, selected from the following structures:

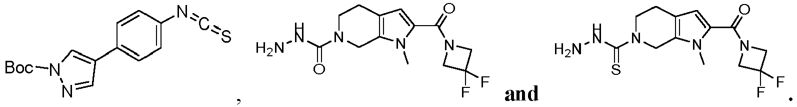

12. Use of at least one of the compound represented by formula (I) and the racemate, the stereoisomer, the tautomer, the nitrogen oxide, the isotopically labeled compound, the solvate, the polymorph, the metabolite, the ester, the pharmaceutically acceptable salt and the prodrug thereof according to any one of claims 1-9 for manufacturing a medicament, wherein the medicament is an inhibitor of protein kinase.

13. The use according to claim 12, wherein the medicament has the function of regulating Rho-kinase.

14. The use according to claim 12 or 13, wherein the medicament is used for preventing or treating one or more diseases caused by high expression of ROCK or excessive activation of ROCK;
preferably, the medicament is used for preventing or treating the following diseases: cardiovascular and cerebrovascular diseases, neurological diseases, fibrosis diseases, ocular diseases, tumors, arterial thrombotic disorders, radiation damage, respiratory diseases, autoimmune diseases, etc., including atherosclerosis, acute coronary syndrome, hypertension, cerebral vasospasm, cerebral ischemia, ischemic stroke, restenosis, heart disease, heart failure, cardiac hypertrophy, myocardial ischemia-reperfusion injury, diabetes, diabetic nephropathy, cancer, neuronal degeneration (peripheral or central), nerve injury diseases, spinal cord injury, erectile dysfunction, platelet aggregation, leukocyte aggregation, glaucoma, ocular hypertension, asthma, osteoporosis, pulmonary fibrosis (such as idiopathic pulmonary fibrosis), hepatic fibrosis, renal fibrosis, COPD, kidney dialysis (epithelial stability), glomerulosclerosis, and neuronal degeneration inflammation.

15. A pharmaceutical composition, comprising a therapeutically effective amount of at least one of the compound represented by formula (I) and the racemate, the stereoisomer, the tautomer, the nitrogen oxide, the isotopically labeled compound, the solvate, the polymorph, the metabolite, the ester, the pharmaceutically acceptable salt and the prodrug thereof according to any one of claims 1-9.

16. The pharmaceutical composition according to claim 15, wherein the pharmaceutical composition comprises a pharmaceutically acceptable carrier or excipient; preferably, the auxiliary material is selected from at least one of the following: a disintegrant, a glidant, a lubricant, a diluent, a filler, an adhesive and a colorant.
